# EUROPEAN PATENT SPECIFICATION

(11) **EP 4 155 305 B1**
(45) Date of publication and mention of the grant of the patent: **09.09.2026**
(21) Application number: 21808555.3
(22) Date of filing: 21.05.2021
(51) Int. Cl.: C07D 403/12, C07D 401/12, C07D 405/12, C07D 413/12, C07D 417/12, C07D 471/12, C07D 471/04, C07D 487/04, C07D 209/52, A61K 31/403, A61P 11/06, A61P 29/00, A61P 3/10, A61P 25/00, A61P 13/00

(54) **FUSED RING COMPOUNDS, PREPARATION METHOD THEREFOR, PHARMACEUTICAL COMPOSITIONS AND USE THEREOF**
KONDENSIERTE RINGVERBINDUNGEN, HERSTELLUNGSVERFAHREN DAFÜR, PHARMAZEUTISCHE ZUSAMMENSETZUNGEN UND VERWENDUNG DAVON
COMPOSÉS CYCLIQUES CONDENSÉS, LEUR PROCÉDÉ DE PRÉPARATION, COMPOSITIONS PHARMACEUTIQUES ET LEUR UTILISATION

(30) Priority: 21.05.2020 CN 202010433988; 14.05.2021 CN 202110529033
(43) Date of publication of application: 29.03.2023
(73) Proprietor: Guangzhou Fermion Technology Co., Ltd., Guangzhou, Guangdong 510000 (CN)
(72) Inventor: DENG, Daiguo, Guangzhou, Guangdong 510000 (CN); QIU, Guanpeng, Guangzhou, Guangdong 510000 (CN); WANG, Yonggang, Guangzhou, Guangdong 510000 (CN); LEI, Zengrong, Guangzhou, Guangdong 510000 (CN)
(74) Representative: Mewburn Ellis LLP
(86) International application number: PCT/CN2021/095191
(87) International publication number: WO 2021/233427

(56) References cited:
- WO-A1-2012/125661
- WO-A1-2014/184275
- CN-A- 105 473 574
- US-A1- 2014 343 295
- US-A1- 2017 014 381
- US-A1- 2018 092 880
- US-A1- 2019 269 650

## Description

### TECHNICAL FIELD

The present invention relates to an organic compound, in particular to a fused-ring compound and a preparation method, a pharmaceutical composition, and an application thereof.

### BACKGROUND

Somatostatin receptors (SSTRs) are a family of G protein-coupled receptors that mediate somatostatin and analogs thereof and have a variety of biological effects, the physiological functions and function mechanisms of which have received much attention for a long term. Studies have shown that these specific membrane receptors present on cell membranes include SSTR1, SSTR2, SSTR3, SSTR4, and SSTR5, which can play an important role in biological processes such as regulation of growth hormone (GH) secretion, induction of cell apoptosis, inhibition of tumor cell proliferation, inhibition of insulin action, and inhibition of cell growth via cAMP, PTP, and MAPK signaling pathways, while exhibiting kinetic characteristics similar to properties of other G protein-coupled receptors.

Somatostatin (SST) is a cyclic polypeptide widely distributed in the central nervous system and surrounding tissues, which exists *in vivo* in two forms, 14-peptide (SST-14) and 28-peptide (SST-28). Previous studies have shown that SST as a signaling molecule is mediated by the SST receptor family on the cell membrane. SST is present in only two forms. The complexity of SST physiological functions is reflected by the complexity of receptors. Therefore, the biological significance of SSTRs is more important than that of SST to some extent. The SSTRs are structurally similar to other G protein-coupled receptors, having seven transmembrane (TM) α-helix structures, and an N-terminal region having an N-glycosylation site and a palmitoylation site (except SSTR3). In addition, there is a highly conserved amino acid sequence unique to the SSTRs in TM7.

The SSTRs are coupled to a variety of cellular effector systems via G proteins, mainly involving the following four important signal transduction pathways: the first is a cyclic adenylate (cAMP) pathway; the second is a voltage-dependent Ca²⁺ pathway; the third is a mitogen-activated protein kinase (MAPK) pathway; and the fourth is a protein tyrosine phosphatase (PTP) pathway.

SSTR1 is related to the inhibition of cell growth; SSTR3, in addition to inducing apoptosis, is also involved in inhibiting the release of GH and insulin, and processing and regulating sensory signals, as well as the integration of sensory and visceral functions, sense of smell, and other sensory functions; SSTR4 also inhibits the release of GH and insulin, and coordinates extrapyramidal motor and sensory functions; SSTR2 and SSTR5 play a major role in regulating the growth of animals, mainly inhibiting the release of GH and insulin, participating in central integration, and also participating in mediating antiproliferation of tumor growth and induction of apoptosis, which is a dominant subtype that mediates antitumor effects. All these results reveal a close relationship between endocrine and immunity.

Among these five receptors, SSTR4 has come to the forefront as a potential mediator of central nervous system pathology, inflammation, and even pain mechanisms. Targeting SSTR4 has the added advantage of limiting pituitary secretion without inhibiting secretion of glucagon, growth hormone, or insulin. In the central nervous system, SSTR4 is expressed at relatively high levels in the hippocampus and neocortex, memory and learning regions, and Alzheimer's disease pathology. Recent studies have indeed shown that SSTR4 agonists can improve learning and memory in rodent models of Alzheimer's disease, which corresponds to reduced levels of β-amyloid. In addition, studies also found that SSTR4 receptor stimulation can dose-dependently enhance cued memory, and thus may have direct cognitive-enhancing activity. Other studies have shown that SSTR4 binding to K⁺ ion channels can modulate hippocampal excitability, which has implications for the treatment of certain forms of epilepsy using SSTR4 agonists. Additionally, the effects of SSTR4 agonists are potent in rodent models of pain associated with acute and chronic anti-peripheral nociceptive and anti-inflammatory activity. Recent data shows that SRIF released by nociceptors expressed by a capsaicin-sensitive receptor TRPV1 acts on SSTR4 and SSTR2 to produce antinociceptive effects.

Pain is the most common and most troublesome symptom in clinical practice, and is also one of main reasons patients seek medical treatment. According to the duration of pain, pain can be divided into acute pain and chronic pain. The acute pain includes pain caused by tissue damage and postoperative inflammation; the chronic pain includes nociceptive pain, neuropathic pain, visceral pain, and mixed pain. At present, drugs bearing the burden of pain treatment are still well-known analgesics, including narcotic analgesics (lidocaine, etc.), opioids, and non-steroidal anti-inflammatory drugs (NSAIDs). Drugs having novel mechanisms of action have also been included to the ranks of analgesics, such as antidepressants and anticonvulsants. Although many patients can benefit from existing analgesics, these drugs only provide adequate relief of symptoms in a quarter of patients. Furthermore, existing drugs usually have problems such as low tolerance, severe toxic side effects, poor long-term safety, potential drug abuse, and inconvenience in use, and patients therefore urgently desire safer and more effective analgesics. The use of SSTR4 agonists for analgesia has received increasing attention, and the development of novel SSTR4 agonists has broad application prospects and is also urgently desired.

CN 105473574 A discloses the following compound of Formula (I), which is an agonist of SSTR4, and applicable to the prevention or treatment of medical conditions associated with SSTR4. However, there is still a broad demand in the art for SSTR4 agonists having good efficacy, high bioavailability, and good solubility.

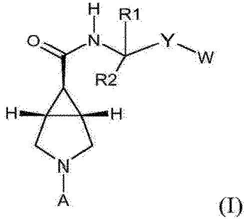

### SUMMARY

In view of this, it is necessary to provide a compound which belongs to a class of somatostatin receptor subtype 4 (SSTR4) agonist compounds having novel structures, improved efficacy, high bioavailability, and improved solubility. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

Specific technical solutions are as follows: The invention is defined exclusively by the scope of the claims. Accordingly, only compounds falling within the scope of formula (I) form part of the invention. Prodrugs and metabolites that do not fall under this definition are not covered. Any references in the subsequent paragraphs to "prodrugs" or "metabolites" that do not fall within the scope of compounds of formula (I) are therefore to be disregarded.

A compound of Formula (I), or a stereoisomer, an N-oxide, a hydrate, a solvate, a pharmaceutically acceptable salt, a polymorph thereof: 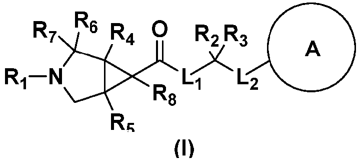
wherein R₁ is selected from -H and a C₁₋₆ alkyl;
L₁ is selected from -NH- and -O-;
R₂ and R₃ are each independently selected from -H, a C₁₋₆ alkyl, and a C₃₋₆ cycloalkyl, wherein R₂ and R₃ are not both -H; or R₂ and R₃ together form a 3- to 6-membered saturated cyclic group comprising 0 to 1 group selected from -O-, -NR₉-, -SO-, and -SO₂-;
R₄, R₅, R₆, R₇, and R₈ are each independently selected from -H, a C₁₋₆ alkyl, a C₁₋₆ alkoxy, a C₁₋₆ alkoxy C₁₋₆ alkyl, a -(CH₂)ₘ-C₃₋₁₀ carbocyclyl, a -(CH₂)ₘ-(3- to 10-membered heterocyclyl), a -(CH₂)ₘ-O-C₃₋₁₀ carbocyclyl, a -(CH₂)ₘ-O-(3- to 10-membered heterocyclyl), phenyl, and a 5- to 6-membered heteroaryl, the heterocyclyl or the heteroaryl contains 1 to 4 heteroatoms selected from N, O, and S, and the alkyl, the alkoxy, the carbocyclyl, the phenyl, the heteroaryl, or the heterocyclyl in R₄, R₅, R₆, R₇, and R₈ is each independently optionally further substituted with 0 to 4 substituents selected from -H, -F, -Cl, -Br, -I, hydroxy, sulfydryl, cyano, amino, a C₁₋₄ alkyl, and a C₁₋₄ alkoxy;
R₉ is selected from -H, a C₁₋₆ alkyl, a C₁₋₄ alkoxy C₁₋₆ alkyl, a halogen, hydroxy, cyano, and a C₃₋₆ cycloalkyl;
A is selected from a C₆₋₁₄ aryl, a 5- to 14-membered heteroaryl, a 5- to 14-membered heterocyclyl, and a 5- to 14-membered cycloalkyl, and the aryl, the heteroaryl, the heterocyclyl, and the cycloalkyl in A are optionally further substituted with 1 to 4 R₁₀, wherein the heteroaryl or the heterocyclyl contains 1 to 4 heteroatoms selected from N, O, and S;
when L₂ is selected from a single bond, -(CRₐR_{b})ₙ-, and -(CRₐR_{b})ₙO-, R₁₀ is independently selected from -H, -F, -Cl, -Br, -I, hydroxy, cyano, amino, a C₁₋₆ alkyl, a C₁₋₄ alkoxy C₁₋₆ alkyl, a C₃₋₆ cycloalkyl, and -SR₁₁, and at least one R₁₀ is -SR₁₁, wherein R₁₁ is each independently selected from -H, a C₁₋₆ alkyl, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a C₆₋₁₀ aryl, and a 5- to 6-membered heteroaryl, the heterocyclyl and the heteroaryl contain 1 to 4 heteroatoms selected from N, O, and S, and the alkyl, the alkoxy, the aryl, the heteroaryl, the carbocyclyl, or the heterocyclyl is each independently optionally further substituted with 0 to 4 substituents selected from -H, a halogen, hydroxy, cyano, a C₁₋₄ alkyl, and a C₁₋₄ alkoxy;
when L₂ is selected from -(CRₐR_{b})ₙS-, R₁₀ is independently selected from -H, -F, -Cl, -Br, -I, hydroxy, cyano, amino, a C₁₋₄ alkyl, a C₁₋₄ alkoxy, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -O-(CH₂)ₙ-C₃₋₁₀ carbocyclyl or -O-(CH₂)ₙ-(3- to 10-membered heterocyclyl), and -SR₁₁, wherein R₁₁ is each independently selected from -H, a C₁₋₆ alkyl, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3-to 10-membered heterocyclyl), a C₆₋₁₀ aryl, and a 5- to 6-membered heteroaryl, the heterocyclyl or the heteroaryl contains 1 to 4 heteroatoms selected from N, O, and S, and the alkyl, the alkoxy, the aryl, the heteroaryl, the carbocyclyl, or the heterocyclyl is each independently optionally further substituted with 0 to 4 substituents selected from H, a halogen, hydroxy, cyano, a C₁₋₄ alkyl, and a C₁₋₄ alkoxy;
Rₐ and R_{b} are each independently selected from -H and a C₁₋₆ alkyl;
m is independently selected from 0, 1, 2, and 3 at each occurrence;
n is independently selected from 0, 1, 2, and 3 at each occurrence.

The present invention further provides a pharmaceutical composition comprising a therapeutically effective dose of the compound or the stereoisomer, the N-oxide, the hydrate, the solvate, the metabolite, the pharmaceutically acceptable salt, the polymorph, or the prodrug thereof as described above, and a pharmaceutically acceptable carrier or excipient.

The present invention further provides an application of the compound or the stereoisomer, the N-oxide, the hydrate, the solvate, the metabolite, the pharmaceutically acceptable salt, the polymorph, or the prodrug thereof as described above or the pharmaceutical composition as described above in the preparation of a drug for treatment and/or prevention of a disease or a symptom affected by SSTR4 activation.

The present invention further provides an application of the compound or the stereoisomer, the N-oxide, the hydrate, the solvate, the metabolite, the pharmaceutically acceptable salt, the polymorph, or the prodrug thereof as described above or the pharmaceutical composition as described above in the preparation of a drug for treatment and/or prevention of pain.

Compared with the prior art, the present invention has one or more of the following beneficial effects:
Regarding the compound provided by the present invention, sulfur is introduced at a specific site of the core structure, such that a novel compound obtained thereby has an excellent SSTR4 agonistic effect, and/or has better selectivity for SSTR4 than other SSTRs, and can be used as a SSTR4 agonist to prevent and/or treat diseases or conditions affected by SSTR4 activation, for example, Alzheimer's disease and other CNS disorders such as epilepsy and depression, and can also be used to treat pain and/or inflammation of various origins. In addition, experimental studies have shown that the compound of the present invention has high metabolic stability, and/or excellent pharmacokinetics, and/or excellent pharmacodynamic effects.

### BRIEF DESCRIPTION OF DRAWINGS

FIG. 1 Results of a single-dose pharmacodynamic study in a rat CFA pain model
FIG. 2 Results of a multi-dose pharmacodynamic study in a rat CFA pain model

### DETAILED DESCRIPTION

The compound and the preparation method, pharmaceutical composition, and application thereof of the present invention will be described in further detail below with reference to specific examples. The present invention may be implemented in many different forms and is not limited to the embodiments described herein. On the contrary, these embodiments are provided for the purpose of providing people with a thorough and complete understanding of the present disclosure.

Unless otherwise defined, all technical and scientific terms used herein have the meaning commonly understood by one of ordinary skill in the art. The terms used herein in the description are for the purpose of describing particular embodiments only and are not intended to limit the present invention. As used herein, the term "and/or" means any and all combinations of one or more of associated listed items.

Unless stated to the contrary, terms used in the description and claims have the following meanings.

Elements involved in the groups and compounds described in the present invention include carbon, hydrogen, oxygen, sulfur, nitrogen, or halogens, including their isotopes. Further, the elements carbon, hydrogen, oxygen, sulfur, or nitrogen involved in the groups and compounds of the present invention are optionally further replaced by one or more of their corresponding isotopes, in which the isotopes of carbon include ¹²C, ¹³C, and ¹⁴C, the isotopes of hydrogen include protium (H), deuterium (D, also known as heavy hydrogen), and tritium (T, also known as super heavy hydrogen), the isotopes of oxygen include ¹⁶O, ¹⁷O, and ¹⁸O, and the isotopes of sulfur include ³²S, ³³S, ³⁴S, and ³⁶S, the isotopes of nitrogen include ¹⁴N and ¹⁵N, the isotopes of fluorine include ¹⁹F, the isotopes of chlorine include ³¹Cl, ³⁶Cl, and ³⁷Cl, and the isotopes of bromine include ⁷⁹Br and ⁸¹Br.

The term "alkyl" refers to a saturated straight or branched chain aliphatic hydrocarbon group, specifically a saturated hydrocarbon containing a primary (normal) carbon atom, a secondary carbon atom, a tertiary carbon atom, a quaternary carbon atom, or a combination thereof. Phrases containing this term, for example, "C₁₋₆ alkyl" refers to an alkyl containing 1 to 6 carbon atoms, and may independently be a C₁ alkyl, a C₂ alkyl, a C₃ alkyl, a C₄ alkyl, a C₅ alkyl, or a C₆ alkyl at each occurrence. One of embodiments may comprise an alkyl having 1 to 20 carbon atoms, preferably an alkyl containing 1 to 10 carbon atoms, and more preferably an akyl having 1 to 4 carbon atoms. Non-limiting examples include: methyl, ethyl, 1-propyl, 2-propyl (i-Pr, i-propyl, -CH(CH₃)₂), 1-butyl (n-Bu, n-butyl, -CH₂CH₂CH₂CH₃), 2-methyl-propan-1-yl (i-Bu, i-butyl, -CH₂CH(CH₃)₂), 2-butyl (s-Bu, s-butyl, -CH(CH₃)CH₂CH₃), 2-methyl-propan-2-yl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-butan-2-yl (-C(CH₃)₂CH₂CH₃), 3-methyl-butan-2-yl (-CH(CH₃)CH(CH₃)₂), 3-methyl-butan-1-yl (-CH₂CH₂CH(CH₃)₂), 2-methyl-butan-1-yl (-CH₂CH(CH₃)CH₂CH₃), 1-hexyl (-CH₂CH₂CH₂CH₂CH₂CH₃), hex-2-yl (-CH(CH₃)CH₂CH₂CH₂CH₃), hex-3-yl (-CH(CH₂CH₃)(CH₂CH₂CH₃)), 2-methyl-pentan-2-yl (-C(CH₃)₂CH₂CH₂CH₃), 3-methyl-pentan-2-yl (-CH(CH₃)CH(CH₃)CH₂CH₃), 4-methyl-pentan-2-yl (-CH(CH₃)CH₂CH(CH₃)₂), 3-methyl-pentan-3-yl (-C(CH₃)(CH₂CH₃)₂), 2-methyl-pentan-3-yl (-CH(CH₂CH₃)CH(CH₃)₂), 2,3-dimethyl-butan-2-yl (-C(CH₃)₂CH(CH₃)₂), 3,3-dimethyl-butan-2-yl (-CH(CH₃)C(CH₃)₃, octyl (-(CH₂)₇CH₃), and n-nonyl, and various branched isomers thereof. The alkyl may be substituted or unsubstituted, and when substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Alkoxy" refers to an -O-alkyl, where the alkyl is as defined above herein and is preferably an alkyl having 1 to 12 carbon atoms. Phrases containing this term, for example, "C₁₋₄ alkoxy" refer to an -O-alkyl whose alkyl moiety contains 1 to 4 carbon atoms. At each occurrence, the C₁₋₄ alkoxy may be independently a C₁ alkoxy, a C₂ alkoxy, a C₃ alkoxy, or a C₄ alkoxy. The alkoxy may be substituted or unsubstituted, and non-limiting examples thereof include, methoxy, ethoxy, propoxy, isopropoxy, butoxy, t-butoxy, pentyloxy, or hexyloxy. When the alkoxy is substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Alkoxyalkyl" refers to an alkyl substituted with an alkoxy as described above. Phrases containing this term, for example, "C₁₋₄ alkoxy C₁₋₆ alkyl" refers to a C₁₋₆ alkyl substituted with a C₁₋₄ alkoxy, and may be independently a C₁ alkoxy C₁₋₆ alkyl, a C₂ alkoxy C₁₋₆ alkyl, a C₃ alkoxy C₁₋₆ alkyl, or a C₄ alkoxy C₁₋₆ alkyl at each occurrence. The alkoxyalkyl may be substituted or unsubstituted. Non-limiting examples of alkoxyalkyls include, methoxymethyl, methoxyethyl, ethoxymethyl, ethoxyethyl, propoxymethyl, propoxyethyl, isoproxymethyl, butoxypropyl, t-butoxyethyl, pentoxyethyl, hexyloxyethyl, cyclopropoxymethyl, cyclopropoxyethyl, cyclopropoxypropyl, or cyclohexyloxymethyl; when substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Alkenyl" is an alkyl as defined herein containing at least one carbon-carbon double bond. In one example, the alkenyl contains 2 to 20 carbon atoms, preferably 2 to 12 carbon atoms, more preferably 2 to 8 carbon atoms, still more preferably 2 to 6 carbon atoms. Non-limiting examples of alkenyls include substituted or unsubstituted vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, or 4-decenyl, etc. When the alkenyl is substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Alkynyl" is an alkyl as defined herein containing at least one carbon-carbon triple bond. In one example, the alkynyl contains 2 to 20 carbon atoms, preferably 2 to 12 carbon atoms, more preferably 2 to 8 carbon atoms, and still more preferably 2 to 6 carbon atoms. Non-limiting examples of alkynyls include substituted or unsubstituted ethynyl, 1-propynyl, 2-propynyl, 1-butynyl, 2-butynyl, 3-butynyl, 4-pentynyl, 3-pentynyl, 2-hexynyl, 3-hexynyl, 3-butynyl, 2-heptynyl, 3-heptynyl, 4-heptynyl, 3-octynyl, 3-heptynyl, or 4-decynyl, etc. When the alkyl is substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Carbocyclyl" or "cycloalkyl" refers to a saturated or partially unsaturated cyclic carbon-containing group. In one of embodiments, the carbocyclyl is a 3- to 6-membered monocyclic, 3- to 8-membered monocyclic, 3- to 10-membered monocyclic, a 4- to 12-membered bicyclic, or 10- to 15-membered tricyclic ring system. The carbocycle include bridged or spiro rings. Non-limiting examples of carbocyclyls include cyclopropyl, cyclobutyl, cyclopentyl, cyclohexyl, cyclohexenyl, cycloheptyl, cyclopentenyl, cyclohexadienyl, cycloheptatrienyl, benzocyclopentyl, bicyclo[3.2.1]octyl, bicyclo[5.2.0]nonyl, tricyclo[5.3.1.1]dodecyl, adamantyl, or spiro[3.3]heptyl, et al. The carbocyclyls may be optionally substituted. When the carbocyclyl is substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Heterocyclyl" or "heterocycle" refers to a substituted or unsubstituted saturated or partially unsaturated cyclic group containing a heteroatom selected from N, O, and S. In one of embodiments, the heterocyclyl may be a 3- to 8-membered monocyclic, 4- to 12-membered bicyclic, or 10- to 15-membered tricyclic ring system, preferably a 3- to 10-membered heterocyclyl, and preferably contains at least 1 to 4 heteroatoms selected from N, O, or S. The heteroatom N or S in the heterocycle may be oxidized to various oxidation states to form, for example, N-oxides. The heterocycle can be attached to other moieties of the molecule via a heteroatom or a carbon atom. The heterocycle includes bridged or spiro rings. Non-limiting examples of heterocycles include, ethylene oxide, aziridinyl, oxetanyl, azetidinyl, 1,3-dioxolane, 1,4-dioxane, 1,3-dioxane, azepanyl, pyranyl, piperidinyl, morpholinyl, thiomorpholinyl, 1,3-dithiane, dihydrofuran, dihydropyran, dithiolanyl, tetrahydrofuranyl, tetrahydropyrrolyl, tetrahydroimidazolyl, tetrahydrothiazolyl, tetrahydropyranyl, dihydrobenzofuran, dihydropyridyl, tetrahydrothienyl, sulfur-oxidized tetrahydrothienyl, tetrahydroquinolinyl, tetrahydroisoquinolinyl, indolyl, etc. When the heterocycle is substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Aryl" refers to a substituted or unsubstituted all-carbon monocyclic or fused polycyclic unsaturated group having a conjugated π electron system. In one of embodiments, the aryl is a 6- to 14-membered aromatic ring, preferably a 6- to 10-membered aromatic ring. Non-limiting examples thereof include phenyl or naphthyl; the aryl may be fused to a heteroaryl, a heterocyclyl, or a cycloalkyl, and the site of attachment to the moiety of the molecule is on the aryl. Non-limiting examples of aryls include benzofuran, benzocyclopentyl, or benzothiazole, etc. When the aryl is substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Heteroaryl" refers to a substituted or unsubstituted monocyclic or fused polycyclic unsaturated group containing at least one heteroatom selected from N, O, and S. In one of embodiments, the heteroaryl is a 5- to 15-membered heteroaryl, a 5- to 14-membered heteroaryl, or preferably a 5- to 10-membered heteroaryl, or more preferably a 5- to 6-membered heteroaryl, where the number of heteroatoms is 1 to 4, preferably 1 to 3, more preferably 1 to 2. Non-limiting examples of heteroaryls include pyridyl, furyl, thienyl, *N-*alkylpyrrolyl, pyrimidinyl, pyrazinyl, pyridazinyl, imidazolyl, benzofuran, benzimidazole, benzopyridine, or pyrrolopyridine, etc. When the heteroaryl is substituted, there are preferably 1 to 5 substituents, and the substituents are independently selected from F, Cl, Br, I, =O, an alkyl, an alkenyl, an alkynyl, an alkoxy, hydroxy, nitro, cyano, and amino.

"Heteroalkyl" refers to a group in which at least one carbon atom is replaced by a non-carbon atom on the basis of an alkyl, and the non-carbon atom may be an N atom, an O atom, and/or an S atom, etc. For example, if a carbon atom in an alkyl attached to a core structure is replaced by a non-carbon atom, the resulting heteroalkyl is an alkoxy (e.g., -OCH₃, etc.), an alkylamino (e.g., -NHCH₃, -N(CH₃)₂, etc.), or an alkylthio (e.g., -SCH₃), respectively. If the carbon atom in the alkyl attached to the core structure is not replaced by a non-carbon atom and the heteroatom is embedded within the group, the resulting heteroalkyl group is an alkyloxyalkyl (e.g., -CH₂CH₂-O-CH₃, etc.), an alkylaminoalkyl (e.g., -CH₂NHCH₃, -CH₂N(CH₃)₂, etc.), or an alkylthioalkyl (e.g., -CH₂-S-CH₃), respectively. If a terminal carbon atom of the alkyl is replaced by a non-carbon atom, the resulting heteroalkyl is a hydroxyalkyl (e.g., -CH₂CH₂-OH), an aminoalkyl (e.g., -CH₂NH₂), or a mercaptoalkyl (e.g., -CH₂CH₂-SH), respectively.

"Amino" refers to a derivative of ammonia having structural features of formula -N(X)₂ or formula -NR'R", where each of "X", R', and R" is independently H, a substituted or unsubstituted alkyl, a substituted or unsubstituted cycloalkyl, a substituted or unsubstituted heterocyclyl, a substituted or unsubstituted aryl, or a substituted or unsubstituted heteroaryl. Non-limiting types of aminos include -NH₂, -*N*(alkyl)₂, -NH(alkyl), -*N*(cycloalkyl)₂, - NH(cycloalkyl), -*N*(heterocyclyl)₂, -NH(heterocyclyl), -*N*(aryl)₂, -NH(aryl), -N(alkyl)(aryl), - N(alkyl)(heterocyclyl), -N(cycloalkyl)(heterocyclyl), -N(aryl)(heteroaryl), - N(alkyl)(heteroaryl), etc.

"Halogen" means F, Cl, Br, or I. "Halogenated" refers to replacement of one or more hydrogen atoms in a molecule or group with a halogen selected from F, Cl, Br, or I.

"Pharmaceutically acceptable salt" refers to a pharmaceutically acceptable salt of a non-toxic acid or base, including salts formed with inorganic acids or bases, or salts formed with organic acids and organic bases. Salts derived from inorganic bases include, but are not limited to metal salts formed with Al, Ca, Li, Mg, K, Na, and Zn; salts derived from organic bases include, but are not limited to salts formed with primary, secondary, or tertiary amines. The primary, secondary, or tertiary amines include naturally occurring substituted or unsubstituted amines, cyclic amines, and basic ion exchange resins, for example, ammonia, isopropylamine, trimethylamine, diethylamine, triethylamine, tripropylamine, diethanolamine, ethanolamine, dimethylethanolamine, 2-dimethylaminoethanol, 2-diethylaminoethanol, dicyclohexylamine, caffeine, procaine, choline, betaine, benethamine penicillin, ethylenediamine, glucosamine, methylglucamine, theobromine, triethanolamine, tromethamine, purine, piperazine, piperidine, N-ethylpiperidine, or polyamine resins; the salts derived from inorganic acids and organic acids include, but are not limited to, salts formed with sulfuric acid, phosphoric acid, nitric acid, hydrobromic acid, hydrochloric acid, formic acid, acetic acid, propionic acid, benzenesulfonic acid, benzoic acid, phenylacetic acid, salicylic acid, alginic acid, anthranilic acid, camphoric acid, citric acid, vinyl sulfonic acid, formic acid, fumaric acid, furoic acid, gluconic acid, glucuronic acid, glutamic acid, glycolic acid, isethionic acid, lactic acid, maleic acid acid, malic acid, mandelic acid, mucic acid, pamoic acid, pantothenic acid, stearic acid, succinic acid, sulfanilic acid, tartaric acid, p-toluenesulfonic acid, malonic acid, 2-hydroxypropionic acid, oxalic acid, glycolic acid, glucuronic acid, galacturonic acid, citric acid, lysine, arginine, aspartic acid, cinnamic acid, p-toluenesulfonic acid, methanesulfonic acid, ethanesulfonic acid, or trifluoromethanesulfonic acid, etc.

"Stereoisomer" refer to an isomer resulting from different arrangements of atoms in a molecule in space, including *cis-trans* isomers, enantiomers, and conformational isomers.

"Pharmaceutical composition" means a mixture of one or more of the compounds described herein, or a physiologically/pharmaceutically acceptable salt or prodrug thereof, and other chemical components. The other components are, for example, physiological/pharmaceutically acceptable carriers or excipients. The purpose of the pharmaceutical composition is to facilitate the administration of a compound to an organism.

"Prodrug" refers to a substance that can be converted to a biologically active compound of the present invention under physiological conditions or by degradation. The prodrug of the present invention are prepared by modifying a functional group in the compound, and the modification can be removed according to conventional procedures or removed *in vivo* to obtain the parent compound. The prodrugs include compounds in which hydroxy, amino, or mercapto in the compounds of the present invention is attached to any group. When a prodrug of a compound of the present invention is administered to a mammalian subject, the prodrug is cleaved to form a free hydroxy group, a free amino group, or a free mercapto group, respectively. Examples of prodrugs include, but are not limited to, compounds formed by hydroxy or amino functional groups in the compounds of the present invention with formic acid, acetic acid, or benzoic acid.

"Optional" or "optionally" means that a subsequently described event or circumstance may, but not necessarily, occur, including cases where the event or circumstance does or does not occur. For example, "an aryl is optionally substituted with an alkyl" means that the alkyl may, but not necessarily, be present, and the term includes instances where the aryl is substituted with an alkyl and instances where the aryl is not substituted with an alkyl.

"Pharmaceutically acceptable carrier" refers to a pharmaceutically acceptable material, composition, or agent, such as a liquid or solid filler, a diluent, an excipient, a solvent, or an encapsulating material. As used herein, the term "pharmaceutically acceptable carrier" includes buffers, sterile water for injection, solvents, dispersion media, coatings, antibacterial and antifungal agents, isotonicity agents, and absorption retarders, etc. compatible with pharmaceutical administration. Each carrier needs to be "pharmaceutically acceptable" in the sense of being compatible with other ingredients in a formulation and not harmful to a patient. Suitable examples include, but are not limited to: (1) sugars such as lactose, glucose, and sucrose; (2) starches such as corn starch, potato starch, and substituted or unsubstituted β-cyclodextrins; (3) cellulose and derivatives thereof, such as sodium carboxymethyl cellulose, ethyl cellulose, and cellulose acetate; (4) powdered tragacanth; (5) malt; (6) gelatin; (7) talc; (8) excipients such as cocoa butter and suppository wax; (9) oils such as peanut oil, cottonseed oil, safflower oil, sesame oil, olive oil, corn oil, and soybean oil; (10) glycols such as propylene glycol; (11) polyhydric alcohols such as glycerol, sorbitol, mannitol, and polyethylene glycol; (12) esters such as ethyl oleate and ethyl laurate; (13) agar; (14) buffers such as magnesium hydroxide and hydrogen; (15) alginic acid; (16) pyrogen-free water; (17) isotonic saline; (18) Ringer's solution; (19) ethanol; (20) phosphate buffer; and (21) other non-toxic compatible substances used in drug formulations.

The term "solvate" refers to a solvent-bound compound usually formed by a solvolysis reaction or a salt form thereof. This physical association may include hydrogen bonding. Common solvents include water, methanol, ethanol, acetic acid, DMSO, THF, diethyl ether, etc. The compounds described herein can be prepared, e.g., in crystalline forms, and can be solvated. Suitable solvates include pharmaceutically acceptable solvates and further include stoichiometric and non-stoichiometric solvates. In some cases, the solvate will be capable of being isolated, for example, when one or more solvent molecules are incorporated into crystal lattices of a crystalline solid. The term "solvate" includes solvates in solution and isolatable solvates. Representative solvates include hydrates, ethanolates, and methanolates.

The term "metabolite" refers to a substance including a product of *in vivo* metabolism of a compound of the present invention, including intermediates and final metabolites.

The term "polymorph" refers to a crystalline form of a compound (or a salt, hydrate, or solvate thereof) of a particular crystal packing arrangement. All polymorphs have the same elemental composition. Different crystalline forms typically have different X-ray diffraction patterns, infrared spectra, melting points, densities, hardness, crystal shapes, optoelectronic properties, stability, and solubility. Recrystallization solvent, rate of crystallization, storage temperature, and other factors can cause one crystalline form to dominate. Various polymorphs of a compound can be prepared by crystallization under different conditions.

The dosage form and administration route of the compounds of the present invention or the pharmaceutical compositions thereof are not particularly limited.

Representative administration routes include, but are not limited to, oral, intratumoral, rectal, parenteral (intravenous, intraperitoneal, intramuscular, or subcutaneous) injection and/or topical administration.

Solid dosage forms for oral administration include capsules, tablets, pills, powders, and granules. In these solid dosage forms, active compounds are mixed with at least one conventional inert excipient (or carrier), such as sodium citrate or dicalcium phosphate, or with (a) fillers or compatibilizers such as starch, lactose, sucrose, glucose, mannitol, and silicic acid; (b) binders such as hydroxymethylcellulose, alginate, gelatin, polyvinylpyrrolidone, sucrose, and acacia; (c) humectants such as glycerol; (d) disintegrants such as agar, calcium carbonate, potato or tapioca starch, alginic acid, complex silicates, and sodium carbonate; (e) solvents such as paraffin; (f) absorption accelerators such as quaternary amine compounds; (g) wetting agents such as cetyl alcohol and glyceryl monostearate; (h) adsorbents such as kaolin; and (i) lubricants such as talc, calcium stearate, magnesium stearate, solid polyethylene glycol, sodium lauryl sulfate, or mixtures thereof.

Liquid dosage forms for oral administration include pharmaceutically acceptable emulsions, solutions, suspensions, syrups, or tinctures. In addition to the active compounds, liquid dosage forms can contain inert diluents (e.g., water or other solvents), solubilizers, and emulsifiers conventionally used in the art. Specific examples are, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, and sesame oil, or mixtures of these substances. Besides inert diluents, the compositions may further contain adjuvants such as wetting agents, suspending agents, sweeteners, flavors, and perfuming agents. For example, suspensions may contain suspending agents. Specific examples are, for example, ethoxylated isostearyl alcohol, polyoxyethylene sorbitol and sorbitan esters, microcrystalline cellulose, aluminum methoxide and agar, or mixtures thereof.

Compositions for parenteral injection may include physiologically acceptable sterile aqueous or anhydrous solutions, dispersions, suspensions, or emulsions, and sterile powders for reconstitution into sterile injectable solutions or dispersions. Suitable aqueous or non-aqueous carriers, diluents, solvents, or excipients are selected from water, ethanol and polyols, or suitable mixtures thereof.

Dosage forms for topical administration include ointments, powders, patches, sprays, and inhalants, which are prepared by mixing active ingredients with a pharmaceutically acceptable carrier together with a preservative, a buffer, and/or a propellant as required under sterile conditions.

The present invention relates to the following embodiments.

In one embodiment, the present invention relates to a compound of Formula (I), or a stereoisomer, an *N*-oxide, a hydrate, a solvate, a metabolite, a pharmaceutically acceptable salt, a polymorph, or a prodrug thereof: 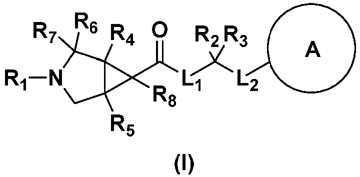
wherein R₁ is selected from -H and a C₁₋₆ straight or branched chain alkyl;
L₁ is selected from -NH- and -O-;
R₂ and R₃ are each independently selected from -H, a C₁₋₆ straight or branched chain alkyl, and a C₃₋₆ cycloalkyl, wherein R₂ and R₃ are not both -H; or R₂ and R₃ together form a 3- to 6-membered saturated cyclic group comprising 0 to 1 group selected from -O-, -NR₉-, -SO-, and -SO₂-;
R₄, R₅, R₆, R₇, and R₈ are each independently selected from -H, a C₁₋₆ straight or branched chain alkyl, a C₁₋₆ alkoxy, a C₁₋₆ alkoxy C₁₋₆ alkyl, a -(CH₂)ₘ-C₃₋₁₀ carbocyclyl, a -(CH₂)ₘ-(3-to 10-membered heterocyclyl), a -(CH₂)ₘ-O-C₃₋₁₀ carbocyclyl, a -(CH₂)ₘ-O-(3- to 10-membered heterocyclyl), phenyl, and a 5- to 6-membered heteroaryl, the heterocyclyl or the heteroaryl contains 1 to 4 heteroatoms selected from N, O, and S, and the alkyl, the alkoxy, the carbocyclyl, the phenyl, the heteroaryl, or the heterocyclyl in R₄, R₅, R₆, R₇, and R₈ is each independently optionally further substituted with 0 to 4 substituents selected from -H, -F, -Cl, -Br, -I, hydroxy, sulfydryl, cyano, amino, a C₁₋₄ alkyl, and a C₁₋₄ alkoxy;
R₉ is selected from -H, a C₁₋₆ straight or branched chain alkyl, a C₁₋₄ alkoxy C₁₋₆ alkyl, a halogen, hydroxy, cyano, and a C₃₋₆ cycloalkyl;
A is selected from a C₆₋₁₄ aryl, a 5- to 14-membered heteroaryl, a 5- to 14-membered heterocyclyl, and a 5- to 14-membered cycloalkyl, and the aryl, the heteroaryl, the heterocyclyl, and the cycloalkyl in A are optionally further substituted with 1 to 4 R₁₀, wherein the heteroaryl or the heterocyclyl contains 1 to 4 heteroatoms selected from N, O, and S;
when L₂ is selected from a single bond, -(CRₐR_{b})ₙ-, and -(CRₐR_{b})ₙO-, R₁₀ is independently selected from -H, -F, -Cl, -Br, -I, hydroxy, cyano, amino, a C₁₋₆ straight or branched chain alkyl, a C₁₋₄ alkoxy C₁₋₆ alkyl, a C₃₋₆ cycloalkyl, and -SR₁₁, and at least one R₁₀ is -SR₁₁, wherein R₁₁ is each independently selected from -H, a C₁₋₆ straight or branched chain alkyl, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a C₆₋₁₀ aryl, and a 5- to 6-membered heteroaryl, the heterocyclyl and the heteroaryl contain 1 to 4 heteroatoms selected from N, O, and S, and the alkyl, the alkoxy, the aryl, the heteroaryl, the carbocyclyl, or the heterocyclyl is each independently optionally further substituted with 0 to 4 substituents selected from -H, a halogen, hydroxy, cyano, a C₁₋₄ alkyl, and a C₁₋₄ alkoxy;
when L₂ is selected from -(CRₐR_{b})ₙS-, R₁₀ is independently selected from -H, -F, -Cl, -Br, -I, hydroxy, cyano, amino, a C₁₋₄ alkyl, a C₁₋₄ alkoxy, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -O-(CH₂)ₙ-C₃₋₁₀ carbocyclyl or -O-(CH₂)ₙ-(3- to 10-membered heterocyclyl), and -SR₁₁, wherein R₁₁ is each independently selected from -H, a C₁₋₆ alkyl, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3-to 10-membered heterocyclyl), a C₆₋₁₀ aryl, and a 5- to 6-membered heteroaryl, the heterocyclyl or the heteroaryl contains 1 to 4 heteroatoms selected from N, O, and S, and the alkyl, the alkoxy, the aryl, the heteroaryl, the carbocyclyl, or the heterocyclyl is each independently optionally further substituted with 0 to 4 substituents selected from H, a halogen, hydroxy, cyano, a C₁₋₄ alkyl, and a C₁₋₄ alkoxy;
Rₐ and R_{b} are each independently selected from -H and a C₁₋₆ alkyl;
m is independently selected from 0, 1, 2, and 3 at each occurrence;
n is independently selected from 0, 1, 2, and 3 at each occurrence.

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, L₁ is -NH-; R₁ is -H.

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, the compound has structural features of Formula (VI): 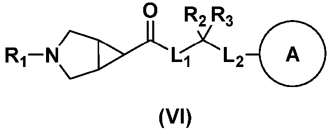 wherein
L₁ is selected from -NH- and -O-;
L₂ is selected from a single bond and -(CRₐR_{b})ₙ- or -(CRₐR_{b})ₙO-, wherein Rₐ and R_{b} are independently selected from -H and a C₁₋₆ straight or branched chain alkyl;
n is independently selected from 0, 1, 2, and 3 at each occurrence.

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, the compound has structural features of Formula (II): 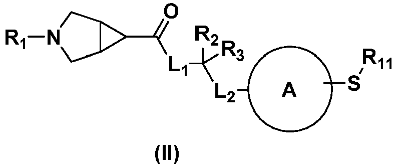 wherein
L₁ is selected from -NH- and -O-;
L₂ is selected from a single bond, -(CRₐR_{b})ₙ-, and -(CRₐR_{b})ₙO-, wherein Rₐ and R_{b} are each independently selected from -H and a C₁₋₆ straight or branched chain alkyl;
n is independently selected from 0, 1, 2, and 3 at each occurrence.

In one embodiment, in the foregoing compound of Formula (II), or the stereoisomer, the *N-*oxide, the hydrate, the solvate, the metabolite, the pharmaceutically acceptable salt, the polymorph, or the prodrug thereof provided by the present invention, R₁ is -H; L₁ is -NH-; L₂ is selected from a single bond, -CH₂-, -CH₂CH₂-, -CH₂CH₂CH₂-, -CH₂O-, and -CH₂CH₂O-.

In one embodiment, in the foregoing compound of Formula (II), or the stereoisomer, the *N-*oxide, the hydrate, the solvate, the metabolite, the pharmaceutically acceptable salt, the polymorph, or the prodrug thereof provided by the present invention, R₂ and R₃ are each independently selected from a C₁₋₆ straight or branched chain alkyl and a C₃₋₆ cycloalkyl; or R₂ and R₃ together form a 3- to 6-membered saturated cyclic group comprising 0 to 1 group selected from -O-, -SO-, and -SO₂-.

In one embodiment, in the foregoing compound of Formula (II), or the stereoisomer, the *N-*oxide, the hydrate, the solvate, the metabolite, the pharmaceutically acceptable salt, the polymorph, or the prodrug thereof provided by the present invention, R₁₁ is each independently selected from -H, a C₁₋₆ straight or branched chain alkyl, a C₁₋₄ alkoxy C₁₋₆ alkyl, a -(CH₂)ₙ-alkenyl, a -(CH₂)ₙ-alkynyl, a -(CH₂)ₙ-C₃₋₁₀ carbocyclyl, a -(CH₂)ₙ-(3- to 10-membered heterocyclyl), a C₆₋₁₀ aryl, and a 5- to 6-membered heteroaryl, the heterocyclyl or the heteroaryl contains 1 to 4 heteroatoms selected from N, O, and S, and the alkyl, the alkoxy, the aryl, the heteroaryl, the carbocyclyl, or the heterocyclyl is independently optionally further substituted with 0 to 4 substituents selected from -H, a halogen, hydroxy, cyano, a C₁₋₄ alkyl, and a C₁₋₄ alkoxy.

In one embodiment, in the foregoing compound of Formula (II), or the stereoisomer, the *N-*oxide, the hydrate, the solvate, the metabolite, the pharmaceutically acceptable salt, the polymorph, or the prodrug thereof provided by the present invention, R₁₁ is each independently selected from methyl, ethyl, isopropyl, trifluoromethyl, cyclopropyl, cyclobutyl, cyclopentyl, cyclohexyl, phenyl, imidazolyl, pyrrolyl, furyl, thiophenyl, and pyridyl.

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, the compound has structural features of Formula (III): 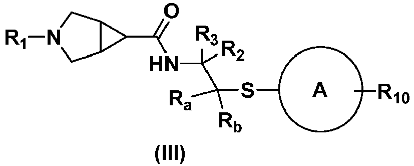
R₁₀ is each independently selected from -H, -F, -Cl, -Br, -I, hydroxy, cyano, amino, a C₁₋₄ alkyl, a C₁₋₄ alkoxy, a C₁₋₄ alkoxy C₁₋₆ alkyl, a -(CH₂)ₙ-alkenyl, a -(CH₂)ₙ-alkynyl, a -O-(CH₂)ₙ-C₃₋₁₀ carbocyclyl, a -O-(CH₂)ₙ-(3- to 10-membered heterocyclyl), a -O-(CH₂)ₙ-C₃₋₁₀ carbocyclyl, a - O-(CH₂)ₙ-(3- to 10-membered heterocyclyl), and -SR₁₁, wherein R₁₁ is each independently selected from -H, a C₁₋₆ straight or branched chain alkyl, a C₁₋₄ alkoxy C₁₋₆ alkyl, a -(CH₂)ₙ-alkenyl, a -(CH₂)ₙ-alkynyl, a -(CH₂)ₙ-C₃₋₁₀ carbocyclyl, a -(CH₂)ₙ-(3- to 10-membered heterocyclyl), a C₆₋₁₀ aryl, and a 5- to 6-membered heteroaryl, the heterocyclyl or the heteroaryl contains 1 to 4 heteroatoms selected from N, O, and S, and the alkyl, the alkoxy, the aryl, the heteroaryl, the carbocyclyl, or the heterocyclyl is each independently optionally further substituted with 0 to 4 substituents selected from -H, a halogen, hydroxy, cyano, a C₁₋₄ alkyl, and a C₁₋₄ alkoxy;
Rₐ and R_{b} are each independently selected from -H and a C₁₋₆ straight or branched chain alkyl.

In one embodiment, in the foregoing compound of Formula (III), or the stereoisomer, the *N-*oxide, the hydrate, the solvate, the metabolite, the pharmaceutically acceptable salt, the polymorph, or the prodrug thereof provided by the present invention, R₁₀ is each independently selected from -H, -F, -Cl, -Br, -I, hydroxy, cyano, amino, methyl, ethyl, isopropyl, trifluoromethyl, cyclopropyl, cyclobutyl, cyclopentyl, cyclobutoxy, tetrahydrofuranyl, and - SR₁₁, wherein R₁₁ is each independently selected from methyl, ethyl, isopropyl, trifluoromethyl, cyclopropyl, cyclobutyl, cyclopentyl, cyclohexyl, phenyl, imidazolyl, pyrrolyl, furyl, thienyl, and pyridyl; Rₐ and R_{b} are each independently selected from -H, methyl, ethyl, and isopropyl.

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, the compound has structural features of Formula (IV): 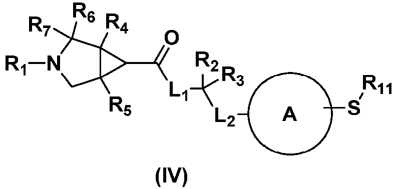 R₄ is selected from a C₁₋₆ straight or branched chain alkyl, a C₁₋₆ alkoxy, a C₁₋₆ alkoxy C₁₋₆ alkyl, a -(CH₂)ₘ-C₃₋₁₀ carbocyclyl, a -(CH₂)ₘ-(3- to 10-membered heterocyclyl), a -(CH₂)ₘ-O-C₃₋₁₀ carbocyclyl, a -(CH₂)ₘ-O-(3- to 10-membered heterocyclyl), phenyl, and a 5- to 6-membered heteroaryl, the heterocyclyl or the heteroaryl contains 1 to 4 heteroatoms selected from N, O, and S, and the alkyl, the alkoxy, the carbocyclyl, the phenyl, the heteroaryl, or the heterocyclyl in R₄ is each independently optionally further substituted with 0 to 4 substituents selected from -H, -F, -Cl, -Br, -I, hydroxy, sulfydryl, cyano, amino, a C₁₋₄ alkyl, and a C₁₋₄ alkoxy; m is independently selected from 0, 1, 2, and 3 at each occurrence.

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, the compound has structural features of Formula (V): 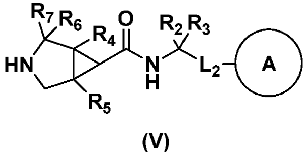
R₂ and R₃ are each independently selected from a C₁₋₆ straight or branched chain alkyl and a C₃₋₆ cycloalkyl, wherein R₂ and R₃ are not both -H; or R₂ and R₃ together form a 3- to 6-membered saturated cycloalkyl;
R₄, R₅, R₆, and R₇ are each independently selected from -H, a C₁₋₆ straight or branched chain alkyl, a C₁₋₆ alkoxy, and a C₁₋₆ alkoxy C₁₋₆ alkyl, and the alkyl and the alkoxy in R₄, R₅, R₆, and R₇ are each independently optionally further substituted with 0 to 4 substituents selected from -H, -F, -Cl, -Br, -I, hydroxy, sulfydryl, cyano, amino, a C₁₋₄ alkyl, and a C₁₋₄ alkoxy;
A is selected from a C₆₋₁₄ aryl, a 5- to 14-membered heteroaryl, a 5- to 14-membered heterocyclyl, and a 5- to 14-membered cycloalkyl, and the aryl, the heteroaryl, the heterocyclyl, and the cycloalkyl in A are optionally further substituted with 1 to 4 R₁₀, wherein the heteroaryl or the heterocyclyl contains 1 to 4 N atoms;
when L₂ is selected from a single bond, R₁₀ is independently selected from -H, -F, -Cl, -Br, -I, hydroxy, cyano, amino, a C₁₋₆ straight or branched chain alkyl, a C₁₋₄ alkoxy C₁₋₆ alkyl, a C₃₋₆ cycloalkyl, and -SR₁₁, and at least one R₁₀ is -SR₁₁, wherein R₁₁ is each independently selected from -H, a C₁₋₆ straight or branched chain alkyl, a C₁₋₄ alkoxy C₁₋₆ alkyl, a -(CH₂)ₙ-C₃₋₁₀ carbocyclyl, a -(CH₂)ₙ-(3- to 10-membered heterocyclyl), and a 5- to 6-membered heteroaryl, the heterocyclyl or the heteroaryl contains 1 to 4 heteroatoms selected from O, S, and N, and the alkyl, the alkoxy, the carbocyclyl, or the heterocyclyl is each independently optionally further substituted with 0 to 4 substituents selected from -H, a halogen, hydroxy, cyano, a C₁₋₄ alkyl, and a C₁₋₄ alkoxy;
when L₂ is selected from -(CRₐR_{b})ₙS-, R₁₀ is each independently selected from -H, -F, -Cl, -Br, -I, hydroxy, cyano, amino, a C₁₋₄ alkyl, a C₁₋₄ alkoxy, and a C₁₋₄ alkoxy C₁₋₆ alkyl, the alkyl and the alkoxy are each independently optionally further substituted with 0 to 4 substituents selected from H, a halogen, hydroxy, cyano, a C₁₋₄ alkyl, and a C₁₋₄ alkoxy;
Rₐ and R_{b} are each independently selected from -H and a C₁₋₆ straight or branched chain alkyl;
n is independently selected from 0, 1, 2, and 3 at each occurrence.

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, A is selected from the following structures:

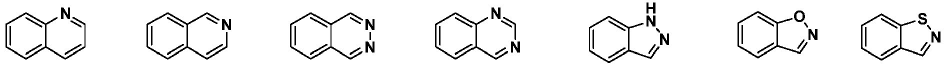

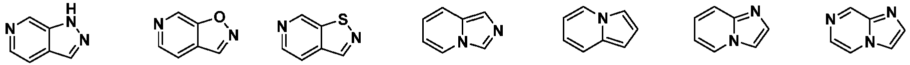

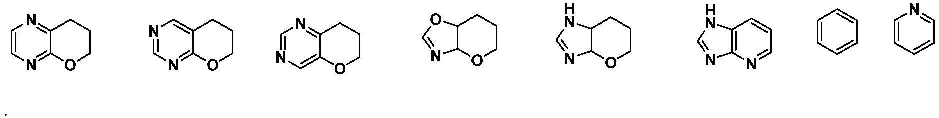

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, A is selected from one of the following structures:

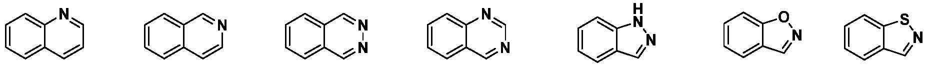

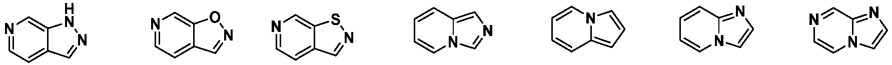

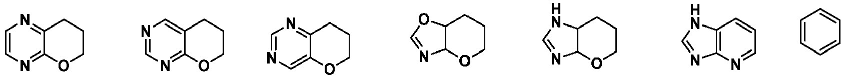

In one embodiment, in the foregoing compound of Formula (I), or the stereoisomer, the *N-*oxide, the hydrate, the solvate, the metabolite, the pharmaceutically acceptable salt, the polymorph, or the prodrug thereof provided by the present invention, A is selected from one of the following structures:

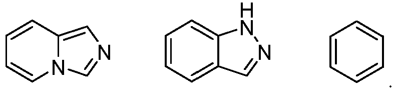

In one embodiment, the present invention provides a compound of Formula (a) or a stereoisomer, an *N*-oxide, a hydrate, a solvate, a metabolite, a pharmaceutically acceptable salt, a polymorph, or a prodrug thereof, 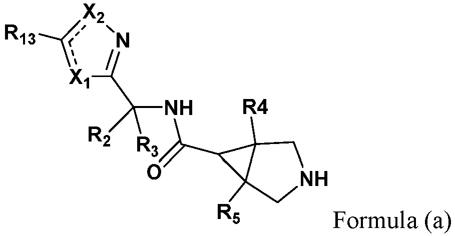 wherein
-̅ -̅ -̅ represents a single or double bond, provided that one and only one of two -̅ -̅ -̅ represents a double bond;
X₁ is CR₁₃ or NR₁₂;
X₂ is CR₁₃ or NR₁₂;
alternatively, X₁ and a substituent thereof together with an adjacent carbon atom and a substituent R₁₃ thereof form a benzene ring or a 5- to 6-membered heteroaromatic ring, and the benzene ring or the 5- to 6-membered heteroaromatic ring is optionally substituted with 1 to 4 R13;
R₁₃ is each independently selected from -H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁;
R₁₂ is selected from H, a C₁₋₆ alkyl, and a halogenated C₁₋₆ alkyl;
R₁₁ is selected from -H, a C₁₋₆ alkyl, a halogenated C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
R₂ and R₃ are each independently selected from H, a C₁₋₆ alkyl, a halogenated C₁₋₆ alkyl, and - (CH₂)ₙ-OR;
R₄ and R₅ are each independently selected from H, a C₁₋₆ alkyl, a halogenated C₁₋₆ alkyl, and - (CH₂)ₙ-OR;
n is 0, 1, 2, or 3,
provided that at least one R₁₃ is an -SR₁₁ substituent.

In one embodiment, in the foregoing compound of Formula (a), or the stereoisomer, the *N-*oxide, the hydrate, the solvate, the metabolite, the pharmaceutically acceptable salt, the polymorph, or the prodrug thereof provided by the present invention,
X₂ is CR₁₃;
X₁ and the substituent thereof together with the adjacent carbon atom and the substituent R₁₃ thereof form a benzene ring or a 5- to 6-membered heteroaromatic ring, and the benzene ring or the 5- to 6-membered heteroaromatic ring is substituted with 1 to 4 R₁₃;
R₁₃ is each independently selected from -H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁;
R₁₁ is selected from -H, a C₁₋₆ alkyl, a halogenated C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
R₂ and R₃ are each independently selected from H, a C₁₋₆ alkyl, a halogenated C₁₋₆ alkyl, and - (CH₂)ₙ-OR;
R₄ and R₅ are each independently selected from H, a C₁₋₆ alkyl, a halogenated C₁₋₆ alkyl, and - (CH₂)ₙ-OR;
n is 0, 1, 2, or 3,
provided that at least one R₁₃ is an -SR₁₁ substituent.

In one embodiment, in the foregoing compound of Formula (a), or the stereoisomer, the *N-*oxide, the hydrate, the solvate, the metabolite, the pharmaceutically acceptable salt, the polymorph, or the prodrug thereof provided by the present invention,
X₂ is NR₁₂;
X₁ and the substituent thereof together with the adjacent carbon atom and the substituent R₁₃ thereof form a benzene ring or a 5- to 6-membered heteroaromatic ring, and the benzene ring or the 5- to 6-membered heteroaromatic ring is substituted with 1 to 4 R₁₃;
R₁₃ is each independently selected from -H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁;
R₁₁ is selected from -H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
R₂ and R₃ are each independently selected from H, a C₁₋₆ alkyl, a halogenated C₁₋₆ alkyl, and - (CH₂)ₙ-OR;
R₄ and R₅ are each independently selected from H, a C₁₋₆ alkyl, a halogenated C₁₋₆ alkyl, and - (CH₂)ₙ-OR;
n is 0, 1, 2, or 3,
provided that at least one R₁₃ is -SR₁₁.

In one embodiment, the present invention provides a compound of Formula (b) or a stereoisomer, an *N*-oxide, a hydrate, a solvate, a metabolite, a pharmaceutically acceptable salt, a polymorph, or a prodrug thereof, 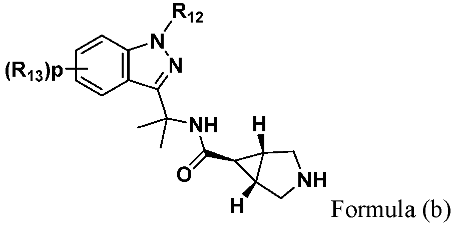 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁, and at least one R₁₃ is -SR₁₁;
R₁₂ is selected from H, a C₁₋₆ alkyl, and a halogenated C₁₋₆ alkyl;
R₁₁ is selected from H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R is selected from hydrogen, a C₁₋₆ alkyl, and a halogenated C₁₋₆ alkyl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
n is 0, 1, 2 or 3;
p is 1, 2, 3 or 4.

In one embodiment, in the foregoing compound of Formula (b), or the stereoisomer, the *N-*oxide, the hydrate, the solvate, the metabolite, the pharmaceutically acceptable salt, the polymorph, or the prodrug thereof provided by the present invention,
p is 1, 2, or 3;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁;
R₁₁ is selected from a C₁₋₆ alkyl, a halogenated C₁₋₆ alkyl, and a -(CH₂)ₙ-C₃₋₆ carbocyclyl;
R₁₂ is selected from H, a C₁₋₆ alkyl, or a halogenated C₁₋₆ alkyl;
n is 0 or 1.

In one embodiment, in the foregoing compound of Formula (b), or the stereoisomer, the *N-*oxide, the hydrate, the solvate, the metabolite, the pharmaceutically acceptable salt, the polymorph, or the prodrug thereof provided by the present invention,
p is 1 or 2;
R₁₃ is each independently selected from H, a C₁₋₆ alkyl, a halogenated C₁₋₆ alkyl, and -SR₁₁, and at least one R₁₃ is -SR₁₁;
R₁₁ is selected from a C₁₋₄ alkyl and a -(CH₂)ₙ-C₃₋₄ carbocyclyl;
R₁₂ is selected from H, a C₁₋₄ alkyl, and a halogenated C₁₋₄ alkyl;
n is 0 or 1.

In one embodiment, in the foregoing compound of Formula (b), or the stereoisomer, the *N-*oxide, the hydrate, the solvate, the metabolite, the pharmaceutically acceptable salt, the polymorph, or the prodrug thereof provided by the present invention,
P is 1;
R₁₃ is -SMe;
R₁₂ is H or a C₁₋₄ alkyl.

In one embodiment, the present invention provides a compound of Formula (b-1) or a stereoisomer, an *N*-oxide, a hydrate, a solvate, a metabolite, a pharmaceutically acceptable salt, a polymorph, or a prodrug thereof, 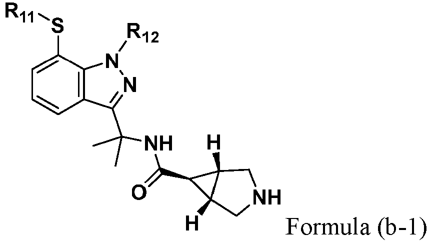 wherein
R₁₁ is selected from H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R₁₂ is selected from H, a C₁₋₆ alkyl, and a halogenated C₁₋₆ alkyl; and
n is 0, 1, 2, or 3.

In one embodiment, in the foregoing compound of Formula (b-1), or the stereoisomer, the *N-*oxide, the hydrate, the solvate, the metabolite, the pharmaceutically acceptable salt, the polymorph, or the prodrug thereof provided by the present invention,
R₁₁ is selected from a C₁₋₆ alkyl, a halogenated C₁₋₆ alkyl, and a -(CH₂)ₙ-C₃₋₆ carbocyclyl;
R₁₂ is selected from H, a C₁₋₆ alkyl, and a halogenated C₁₋₆ alkyl; and
n is 0 or 1.

In one embodiment, in the foregoing compound of Formula (b-1), or the stereoisomer, the *N-*oxide, the hydrate, the solvate, the metabolite, the pharmaceutically acceptable salt, the polymorph, or the prodrug thereof provided by the present invention,
R₁₁ is selected from a C₁₋₄ alkyl and a -(CH₂)ₙ-C₃₋₄ carbocyclyl;
R₁₂ is selected from H, a C₁₋₄ alkyl, and a halogenated C₁₋₄ alkyl;
n is 0 or 1.

In one embodiment, in the foregoing compound of Formula (b-1), or the stereoisomer, the *N-*oxide, the hydrate, the solvate, the metabolite, the pharmaceutically acceptable salt, the polymorph, or the prodrug thereof provided by the present invention,
R₁₁ is methyl;
R₁₂ is H or a C₁₋₄ alkyl.

In one embodiment, the present invention provides a compound of Formula (c) or a stereoisomer thereof of Formula (c-1) or Formula (c-2), an N-oxide, a hydrate, a solvate, a metabolite, a pharmaceutically acceptable salt, a polymorph, or a prodrug thereof, 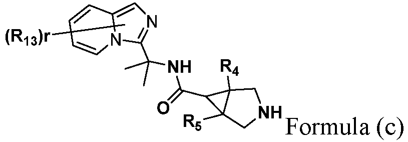 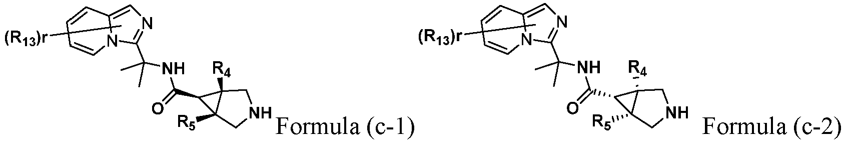 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁, and at least one R₁₃ is -SR₁₁;
R₁₁ is selected from H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R₄ and R₅ are each independently selected from H, a C₁₋₆ alkyl, a halogenated C₁₋₆ alkyl, and - (CH₂)ₙ-OR;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
r is 1, 2, 3, 4, or 5.

In one embodiment, in the foregoing compound of Formula (c) or the stereoisomer thereof of Formula (c-1) or Formula (c-2), the N-oxide, the hydrate, the solvate, the metabolite, the pharmaceutically acceptable salt, the polymorph, or the prodrug thereof provided by the present invention,
r is 1, 2, or 3;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁;
R₁₁ is selected from a C₁₋₆ alkyl, a halogenated C₁₋₆ alkyl, and a -(CH₂)ₙ-C₃₋₆ carbocyclyl;
R₄ and R₅ are each independently selected from H and a C₁₋₆ alkyl;
n is 0 or 1.

In one embodiment, in the foregoing compound of Formula (c) or the stereoisomer thereof of Formula (c-1) or Formula (c-2), the N-oxide, the hydrate, the solvate, the metabolite, the pharmaceutically acceptable salt, the polymorph, or the prodrug thereof provided by the present invention,
r is 1 or 2;
R₁₃ is each independently selected from H, a C₁₋₆ alkyl, a halogenated C₁₋₆ alkyl, and -SR₁₁, and at least one R₁₃ is -SR₁₁;
R₁₁ is selected from a C₁₋₄ alkyl and a -(CH₂)ₙ-C₃₋₄ carbocyclyl;
R₄ and R₅ are each independently selected from H and a C₁₋₆ alkyl;
n is 0 or 1.

In one embodiment, in the foregoing compound of Formula (c) or the stereoisomer thereof of Formula (c-1) or Formula (c-2), the *N*-oxide, the hydrate, the solvate, the metabolite, the pharmaceutically acceptable salt, the polymorph, or the prodrug thereof provided by the present invention, r is 2; one of R₁₃ is -SMe, and another one is selected from H and a C₁₋₄ alkyl; R₄ and R₅ are each independently selected from H and a C₁₋₄ alkyl.

In one embodiment, in the foregoing compound of Formula (c) or the stereoisomer thereof of Formula (c-1) or Formula (c-2), the N-oxide, the hydrate, the solvate, the metabolite, the pharmaceutically acceptable salt, the polymorph, or the prodrug thereof provided by the present invention, r is 1; R₁₃ is -SMe; R₄ and R₅ are each independently selected from H and a C₁₋₄ alkyl.

In one embodiment, the present invention provides a compound of Formula (d) or a stereoisomer, an *N*-oxide, a hydrate, a solvate, a metabolite, a pharmaceutically acceptable salt, a polymorph, or a prodrug thereof, 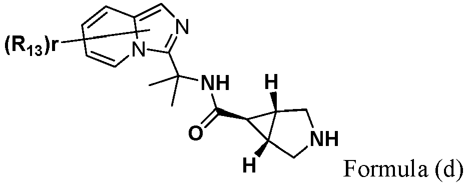 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁, and at least one R₁₃ is -SR₁₁;
R₁₁ is selected from H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
r is 1, 2, 3, 4, or 5.

In one embodiment, in the foregoing compound of Formula (d), or the stereoisomer, the *N-*oxide, the hydrate, the solvate, the metabolite, the pharmaceutically acceptable salt, the polymorph, or the prodrug thereof provided by the present invention, r is 1, 2 or 3; R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁; R₁₁ is selected from a C₁₋₆ alkyl, a halogenated C₁₋₆ alkyl, and a -(CH₂)ₙ-C₃₋₆ carbocyclyl; n is 0 or 1.

In one embodiment, in the foregoing compound of Formula (d), or the stereoisomer, the *N-*oxide, the hydrate, the solvate, the metabolite, the pharmaceutically acceptable salt, the polymorph, or the prodrug thereof provided by the present invention, r is 1 or 2; R₁₃ is each independently selected from H, a C₁₋₆ alkyl, a halogenated C₁₋₆ alkyl, and -SR₁₁, and at least one R₁₃ is -SR₁₁; R₁₁ is selected from a C₁₋₄ alkyl and a -(CH₂)ₙ-C₃₋₄ carbocyclyl; n is 0 or 1.

In one embodiment, in the foregoing compound of Formula (d), or the stereoisomer, the *N-*oxide, the hydrate, the solvate, the metabolite, the pharmaceutically acceptable salt, the polymorph, or the prodrug thereof provided by the present invention, r is 2; one of R₁₃ is -SMe and another one is selected from H and a C₁₋₄ alkyl.

In one embodiment, in the foregoing compound of Formula (d), or the stereoisomer, the *N-*oxide, the hydrate, the solvate, the metabolite, the pharmaceutically acceptable salt, the polymorph, or the prodrug thereof provided by the present invention, r is 1; R₁₃ is -SMe.

In one embodiment, the present invention provides a compound of Formula (d-1) or a stereoisomer, an *N*-oxide, a hydrate, a solvate, a metabolite, a pharmaceutically acceptable salt, a polymorph, or a prodrug thereof, 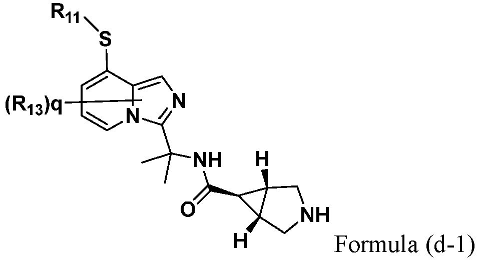 wherein
R₁₁ is selected from H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
q is 0, 1, 2, 3, or 4.

In one embodiment, in the foregoing compound of Formula (d-1), or the stereoisomer, the *N-*oxide, the hydrate, the solvate, the metabolite, the pharmaceutically acceptable salt, the polymorph, or the prodrug thereof provided by the present invention,
q is 0, 1 or 2;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁;
R₁₁ is selected from a C₁₋₆ alkyl, a halogenated C₁₋₆ alkyl, and a -(CH₂)ₙ-C₃₋₆ carbocyclyl; n is 0 or 1.

In one embodiment, in the foregoing compound of Formula (d-1), or the stereoisomer, the *N-*oxide, the hydrate, the solvate, the metabolite, the pharmaceutically acceptable salt, the polymorph, or the prodrug thereof provided by the present invention, q is 0 or 1; R₁₁ is selected from a C₁₋₄ alkyl and a -(CH₂)ₙ-C₃₋₄ carbocyclyl; R₁₃ is selected from H, a C₁₋₄ alkyl, and a halogenated C₁₋₄ alkyl; n is 0 or 1.

In one embodiment, in the foregoing compound of Formula (d-1), or the stereoisomer, the *N-*oxide, the hydrate, the solvate, the metabolite, the pharmaceutically acceptable salt, the polymorph, or the prodrug thereof provided by the present invention, q is 0; R₁₁ is a C₁₋₄ alkyl.

In one embodiment, the present invention provides a compound of Formula (d-2) or a stereoisomer, an *N*-oxide, a hydrate, a solvate, a metabolite, a pharmaceutically acceptable salt, a polymorph, or a prodrug thereof, 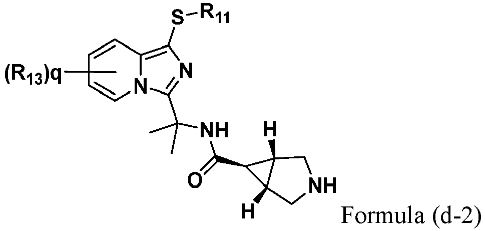 wherein
R₁₁ is selected from H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
q is 0, 1, 2, 3, or 4.

In one embodiment, in the foregoing compound of Formula (d-2), or the stereoisomer, the *N-*oxide, the hydrate, the solvate, the metabolite, the pharmaceutically acceptable salt, the polymorph, or the prodrug thereof provided by the present invention, q is 0, 1, or 2; R₁₁ is selected from a C₁₋₆ alkyl, a halogenated C₁₋₆ alkyl, and a -(CH₂)ₙ-C₃₋₆ carbocyclyl; R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁; n is 0 or 1.

In one embodiment, in the foregoing compound of Formula (d-2), or the stereoisomer, the *N-*oxide, the hydrate, the solvate, the metabolite, the pharmaceutically acceptable salt, the polymorph, or the prodrug thereof provided by the present invention, q is 0 or 1; R₁₁ is selected from a C₁₋₄ alkyl, a halogenated C₁₋₄ alkyl, and a -(CH₂)ₙ-C₃₋₄ carbocyclyl; R₁₃ is each independently selected from H, a C₁₋₆ alkyl, and a halogenated C₁₋₆ alkyl; n is 0 or 1.

In one embodiment, in the foregoing compound of Formula (d-2), or the stereoisomer, the *N-*oxide, the hydrate, the solvate, the metabolite, the pharmaceutically acceptable salt, the polymorph, or the prodrug thereof provided by the present invention, q is 1; R₁₁ is methyl; R₁₃ is H or a C₁₋₄ alkyl.

In one embodiment, the present invention provides a compound of Formula (d-3) or a stereoisomer, an *N*-oxide, a hydrate, a solvate, a metabolite, a pharmaceutically acceptable salt, a polymorph, or a prodrug thereof, 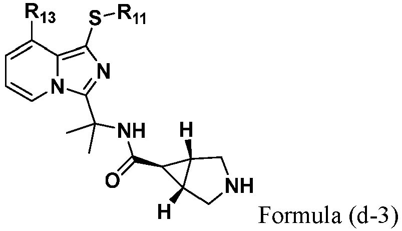 wherein
R₁₁ is selected from H, a C₁₋₆ alkyl, a halogenated C₁₋₆ alkyl, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a - (CH₂)ₙ-C₆₋₁₀ aryl, and a -(CH₂)ₙ-5- to 6-membered heteroaryl;
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, a -(CH₂)ₙ-5- to 6-membered heteroaryl, and -SR₁₁;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3.

In one embodiment, in the foregoing compound of Formula (d-3), or the stereoisomer, the *N-*oxide, the hydrate, the solvate, the metabolite, the pharmaceutically acceptable salt, the polymorph, or the prodrug thereof provided by the present invention, R₁₁ is selected from a C₁₋₆ alkyl, a halogenated C₁₋₆ alkyl, and a -(CH₂)ₙ-C₃₋₆ carbocyclyl; R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁; n is 0 or 1.

In one embodiment, in the foregoing compound of Formula (d-3), or the stereoisomer, the *N-*oxide, the hydrate, the solvate, the metabolite, the pharmaceutically acceptable salt, the polymorph, or the prodrug thereof provided by the present invention, R₁₁ is selected from a C₁₋₄ alkyl, a halogenated C₁₋₄ alkyl, and a -(CH₂)ₙ-C₃₋₄ carbocyclyl; R₁₃ is each independently selected from H, a C₁₋₄ alkyl, and a halogenated C₁₋₄ alkyl; n is 0 or 1.

In one embodiment, in the foregoing compound of Formula (d-3), or the stereoisomer, the *N-*oxide, the hydrate, the solvate, the metabolite, the pharmaceutically acceptable salt, the polymorph, or the prodrug thereof provided by the present invention, R₁₁ is methyl; R₁₃ is H or a C₁₋₄ alkyl.

In one embodiment, the present invention provides a compound of Formula (e) or a stereoisomer, an *N*-oxide, a hydrate, a solvate, a metabolite, a pharmaceutically acceptable salt, a polymorph, or a prodrug thereof, 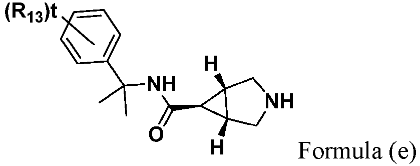 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₃₋₁₀ cycloaryl, and -SR₁₁, and at least one R₁₃ is -SR₁₁;
R₁₁ is selected from H, a C₁₋₆ alkyl, a halogenated C₁₋₆ alkyl, -(CH₂)ₙ-OR, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
t is 1, 2, 3, 4, or 5.

In one embodiment, in the foregoing compound of Formula (e), or the stereoisomer, the *N-*oxide, the hydrate, the solvate, the metabolite, the pharmaceutically acceptable salt, the polymorph, or the prodrug thereof provided by the present invention, t is 1, 2, or 3; R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁; R₁₁ is selected from a C₁₋₆ alkyl, a halogenated C₁₋₆ alkyl, and a -(CH₂)ₙ-C₃₋₆ carbocyclyl; n is 0 or 1.

In one embodiment, in the foregoing compound of Formula (e), or the stereoisomer, the *N-*oxide, the hydrate, the solvate, the metabolite, the pharmaceutically acceptable salt, the polymorph, or the prodrug thereof provided by the present invention, t is 1 or 2; R₁₃ is each independently selected from H, a C₁₋₆ alkyl, a halogenated C₁₋₆ alkyl, and -SR₁₁, and at least one R₁₃ is -SR₁₁; R₁₁ is selected from a C₁₋₄ alkyl and a -(CH₂)ₙ-C₃₋₄ carbocyclyl; n is 0 or 1.

In one embodiment, in the foregoing compound of Formula (e), or the stereoisomer, the *N-*oxide, the hydrate, the solvate, the metabolite, the pharmaceutically acceptable salt, the polymorph, or the prodrug thereof provided by the present invention, t is 1; R₁₁ is a C₁₋₄ alkyl.

In one embodiment, the present invention provides a compound of Formula (e-1) or a stereoisomer, an *N*-oxide, a hydrate, a solvate, a metabolite, a pharmaceutically acceptable salt, a polymorph, or a prodrug thereof, 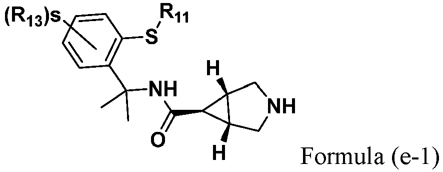 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₃₋₁₀ cycloaryl, and -SR₁₁;
R₁₁ is selected from H, a C₁₋₆ alkyl, a halogenated C₁₋₆ alkyl, -(CH₂)ₙ-OR, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
s is 0, 1, 2, 3, or 4.

In one embodiment, in the foregoing compound of Formula (e-1), or the stereoisomer, the *N-*oxide, the hydrate, the solvate, the metabolite, the pharmaceutically acceptable salt, the polymorph, or the prodrug thereof provided by the present invention, s is 0, 1, or 2; R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁; R₁₁ is selected from a C₁₋₆ alkyl, a halogenated C₁₋₆ alkyl, and a - (CH₂)ₙ-C₃₋₆ carbocyclyl; n is 0 or 1.

In one embodiment, in the foregoing compound of Formula (e-1), or the stereoisomer, the *N-*oxide, the hydrate, the solvate, the metabolite, the pharmaceutically acceptable salt, the polymorph, or the prodrug thereof provided by the present invention, s is 0 or 1; R₁₃ is each independently selected from H, a C₁₋₆ alkyl, a halogenated C₁₋₆ alkyl, a -(CH₂)ₙ-C₃₋₁₀ carbocyclyl, and -SR₁₁; R₁₁ is selected from a C₁₋₄ alkyl and a -(CH₂)ₙ-C₃₋₄ carbocyclyl; n is 0 or 1.

In one embodiment, in the foregoing compound of Formula (e-1), or the stereoisomer, the *N-*oxide, the hydrate, the solvate, the metabolite, the pharmaceutically acceptable salt, the polymorph, or the prodrug thereof provided by the present invention, s is 0; R₁₁ is a C₁₋₄ alkyl.

In one specific embodiment, the compound is selected from the following structures:

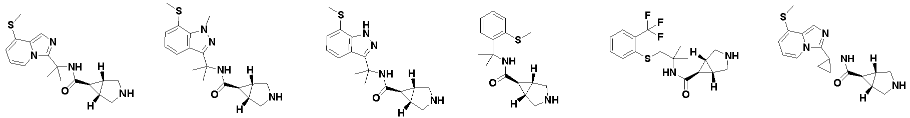

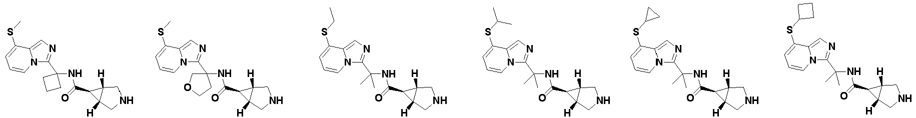

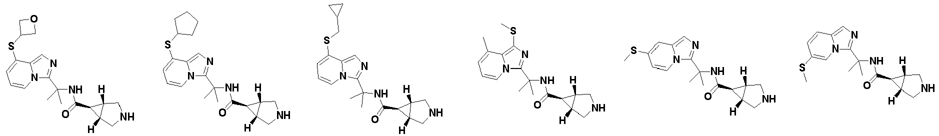

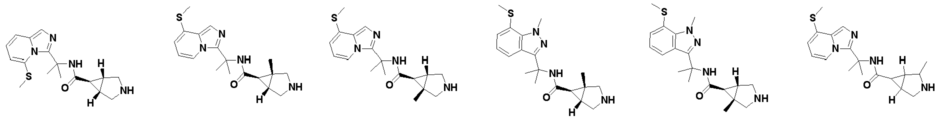

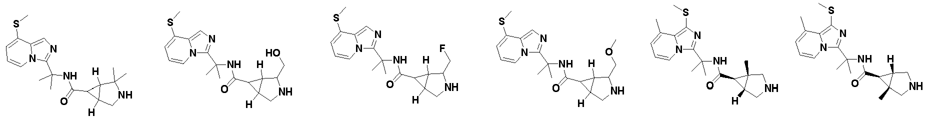

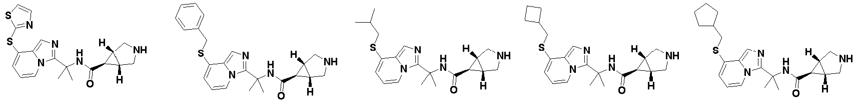

In one specific embodiment, the pharmaceutically acceptable salt is selected from a hydrochloride, a hydrobromide, a sulfate, a nitrate, a phosphate, an acetate, a maleate, a succinate, a mandelate, a fumarate, a malonate, a malate, a 2-hydroxypropionate, an oxalate, a glycolate, a salicylate, a glucuronate, a galacturonate, a citrate, a tartrate, an aspartate, a glutamate, a benzoate, a cinnamate, a *p*-toluenesulfonate, a benzenesulfonate, a mesylate, an ethanesulfonate, and a trifluoromethanesulfonate, or combinations thereof.

Preferably, the pharmaceutically acceptable salt is selected from a hydrochloride, a sulfate, a phosphate, an acetate, a maleate, a succinate, a fumarate, a malate, an oxalate, a tartrate, a benzoate, a cinnamate, a *p*-toluenesulfonate, a benzenesulfonate, a mesylate, and a triflate, or combinations thereof.

Embodiments of the present invention further provide a method for preparing the compound, comprising the steps of: 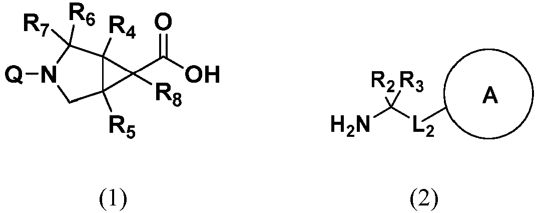
carrying out a condensation reaction with compound 1 and compound 2, wherein Q represents a nitrogen protecting group;
removing the nitrogen protecting group Q from the product of the condensation reaction.

Embodiments of the present invention further provide a pharmaceutical composition containing a therapeutically effective dose of the compound or the stereoisomer, the *N*-oxide, the hydrate, the solvate, the metabolite, the pharmaceutically acceptable salt, the polymorph, or the prodrug thereof as described above, and a pharmaceutically acceptable carrier or excipient.

Embodiments of the present invention further provide an application of the compound or the stereoisomer, the *N*-oxide, the hydrate, the solvate, the metabolite, the pharmaceutically acceptable salt, the polymorph, or the prodrug thereof as described above or the pharmaceutical composition as described above in the preparation of a drug for treatment and/or prevention of a disease or a symptom affected by SSTR4 activation.

In one specific embodiment, the disease or symptom affected by SSTR4 activation is pain.

Embodiments of the present invention further provide an application of the compound or the stereoisomer, the *N*-oxide, the hydrate, the solvate, the metabolite, the pharmaceutically acceptable salt, the polymorph, or the prodrug thereof as described above or the pharmaceutical composition as described above in the preparation of a drug for treatment and/or prevention of pain.

In one embodiment, the compound or the stereoisomer, the *N*-oxide, the hydrate, the solvate, the metabolite, the pharmaceutically acceptable salt, the polymorph, or the prodrug provided in the present invention or the pharmaceutical composition provided in the present invention is used to prepare a drug for treatment and/or prevention of a disease or a symptom affected by SSTR4 activation. In one embodiment, the compound or the composition is used to treat and/or prevent pain.

In one embodiment, the present invention provides a method for treating a disease or a symptom affected by SSTR4 activation, comprising administering the compound or the stereoisomer, the *N*-oxide, the hydrate, the solvate, the metabolite, the pharmaceutically acceptable salt, the polymorph, or the prodrug thereof according to the present invention or the pharmaceutical composition according to the present invention. In one embodiment, the present invention provides a method for treating pain, comprising administering the compound or the pharmaceutical composition according to the present invention.

In one specific embodiment, the pain is neuralgia.

In one specific embodiment, the pain is back pain, chronic back pain, trigeminal neuralgia, type I complex regional pain syndrome, type II complex regional pain syndrome, irritable bowel syndrome symptoms, diabetic neuropathy, osteoarthritis-caused pain, tumor pain, and fibromyalgia.

The implementation process and beneficial effects of the present invention will be described in detail below through specific embodiments. The embodiments are intended to help readers better understand the essence and features of the present invention, and are not intended to limit the scope of implementation of the present application.

The structures of the compounds are determined by nuclear magnetic resonance (NMR) or mass spectrometry (MS). NMR displacements (δ) are given in units of 10⁻⁶ (ppm). NMR measurement is conducted using (Bruker Avance III 400 and Bruker Avance 300) nuclear magnetic instruments, and solvents for measurement are deuterated dimethyl sulfoxide (DMSO-d₆), deuterated chloroform (CDCl₃), and deuterated methanol (CD₃OD), and an internal standard is tetramethylsilane (TMS).

Agilent 6120B (ESI) and Agilent 6120B (APCI) are used for MS measurement.

HPLC measurement is conducted using an Agilent 1260 DAD high pressure liquid chromatograph (Zorbax SB-C18 100 × 4.6 mm).

A Yantai Huanghai HSGF 254 or Qingdao GF 254 silica gel plate is used as a thin layer chromatography silica gel plate, the size of a silica gel plate used for thin layer chromatography (TLC) is from 0.15 mm to 0.20 mm, and the size of a silica gel plate used for thin layer chromatography separation and purification of products is from 0.4 mm to 0.5 mm.

Column chromatography generally uses a Yantai Huanghai silica gel 200-300 mesh silica gel as a carrier.

The known starting materials of the present invention can be synthesized by using or according to methods known in the art, or can be purchased from Titan Technology, Annagy Chemical, Shanghai Demo, Chengdu Kelong Chemical, Shaoyuan Chemical Technology, Bailingwei Technology, and other companies.

A nitrogen atmosphere means that a reaction flask is connected to a nitrogen balloon with a volume of about 1 L.

A hydrogen atmosphere means that a reaction flask is connected to a hydrogen balloon with a volume of about 1 L.

In a hydrogenation reaction, a flask is usually vacuumized and filled with a hydrogen gas, and this operation is repeated three times.

In the examples, unless otherwise specifically indicated, the reaction is carried out in a nitrogen atmosphere.

In the examples, unless otherwise specifically indicated, the solution refers to an aqueous solution.

In the examples, unless otherwise specifically indicated, the reaction temperature is room temperature.

The room temperature is the most suitable reaction temperature, ranging from 20°C to 30°C.

Abbreviations related to chemical synthesis:
Bn: Benzyl
Boc: *t*-Butoxycarbonyl
Bz: Benzoyl
DIPEA: Diisopropylethylamine
DMF: *N,N*-Dimethylformamide
DMSO: Dimethyl sulfoxide
DCM: Dichloromethane
DIEA: *N,N*-Diisopropylethylamine
EA: Ethyl acetate
Et: Ethyl
Me: Methyl
Ts: *p*-Toluoyl
HATU: 2-(7-Aza-1*H*-benzotriazol-1-yl)-1,1,3,3-tetramethyluronium hexafluorophosphate
HPLC: High performance liquid chromatography
LiHMDS: Lithium hexamethyldisilazide
MeLi: Methyl lithium
NIS: *N*-Iodosuccinimide
NMP: *N*-Methylpyrrolidone
Raney-Ni: Raney nickel
NEt₃: Triethylamine
THF: Tetrahydrofuran
TLC: Thin layer chromatography
TFA: Trifluoroacetic acid
TBAF: Tetra-*n*-butylammonium fluoride
SEMCl: 2-(Trimethylsilyl)ethoxymethyl chloride
Burgess' reagent: Burgess' reagent, CAS No.: 29684-56-8
RT: Room temperature

### Example 1

### (1R,5S,6R)-N-(2-(8-(Methylthio)imidazo[1,5-a]pyridin-3-yl)prop-2-yl)-3-azabicyclo[3.1.0]hexane-6-carboxamide hydrochloride (Compound 1)

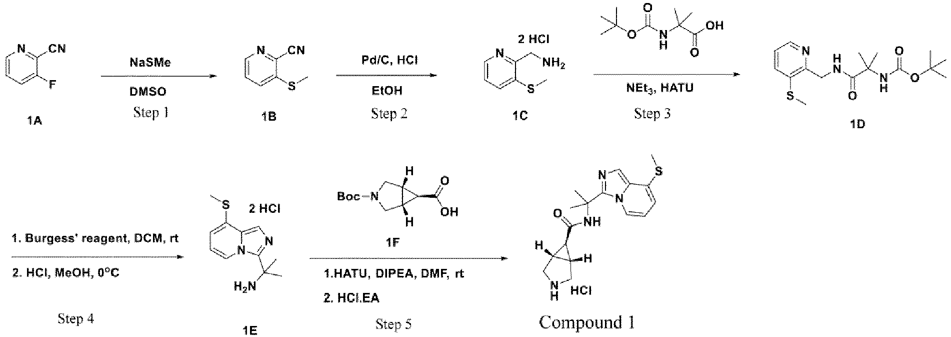

### Step 1: 2-cyano-3-methylthiopyridine (1B)

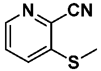

2-Cyano-3-fluoropyridine (2.0 g, 16.38 mol) and sodium methanethiolate (1.2 g. 18.02 mmol) were added to dimethylsulfoxide (10 mL), and the mixture was stirred at room temperature for 5 hours. Ethyl acetate (120 mL) was added, and washed with water and saturated brine. The organic phase was spun in vacuo to dry the solvent, and the residue was purified using a silica gel column (petroleum ether/ethyl acetate = 3/1) to obtain a white solid, which was 2-cyano-3-methylthiopyridine 1B (900 mg, yield: 36.6%).

### Step 2: (3-(methylthio)pyridin-2-yl)methanamine dihydrochloride (1C)

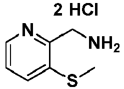

2-Cyano-3-methylthiopyridine 1B (800 mg, 5.33 mmol) was dissolved in ethanol (20 mL), 10% palladium on carbon (520 mg, 0.53 mmol) and hydrochloric acid (6 M, 5 mL) were added, and the resulting reaction mixture was stirred at room temperature for 2 days in a hydrogen atmosphere. After filtration, the filtrate was spun dry in vacuo to give a white solid, which was (3-(methylthio)pyridin-2-yl)methanamine dihydrochloride 1C (1.1 g, crude product, unpurified).

MS (ESI): m/z = 227.1 [M + H]⁺

### Step 3: tert-butyl (2-methyl-1-(((3-(methylthio)pyridin-2-yl)methyl)amino)-1-oxoprop-2-yl)carbamate (1D)

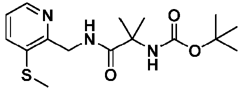

(3-(Methylthio)pyridin-2-yl)methanamine dihydrochloride 1C (1.1 g, 4.84 mmol), *N-tert-*butoxycarbonyl-2-methylalanine (985 mg, 4.84 mmol)), HATU (2.6 g, 7.26 mmol), and triethylamine (1.7 g, 16.94 mmol) were dissolved in *N,N*-dimethylformamide (10 mL) and stirred at room temperature for 16 hours. Ethyl acetate (100 mL) was added, and the organic phase was washed successively with water and saturated brine, and spun dry in vacuo. The residue was purified using a silica gel column (petroleum ether/ethyl acetate = 3/2) to obtain a white solid *tert*-butyl (2-methyl-1-(((3-(methylthio)pyridin-2-yl)methyl)amino)-1-oxoprop-2-yl)carbamate 1D (860 mg, yield: 53.7%).

MS (ESI): m/z = 340.2 [M + H]⁺

### Step 4: 2-(8-(methylthio)imidazo[1,5-a]pyridin-3-yl)propan-2-amine dihydrochloride (1E)

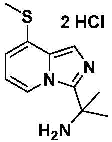

*tert-*Butyl (2-methyl-1-(((3-(methylthio)pyridin-2-yl)methyl)amino)-1-oxoprop-2-yl)carbamate 1D (360 mg, 1.06 mmol) and Burgess' reagent (378 mg, 1.53 mmol) were dissolved in dichloromethane (5 mL) and stirred at room temperature for 16 hours. Ethyl acetate (100 mL) was added, and the organic phase was washed successively with water and saturated brine, dried over anhydrous sodium sulfate, and spun dry in vacuo to obtian a pale yellow oily substance. To the oil was added 4 M hydrochloric acid in methanol (5 mL) at 0°C and stirred for 2 hours. The solvent was removed in vacuo to obtain a white solid, which was 2-(8-(methylthio)imidazo[1,5-a]pyridin-3-yl)propan-2-amine dihydrochloride 1E (220 mg, crude product, unpurified).

MS (ESI): m/z = 222.1 [M + H]⁺

### Step 5: (1R,5S,6R)-N-(2-(8-(methylthio)imidazo[1,5-a]pyridin-3-yl)propan-2-yl)-3-azabicyclo[3.1.0]hexane-6-carboxamide hydrochloride (Compound 1)

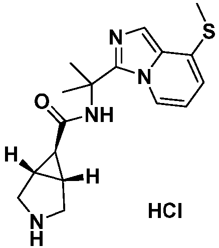

2-(8-(Methylthio)imidazo[1,5-a]pyridin-3-yl)propan-2-amine dihydrochloride 1E (60 mg, 0.20 mmol), compound 1F(1*R*,5*S*,6*R*)-3-(*tert*-butoxycarbonyl)-3-azabicyclo[3.1.0]hexane-6-carboxylic acid (CAS No.: 927679-54-7) (46 mg, 0.20 mmol), HATU (115 mg, 0.30 mmol), and diisopropylethylamine (90 mg, 0.70 mmol) were dissolved in *N,N*-dimethylformamide (2 mL) and stirred at room temperature for 16 hours. The reaction solution was purified by preparative HPLC to obtain a white solid (25 mg), to which a solution of hydrochloric acid in ethyl acetate (3 M, 4 mL) was added and stirred for 2 hours. The solvent was removed by filtration to obtain (1*R*,5*S*,6*R*)-*N*-(2-(8-(methylthio)imidazo[1,5-a]pyridin-3-yl)prop-2-yl)-3-azabicyclo[3.1.0]hexane-6-carboxamide hydrochloride, which was Compound 1 (12 mg, yield: 17.9%).

¹H NMR (400 MHz, CD₃OD) *δ* 9.28 (d, 1H), 8.39 (t, 1H), 8.02 (s, 1H), 7.13 (t, 1H), 7.03 (d, 1H)), 3.52-3.42 (m, 4H), 2.66 (s, 3H), 1.97-1.92 (m, 3H), 1.89 (s, 6H).

MS (ESI): m/z = 331.1 [M + H]⁺

### (1R,5S,6R)-N-(2-(8-(Methylthio)imidazo[1,5-a]pyridin-3-yl)prop-2-yl)-3-azabicyclo[3.1.0]hexane-6-carboxamide (Compound 1a)

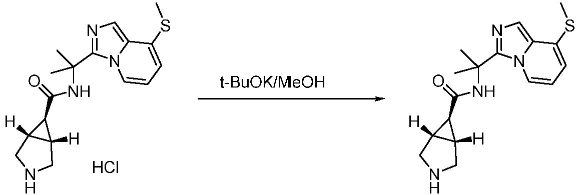

Referring to the synthesis method of Example 1, the synthesis was amplified to obtain Compound 1 (30 g, 81.7 mmol). The compound was dissolved in MeOH (210 mL). In an ice-water bath, a solution of potassium *tert*-butoxide (20.8 g, 185.4 mmol) in methanol (120 mL) prepared in advance was slowly added dropwise, and the pH value of the reaction solution was monitored during this process. When the pH value was greater than 9, the dropwise addition was stopped. Stirring was continued for 10 minutes. After filtration, the filtrate was concentrated and spun dry to obtain a viscous oily crude product. The crude product was purified through a silica gel column (dichloromethane/methanol = 15/1) to obtain an off-white solid. The solid was further purified by beating with ethanol to obtain pure Compound 1a, which was an off-white solid (15 g, yield: 55.5%).

¹H NMR (400 MHz, DMSO-d₆) *δ* 8.38 (s, 1H), 8.11 (d, 1H), 7.19 (s, 1H), 6.61 (t, 1H), 6.54 (d, 1H), 2.87 (d, 2H), 2.67 (d, 2H), 2.54 (s, 3H), 1.64 (s, 6H), 1.56 (t, 1H), 1.47 (m, 2H).

MS (ESI): m/z = 331.2 [M + H]⁺

### Example 2

### (1R,5S,6R)-N-(2-Methyl-1-((2-(trifluoromethyl)phenyl)thio)prop-2-yl)-3-azabicyclo[3.1.0]hexane-6-carboxamide formate (Compound 2)

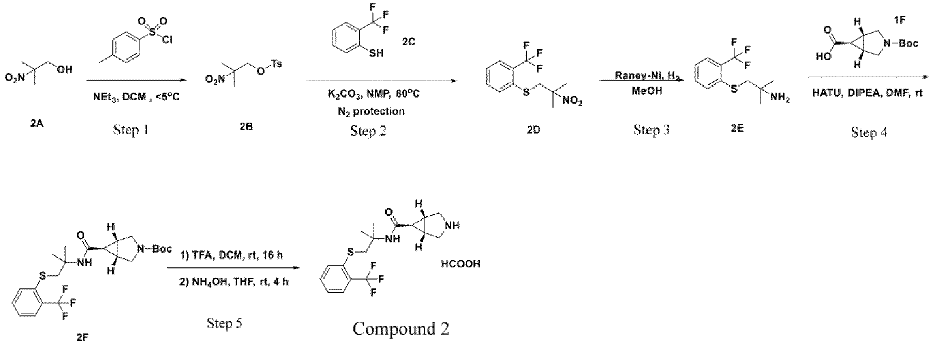

### Step 1: 2-methyl-2-nitropropyl 4-methylbenzenesulfonate (2B)

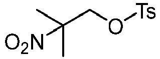

2-Methyl-2-nitropropan-1-ol 2A (1.19 g, 10.00 mmol) and triethylamine (2.02 g, 20.00 mmol) were mixed in dichloromethane (50 mL) and cooled to 0-5°C. 4-Methylbenzenesulfonyl chloride (2.86 g, 15.00 mmol) was added and stirred at room temperature for 18 hours. TLC detection indicated that a new spot was generated. Dichloromethane (100 mL) was added for dilution, 1 M hydrochloric acid (100 mL) and a saturated sodium bicarbonate solution (100 mL) were used for washing, and then saturated brine was used for washing. The organic phase was separated, dried with anhydrous sodium sulfate, filtered, and purified with a silica gel column (petroleum ether/ethyl acetate = 10/1) to obtain 2-methyl-2-nitropropyl 4-methylbenzenesulfonate 2B, an off-white solid (2.46 g, yield: 90%).

¹H NMR (400 MHz, CDCl₃) *δ* 7.77 (d, 2H), 7.37 (d, 2H), 4.28 (s, 2H), 2.47 (s, 3H), 1.60 (s, 6H).

### Step 2: (2-methyl-2-nitropropyl)(2-(trifluoromethyl)phenyl)sulfane (2D)

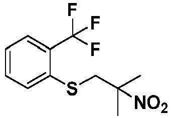

2-Methyl-2-nitropropyl 4-methylbenzenesulfonate 2B (500 mg, 1.83 mmol), compound 2-(trifluoromethyl)thiophenol 2C (326 mg, 1.83 mmol)), and K₂CO₃ (757 mg, 0.45 mmol) were dissolved in *N*-methylpyrrolidone (10 mL) and reacted at 80°C for 16 hours while stirring under the protection of nitrogen. 50 mL of water was added to the reaction solution, followed by extraction with ethyl acetate (50 × 3) three times, and the organic phase was dried over anhydrous sodium sulfate. The solvent was spun dry, and the crude product was purified using a silica gel column (petroleum ether/ethyl acetate = 10/1 to 5/1), to obtain a product (2-methyl-2-nitropropyl)(2-(trifluoromethyl)phenyl)sulfane 2D, which was a yellow oily substance (350 mg, yield: 68.6%).

### Step 3: 2-methyl-1-((2-(trifluoromethyl)phenyl)sulfanyl)propan-2-amine (2E)

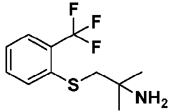

(2-Methyl-2-nitropropyl)(2-(trifluoromethyl)phenyl)sulfane 2D (350 mg, 1.5 mmol) was dissolved in methanol (10 mL) and Raney nickel was added (50 mg). A hydrogen balloon was inserted to replace air three times, to initiate a reaction while stirring at room temperature under hydrogen for 16 hours. The Raney nickel was filtered off, and the filtrate was spun dry to obtain a product 2-methyl-1-((2-(trifluoromethyl)phenyl)sulfanyl)propan-2-amine 2E, which was a yellow oily substance (312 mg, yield: 100%), and was directly sent to the next step without purification.

### Step 4: tert-butyl (1R,5S,6R)-6-((2-methyl-1-((2-(trifluoromethyl)phenyl)thio)propan-2-yl)carbamoyl)-3-azabicyclo[3.1.0]hexane-3-carboxylate (2F)

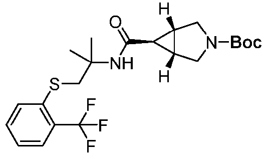

2-Methyl-1-((2-(trifluoromethyl)phenyl)sulfanyl)propan-2-amine 2E (100 mg, 0.40 mmol), (1*R*,5*S*,6*R*)-3-(*tert*-butoxycarbonyl)-3-azabicyclo[3.1.0]hexane-6-carboxylic acid 1F (91 mg, 0.4 mmol), HATU (190 mg, 0.5 mmol), and triethylamine (101 mg, 1 mmol) were dissolved in *N*,*N-*dimethylformamide (1 mL) and stirred at room temperature for 16 hours. Water (2 mL) was added to the reaction solution, which was extracted with ethyl acetate, and the organic phase was dried over sodium sulfate. The solvent was spun dry to obtain a crude product, which was passed through a silica gel column (petroleum ether/ethyl acetate = 10/1) to obtain a white solid, which was *tert-*butyl (1*R*,5*S*,6*R*)-6-((2-methyl-1-((2-(trifluoromethyl)phenyl)thio)propan-2-yl)carbamoyl)-3-azabicyclo[3.1.0]hexane-3-carboxylate) 2F (110 mg, yield: 60.1%).

### Step 5: (1R,5S,6R)-N-(2-methyl-1-((2-(trifluoromethyl)phenyl)thio)propan-2-yl)-3-azabicyclo[3.1.0]hexane-6-carboxamide formate

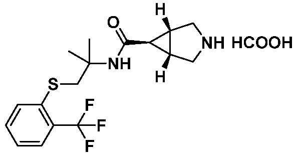

*tert-*Butyl (1*R*,5*S*,6*R*)-6-((2-methyl-1-((2-(trifluoromethyl)phenyl)sulfanyl)propan-2-yl)carbamoyl)-3-azabicyclo[3.1.0]hexane-3-carboxylate 2F (110 mg, 0.30 mmol) was dissolved in dichloromethane (2 mL) and trifluoroacetic acid (0.5 mL) was added, and the mixture was stirred at room temperature for 16 hours. The reaction solution was spun to dry the solvent, then ammonia water (1 mL) was added, and the organic phase was extracted with dichloromethane (5 mL × 3) to obtain a crude product. The crude product was purified by preparative HPLC to give a white solid (25 mg) (1*R*,5*S*,6*R*)-*N*-(2-methyl-1-((2-(trifluoromethyl)phenyl)sulfanyl))propan-2-yl)-3-azabicyclo[3.1.0]hexane-6-carboxamide formate (Compound 2) (25 mg, yield: 22.2%).

¹H NMR (400 MHz, DMSO-d₆) δ 8.83 (s, 1H), 7.93 (s, 1H), 7.69 (d, 1H), 7.65-7.55 (m, 2H), 7.37 (t, 1H), 3.52 (s, 2H), 3.23 (m, 4H), 1.87 (m, 2H), 1.58 (m, 1H), 1.32 (s, 6H).

MS (ESI): m/z = 359.2 [M + H]⁺

### Example 3

### (1R,5S,6R)-N-(2-(8-(Cyclopentylthio)imidazo[1,5-a]pyridin-3-yl)prop-2-yl)-3-azabicyclo[3.1.0]hexane-6-carboxamide (Compound 3)

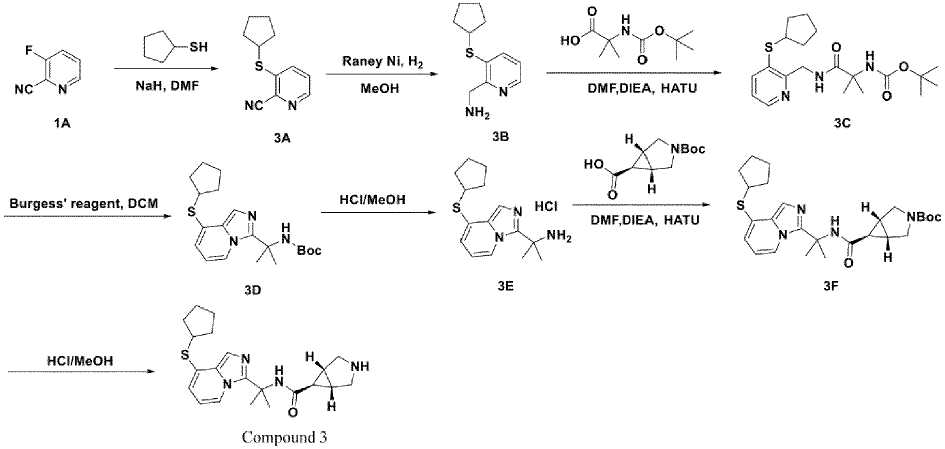

### Step 1: 3-(cyclopentylthio)pyridine-2-carbonitrile (3A)

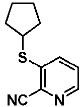

Cyclopentanethiol (918 mg, 9.0 mmol) was dissolved in *N,N*-dimethylformamide (40 mL), and sodium hydride (60%, 540 mg, 13.5 mmol) was slowly added at 0°C and reacted for 30 minutes. 3-Fluoropyridine-2-carbonitrile 1A (1.1 g, 9.0 mmol) was then added, and the solution was slowly warmed to room temperature, and stirred at room temperature overnight. LCMS monitoring showed that the reaction was complete. The reaction solution was poured into water, the aqueous phase was extracted with ethyl acetate, and the merged organic phases were dried over anhydrous sodium sulfate and dried in vacuo to obtain 3-(cyclopentylthio)pyridine-2-carbonitrile 3A (770 mg, yellow oily substance). The crude product was directly used in the next step.

MS (ESI): m/z = 205.1 [M + H]⁺

### Step 2: (3-(cyclopentylthio)pyridin-2-yl)methanamine (3B)

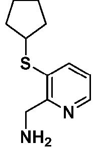

3-(Cyclopentylthio)pyridine-2-carbonitrile 3A was dissolved in methanol (30 mL), ammonia (6 mL) was added, and Raney Ni (catalytic amount) was then added. Air was replaced with hydrogen three times and the solution was stirred at room temperature overnight. After suction filtration of the reaction solution, (3-(cyclopentylthio)pyridin-2-yl)methanamine 3B (800 mg, crude product) was obtained, which was directly used in the next step.

MS (ESI): m/z = 209.1 [M + H]⁺

### Step 3: tert-butyl (1-(((3-(cyclopentylthio)pyridin-2-yl)methyl)amino)-2-methyl-1-oxopropan-2-yl)carbamate (3C)

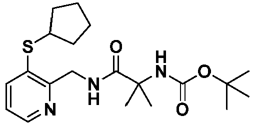

2-((*tert*-butoxycarbonyl)amino)-2-methylpropionic acid (933.8 mg, 4.6 mmol) and HATU (1.7 g, 4.6 mmol) were dissolved in *N,N*-dimethylformamide (30 mL)) at room temperature for half an hour. Then (3-(cyclopentylthio)pyridin-2-yl)methanamine 3B (800 mg, 3.8 mmol) and DIEA (2.1 mL) were added and the mixture was stirred at room temperature overnight. The reaction solution was poured into water, the aqueous phase was extracted with ethyl acetate, and the merged organic phases were washed with saturated brine, dried over anhydrous sodium sulfate, and spun dry in vacuo to obtain a crude product. The crude product was separated and purified by silica gel column chromatography (petroleum ether/ethyl acetate = 3/1) to obtain *tert-*butyl (1-(((3-(cyclopentylthio)pyridin-2-yl)methyl)amino)-2-methyl-1-oxoprop-2-yl)carbamate 3C (600 mg, pale yellow oily substance).

MS (ESI): m/z = 394.1 [M + H]⁺

### Step 4: tert-butyl (2-(8-(cyclopentylthio)imidazo[1,5-a]pyridin-3-yl)propan-2-yl)carbamate (3D)

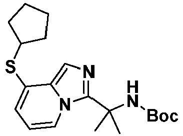

*tert-*Butyl (1-(((3-(cyclopentylthio)pyridin-2-yl)methyl)amino)-2-methyl-1-oxoprop-2-yl)carbamate 3C (600 mg, 1.53 mmol) was dissolved in dichloromethane (20 mL), Burgess reagent (1.46 g, 6.1 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and spun dry in vacuo to obtain a crude product. The crude product was separated and purified by silica gel column chromatography (petroleum ether/ethyl acetate = 5/1) to obtain *tert*-butyl (2-(8-(cyclopentylthio)imidazo[1,5-a]pyridin-3-yl)prop-2-yl)carbamate 3D (260 mg, yield: 45%, yellow oily substance)

MS (ESI): m/z = 376.1 [M + H]⁺

### Step 5: 2-(8-(cyclopentylthio)imidazo[1,5-a]pyridin-3-yl)propan-2-amine hydrochloride (3E)

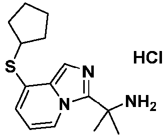

*tert*-Butyl (2-(8-(cyclopentylthio)imidazo[1,5-a]pyridin-3-yl)propan-2-yl)carbamate 3D (260 mg, 0.69 mmol) was dissolved in hydrochloric acid-methanol solution (10 mL) and reacted at room temperature overnight. The reaction solution was spun dry in vacuo to obtain 2-(8-(cyclopentylthio)imidazo[1,5-a]pyridin-3-yl)propan-2-amine 3E hydrochloride (180 mg, crude product), which was directly used in the next step.

MS (ESI): m/z = 276.1 [M + H]⁺

### Step 6: tert-butyl (1R,5S,6R)-6-((2-(8-(cyclopentylthio)imidazo[1,5-a]pyridin-3-yl)propan-2-yl)carbamoyl)-3-azabicyclo[3.1.0]-3-carboxylate (3F)

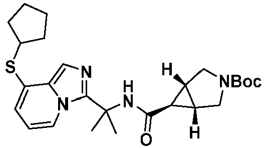

(1*R*,5*S*,6*R*)-3-(*tert*-Butoxycarbonyl)-3-azabicyclo[3.1.0]hexane-6-carboxylic acid (182 mg, 0.8 mmol) and HATU (304 mg, 0.8 mmol) was dissolved in DMF (10 mL), and reacted at room temperature for half an hour, then 2-(8-(cyclopentylthio)imidazo[1,5-a]pyridin-3-yl)propan-2-amine 3E (180 mg, 0.65 mmol) and DIEA (0.5 mL) were added, and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude product was separated and purified by silica gel column chromatography (dichloromethane/methanol = 15/1) to obtain *tert-*butyl (1*R*,5*S*,6*R*)-6-((2-(8-(cyclopentylthio)imidazolium)[1,5-a]pyridin-3-yl)propan-2-yl)carbamoyl)-3-azabicyclo[3.1.0]-3-carboxylate 3F (200 mg, yield: 63.5%, yellow oily substance).

MS (ESI): m/z = 485.1 [M + H]⁺

### Step 7: (1R,5S,6R)-N-(2-(8-(cyclopentylthio)imidazo[1,5-a]pyridin-3-yl)propan-2-yl)-3-azabicyclo[3.1.0]hexane-6-carboxamide (Compound 3)

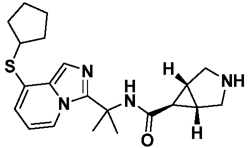

*tert-*Butyl (1*R*,5*S*,6*R*)-6-((2-(8-(cyclopentylthio)imidazo[1,5-a]pyridin-3-yl)prop-2-yl)carbamoyl)-3-azabicyclo[3.1.0]-3-carboxylate 3F (200 mg, 0.41 mmol) was dissolved in a hydrochloric acid-methanol solution (10 mL) and reacted at room temperature for 3 hours. The reaction solution was spun dry to remove the solvent in vacuo to obtain a crude product. The crude product was purified by preparative HPLC to give a pure white solid (Compound 3) (47 mg, yield: 29.8%).

¹H NMR (400 MHz, DMSO-d₆) *δ* 8.40 (s, 1H), 8.14 (d, 1H), 7.20 (s, 1H), 6.68 (d, 1H), 6.61 (t, 1H), 3.85 (m, 1H), 2.92 (m, 2H), 2.76 (m, 2H), 2.12 (m, 2H), 1.73 (m, 2H), 1.64 (s, 6H), 1.62-1.57 (m, 5 H), 1.52 (m, 2H).

MS (ESI): m/z = 385.1 [M + H]⁺

### Example 4

### (1R,5S,6R)-N-(2-(8-(Thiazol-2-ylsulfanyl)imidazo[1,5-a]pyridin-3-yl)prop-2-yl)-3-azabicyclo[3.1.0]hexane-6-carboxamide (Compound 4)

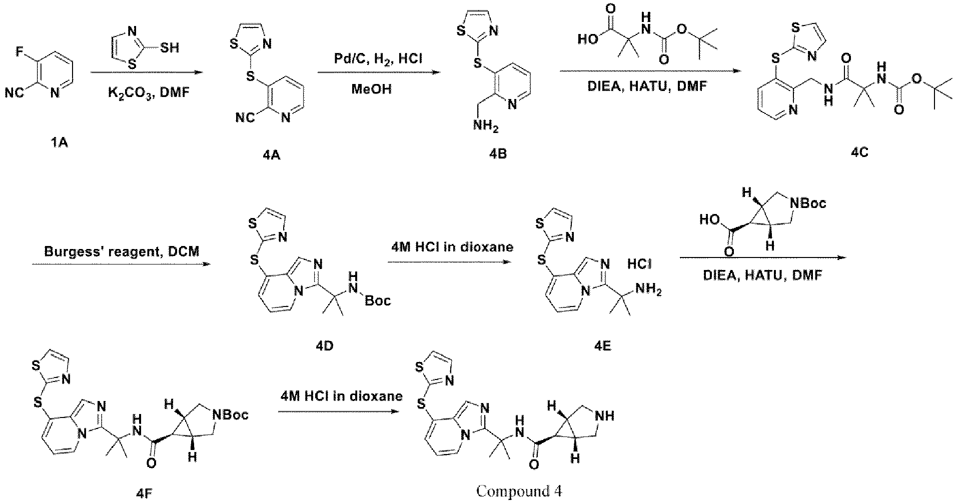

### Step 1: 3-(thiazol-2-ylsulfanyl)pyridine-2-carbonitrile (4A)

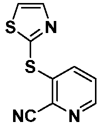

Thiazole-2-thiol (1.44 g, 12.30 mmol) and K₂CO₃ (3.39 g, 24.59 mmol) were dissolved in DMF (30 mL), and after stirring for 5 min, 3-fluoropyridine-2-carbonitrile 1A (1 g, 8.19 mmol) was added, and then the reaction was carried out for 2 h. Upon completion of the reaction, 50 mL of water was added for quenching. The reaction solution was extracted with ethyl acetate (30 mL × 3), the organic phases were merged, washed with 50 mL of saturated brine, and concentrated in vacuo, and the resulting residue was purified using a silica gel column (petroleum ether/ethyl acetate = 2/1) to obtain 3-(thiazol-2-ylsulfanyl)pyridine-2-carbonitrile 4A (1.6 g, yield: 89%).

MS (ESI): m/z = 220.1 [M + H]⁺

### Step 2: (3-(thiazol-2-ylsulfanyl)pyridin-2-yl)methanamine (4B)

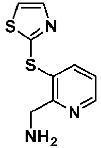

3-(Thiazol-2-ylsulfanyl)pyridine-2-carbonitrile 4A (1.6 g, 7.17 mmol) was dissolved in MeOH (50 mL), palladium/charcoal (200 mg) was added, the air was replaced by hydrogen three times, and the reaction was carried out overnight. Upon completion of the reaction, the reaction solution was filtered, and the organic phase of the filtrate was spun dry to obtain a crude product, which was an off-white solid (3-(thiazol-2-ylsulfanyl)pyridin-2-yl)methanamine 4B (1.8 g).

MS (ESI): m/z = 224.1 [M + H]⁺

### Step 3: tert-butyl (2-methyl-1-oxo-1-(((3-(thiazol-2-ylsulfanyl)pyridin-2-yl)methyl)amino)propan-2-yl)carbamate (4C)

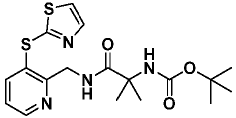

2-((*tert*-Butoxycarbonyl)amino)-2-methylpropionic acid (1.75 g, 8.61 mmol) and HATU (3.60 g, 9.47 mmol) were dissolved in DMF (30 mL) at room temperature, and then (3-(thiazol-2-ylsulfanyl)pyridin-2-yl)methanamine 4B (1.8 g, crude product) and DIEA (2.78 g, 21.52 mmol) were added. After reaction for 1 h, water (50 mL) was added for quenching, and then the reaction solution was extracted with ethyl acetate (40 mL × 3). The organic phase was washed once with 50 mL of a saturated aqueous NaCl solution. The organic phase was dried over anhydrous sodium sulfate, filtered by suction, and spun dry. The resulting residue was purified using a silica gel column (petroleum ether/ethyl acetate = 2/1) to obtain a white solid *tert-*butyl (2-methyl-1-oxo-1-(((3-(thiazol-2-ylthio)pyridin-2-yl)methyl)amino)propan-2-yl)carbamate 4C (1.1 g, yield: 37.0%).

MS (ESI): m/z = 409.1 [M + H]⁺

### Step 4: tert-butyl (2-(8-(thiazol-2-ylsulfanyl)imidazo[1,5-a]pyridin-3-yl)propan-2-yl)carbamate (4D)

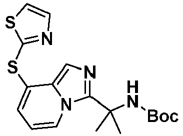

*tert-*Butyl (2-methyl-1-oxo-1-(((3-(thiazol-2-ylthio)pyridin-2-yl)methyl)amino)propan-2-yl)carbamate 4C (1.1 g, 2.70 mmol) was dissolved in dry DCM (10 mL), Burgess reagent (3.30 g, 13.48 mmol) was added, and the mixture was stirred at room temperature overnight. Upon completion of the reaction, water (40 mL) was added for quenching, and the reaction solution was extracted with ethyl acetate (20 mL × 3). The organic phase was washed once with 50 mL of a saturated aqueous NaCl solution. The organic phase was dried over anhydrous sodium sulfate, filtered by suction, and spun dry. The resulting residue was purified using a silica gel column (petroleum ether/ethyl acetate = 1/1) to obtain a white solid, which was *tert*-butyl (2-(8-(thiazol-2-ylsulfanyl)imidazo[1,5-a])pyridin-3-yl)propan-2-yl)carbamate 4D (200 mg, yield: 19.0%).

MS (ESI): m/z = 391.1 [M + H]⁺

### Step 5: 2-(8-(thiazol-2-ylsulfanyl)imidazo[1,5-a]pyridin-3-yl)propan-2-amine hydrochloride (4E)

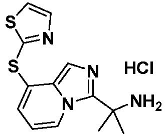

To *tert*-butyl (2-(8-(thiazol-2-ylsulfanyl)imidazo[1,5-a]pyridin-3-yl)propan-2-yl)carbamate 4D (200 mg, 0.51 mmol) was added a 4 M solution of hydrochloric acid in 1,4-dioxane (5 mL), and the mixture was stirred at room temperature for 30 min. Upon completion of the reaction, the reaction solution was spun dry in vacuo to obtain a crude oily product, which was 2-(8-(thiazol-2-ylsulfanyl)imidazo[1,5-a]pyridin-3-yl)propan-2-amine hydrochloride 4E (120 mg).

MS (ESI): m/z = 291.1 [M + H]⁺

### Step 6: tert-butyl (1R,5S,6R)-6-((2-(8-(thiazol-2-ylsulfanyl)imidazo[1,5-a]pyridin-3-yl)prop-2-yl)carbamoyl)-3-azabicyclo[3.1.0]hexane-3-carboxylate (4F)

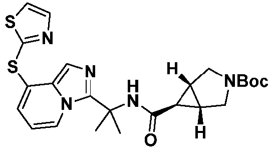

(1*R*,5*S*,6*R*)-3-(*tert*-Butoxycarbonyl)-3-azabicyclo[3.1.0]hexane-6-carboxylic acid (139 mg, 0.61 mmol) and HATU (252 mg), 0.66 mmol) were dissolved in DMF (5 mL), then the crude oil 2-(8-(thiazol-2-ylsulfanyl)imidazo[1,5-a]pyridin-3-yl)propan-2-amine 4E (120 mg) and DIEA (197 mg, 1.53 mmol) were added and reacted at room temperature for 1 h, followed by addition of water (10 mL) for quenching. The reaction solution was then extracted with ethyl acetate (5 mL × 3), the organic phase was washed once with 20 mL of a saturated aqueous NaCl solution, and the organic phase was dried over anhydrous sodium sulfate, and filtered by suction. The organic phase was spun dry and the resulting residue was purified with a preparative plate (petroleum ether:ethyl acetate = 2:1) to obtain a white solid, which was *tert-*butyl (1*R*,5*S*,6*R*)-6-((2-(8-(thiazol-2-ylthio)imidazo[1,5-a]pyridin-3-yl)propan-2-yl)carbamoyl)-3-azabicyclo[3.1.0]hexane-3-carboxylate 4F (110 mg, yield: 43.2%).

MS (ESI): m/z = 500.1 [M + H]⁺

### Step 7: (1R,5S,6R)-N-(2-(8-(thiazol-2-ylthio)imidazo[1,5-a]pyridin-3-yl)propan-2-yl)-3-azabicyclo[3.1.0]hexane-6-carboxamide (Compound 4)

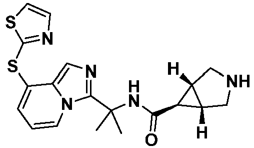

To *tert-*butyl (1*R*,5*S*,6*R*)-6-((2-(8-(thiazol-2-ylthio)imidazo[1,5-a]pyridin-3-yl)propan-2-yl)carbamoyl)-3-azabicyclo[3.1.0]hexane-3-carboxylate 4F (110 mg, 0.22 mmol) was added 4 M/L hydrochloric acid 1,4-dioxane (3 mL), and the mixture was stirred at room temperature for 30 min. Upon completion of the reaction, the reaction solution was dried in vacuo and purified by Pre-HPLC to obtain (1*R*,5*S*,6*R*)-*N*-(2-(8-(thiazol-2-ylsulfanyl)imidazo[1,5-a]]pyridin-3-yl)propan-2-yl)-3-azabicyclo[3.1.0]hexane-6-carboxamide (Compound 4) (38 mg, yield: 43.2%).

¹H NMR (400 MHz, DMSO-d₆) δ 8.40 (s, 1H), 8.37 (d, 1H), 7.82 (d, 1H), 7.77 (d, 1H), 7.19 (s, 1H)), 7.07 (d, 1H), 6.71 (dd, 1H), 2.88 (d, 2H), 2.70 (d, 2H), 1.65 (s, 6H), 1.57 (m, 1H), 1.50 (m, 2H).

MS (ESI): m/z = 400.1 [M + H]⁺

### Example 5

### 1-Methyl-N-(2-(8-(methylthio)imidazo[1,5-a] pyridin-3-yl)propan-2-yl)-3-azabicyclo[3.1.0]hexane-6-carboxamide (Compound 5-P1 and Compound 5-P2)

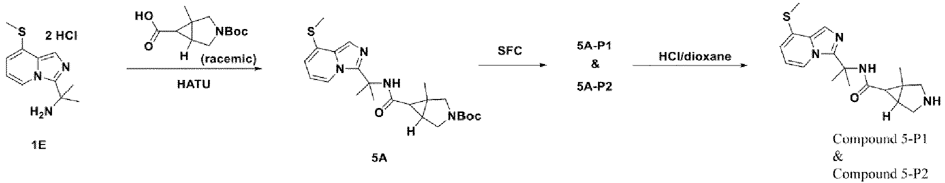

### Step 1: tert-butyl 1-methyl-6-((2-(8-(methylthio)imidazo[1,5-a]pyridin-3-yl)propan-2-yl)carbamoyl)-3-azabicyclo[3.1.0]hexane-3-carboxylate (5A)

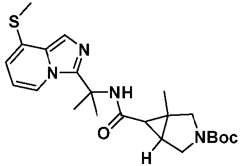

3-(*tert*-Butoxycarbonyl)-1-methyl-3-azabicyclo[3.1.0]hexane-6-carboxylic acid (racemic, 200 mg, 0.83 mmol) was dissolved in DMF (2 mL), and 2-(8-(methylthio)imidazo[1,5-a]pyridin-3-yl)propan-2-amine 1E, DIEA (321 mg, 2.49 mmol), and HATU (473 mg, 1.24 mmol) were added and reacted at room temperature for 6 hours. Ethyl acetate (20 mL) was added to the reaction solution, followed by washing with saturated brine (10 mL × 2). The organic phase was dried over anhydrous sodium sulfate, filtered, and concentrated. The residue was separated and purified by silica gel column chromatography (petroleum ether/ethyl acetate = 1/1 to 1/5) to obtain a yellow solid compound, which was *tert-*butyl 1-methyl-6-((2-(8-(methylsulfide yl)imidazo[1,5-a]pyridin-3-yl)prop-2-yl)carbamoyl)-3-azabicyclo[3.1.0]hexane-3-carboxylate 5A (162 mg, yield: 43.7%).

MS (ESI): m/z = 445.1 [M + H]⁺

Chiral resolution of 5A gave 5A-P1 (65 mg, compound with shorter retention time) and 5A-P2 (63 mg, compound with longer retention time). The conditions for the chiral resolution were shown in the table below:

| | |
|---|---|
| **System** | Waters SFC 150 |
| **Column name** | DAICELCHIRALPAK^{®}OZ |
| **Column size** | 250 * 25 mm 10 µm |
| **Mobile phase A** | Supercritical CO₂ |
| **Mobile phase B** | MeOH (+ 0.1% 7.0 mol/L ammonia in MeOH) |
| **A:B** | 75:25 |
| **Wavelength** | 214 nm |
| **Flow rate** | 70 mL/min |
| **Column temperature** | RT |
| **Back pressure** | 100 bar |
| **Injection volume** | 1.8 mL |
| **Cycle time** | 3 min |
| **Solvent** | MeOH: redistilled grade |
| | Supercritical CO₂: food grade |
| **Preparation of sample solution** | A sample is solution was dissolved in about 60 mL of MeOH. |

### Step 2: 1-methyl-N-(2-(8-(methylthio)imidazo[1,5-a]pyridin-3-yl)propan-2-yl)-3-azabicyclo[3.1.0]hexane-6-carboxamide (Compound 5)

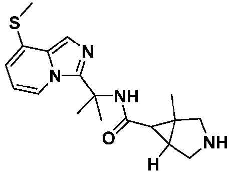

*tert*-Butyl 1-methyl-6-((2-(8-(methylthio)imidazo[1, 5-a]pyridin-3 -yl)propan-2-yl)carbamoyl)-3-azabicyclo[3.1.0]hexane-3-carboxylate 5A-P1 (65 mg, 0.146 mmol) was dissolved in hydrochloric acid-dioxane solution (5 mL) and reacted at room temperature for 1 hour. The reaction solution was spun dry in vacuo to remove the solvent. The residue was dissolved in DCM, neutralized to basic with saturated NaHCO₃, and purified by Prep-TLC (DCM/MeOH) to give the target compound, which was 1-methyl-*N*-(2-(8-(methylthio))imidazo[1,5-a]pyridin-3-yl)propan-2-yl)-3-azabicyclo[3.1.0]hexane-6-carboxamide (Compound 5-P1) (11 mg, yield: 22%).

¹H NMR (400 MHz, DMSO-d₆) δ 8.41 (s, 1H), 8.09 (d, 1H), 7.20 (s, 1H), 6.59 (t, 1H), 6.53 (d, 1H), 2.95-2.80 (m, 3H), 2.67 (d, 1H), 2.54 (s, 3H), 2.05-1.95 (m, 2H), 1.68 (s, 3H), 1.62 (s, 3H), 0.96 (s, 3H).

MS (ESI): m/z = 345.1 [M + H]⁺

5A-P2 (63 mg, 0.142 mmol) was deprotected with hydrochloric acid according to the same method as above to obtain a compound 1-methyl-*N*-(2-(8-(methylthio)imidazo[1,5-a]pyridin-3-yl)propan-2-yl)-3-azabicyclo[3.1.0]hexane-6-carboxamide (Compound 5-P2) (9 mg, yield: 18%).

¹H NMR (400 MHz, DMSO-d₆) δ 8.43 (s, 1H), 8.09 (d, 1H), 7.20 (s, 1H), 6.61 (t, 1H), 6.53 (d, 1H), 2.99-2.87 (m, 3H), 2.73 (d, 1H), 2.54 (s, 3H), 2.01 (m, 2H), 1.68 (s, 3H), 1.62 (s, 3H), 0.97 (s, 3H).

MS (ESI): m/z = 345.1 [M + H]⁺

### Example 6

### (1R,5S,6R)-N-(2-(8-(Ethylthio)imidazo[1,5-a]pyridin-3-yl)prop-2-yl)-3-azabicyclo[3.1.0]hexane-6-carboxamide hydrochloride (Compound 6)

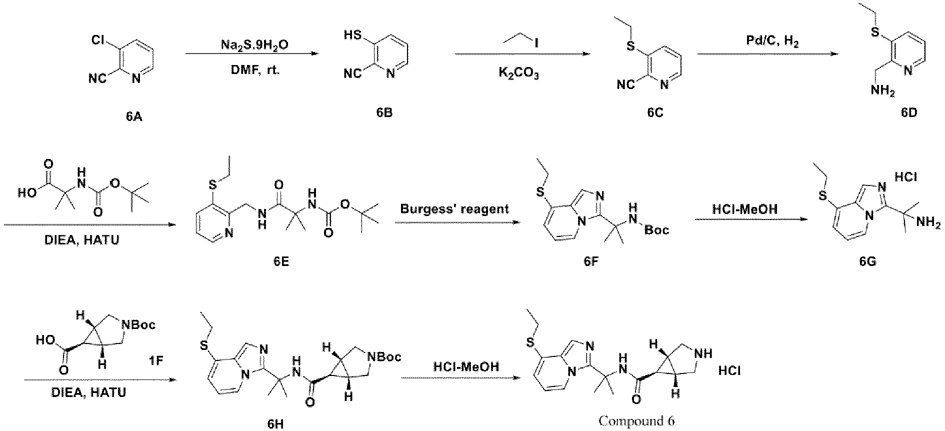

### Step 1: 3-mercaptopyridine-2-carbonitrile (6B)

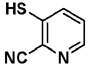

3-Chloro-2-cyanopyridine 6A (2.0 g, 14.4 mmol) and sodium sulfide nonahydrate (3.8 g, 15.8 mmol) were dissolved in *N,N*-dimethylformamide (60 mL) at room temperature, and the mixture was stirred overnight. LCMS monitoring showed that the reaction was complete, and the reaction solution was directly used in the next step without treatment.

### Step 2: 3-(ethylthio)pyridine-2-carbonitrile (6C)

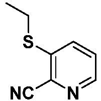

To the reaction solution obtained in step 1, iodoethane (2.75 g, 17.6 mmol) and potassium carbonate (6.1 g, 44.1 mmol) were added, and the mixture was stirred at room temperature overnight. The reaction solution was poured into water, the aqueous phase was extracted with ethyl acetate, and the merged organic phases were dried over anhydrous sodium sulfate and spun dry in vacuo to obtain a crude product. The crude product was separated and purified by silica gel column chromatography (petroleum ether/ethyl acetate = 3/1) to obtain 3-(ethylthio)pyridine-2-carbonitrile 6C (1.2 g, yield: 49.8%, yellow oily substance)).

MS (ESI): m/z = 165.1 [M + H]⁺

### Step 3: (3-(ethylthio)pyridin-2-yl)methanamine (6D)

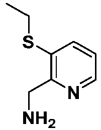

3-(Ethylthio)pyridine-2-carbonitrile 6C (1.1 g, 6.1 mmol) was dissolved in methanol (30 mL), and then 10% palladium/charcoal (catalytic amount) was added and reacted at room temperature for two days (during which the palladium/charcoal needed to be replaced with fresh ones). After suction filtration of the reaction solution, (3-(ethylthio)pyridin-2-yl)methanamine 6D (1.2 g, crude product) was obtained.

MS (ESI): m/z = 169.1 [M + H]⁺

### Step 4: tert-butyl (1-(((3-(ethylthio)pyridin-2-yl)methyl)amino)-2-methyl-1-oxopropan-2-yl)carbamate (6E)

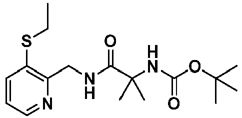

2-((*tert*-Butoxycarbonyl)amino)-2-methylpropionic acid (1.45 g, 7.13 mmol) and HATU (2.99 g, 7.86 mmol) were dissolved in *N,N*-dimethylformamide (30 mL)) at room temperature for half an hour. (3-(Ethylthio)pyridin-2-yl)methanamine 6D (1.2 g, 7.14 mmol) and DIEA (2.37 mL) were then added and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude product was separated and purified by silica gel column chromatography (petroleum ether/ethyl acetate = 3/1) to obtain *tert*-butyl (1-(((3-(ethylthio)pyridin-2-yl)methyl)amino)-2-methyl-1-oxoprop-2-yl)carbamate 6E (1.6 g, yield: 63.5%, pale yellow oily substance).

MS (ESI): m/z = 354.1 [M + H]⁺

### Step 5: tert-butyl (2-(8-(ethylthio)imidazo[1,5-a]pyridin-3-yl)propan-2-yl)carbamate (6F)

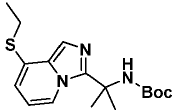

*tert-*Butyl (1-(((3-(ethylthio)pyridin-2-yl)methyl)amino)-2-methyl-1-oxopropan-2-yl)carbamate 6E (1.5 g, 4.24 mmol) was dissolved in dichloromethane (20 mL), Burgess reagent (2.02 g, 8.48 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and dried in vacuo to obtain a crude product. The crude product was separated and purified by silica gel column chromatography (petroleum ether/ethyl acetate = 5/1) to obtain *tert*-butyl (2-(8-(ethylthio)imidazo[1,5-a]pyridin-3-yl)propane-2-yl)carbamate 6F (570 mg, yield: 40.1%, pale yellow oily substance).

MS (ESI): m/z = 336.1 [M + H]⁺

### Step 6: 2-(8-(ethylthio)imidazo[1,5-a]pyridin-3-yl)propan-2-amine hydrochloride (6G)

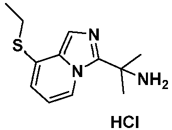

*tert-*Butyl (2-(8-(ethylthio)imidazo[1,5-a]pyridin-3-yl)propan-2-yl)carbamate 6F (570 mg, 1.7 mmol) was dissolved in a hydrochloric acid-methanol solution (20 mL), and reacted at room temperature overnight. The reaction solution was spun dry in vacuo to obtain 2-(8-(ethylthio)imidazo[1,5-a]pyridin-3-yl)propan-2-amine hydrochloride 6G (510 mg, crude product), which was directly used in the next step.

MS (ESI): m/z = 236.1 [M + H]⁺

### Step 7: tert-butyl (1R,5S,6R)-6-((2-(8-(ethylthio)imidazo[1,5-a]pyridin-3-yl)propan-2-yl) carbamoyl)-3-azabicyclo[3.1.0]hexane-carboxylate (6H)

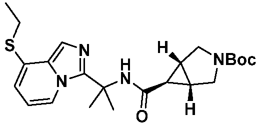

(1*R*,5*S*,6*R*)-3-(*tert*-butoxycarbonyl)-3-azabicyclo[3.1.0]hexane-6-carboxylic acid 1F (300 mg, 1.32 mmol) and HATU (501 mg, 1.32 mmol) were dissolved in *N,N*-dimethylformamide (10 mL) and reacted at room temperature for half an hour. 2-(8-(Ethylthio)imidazo[1,5-a]pyridin-3-yl)propan-2-amine 6G (260 mg, 1.1 mmol) and DIEA (0.5 mL) were then added, and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude product was separated and purified by silica gel column chromatography (dichloromethane/methanol = 15/1) to obtain *tert-*butyl (1*R*,5*S*,6*R*)-6-((2-(8-(ethylthio)imidazo[1,5-a]pyridin-3-yl)propan-2-yl)carbamoyl)-3-azabicyclo[3.1.0]hexane-carboxylate 6H (410 mg, yield: 83.7%, yellow oily substance).

MS (ESI): m/z = 445.2 [M + H]⁺

### Step 8: (1R,5S,6R)-N-(2-(8-(ethylthio)imidazo[1,5-a]pyridin-3-yl)propan-2-yl)-3-azabicyclo[3.1.0]hexane-6-carboxamide hydrochloride (Compound 6)

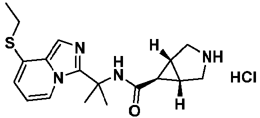

*tert*-Butyl (1*R*,5*S*,6*R*)-6-((2-(8-(ethylthio)imidazo[1,5-a]pyridin-3-yl)prop-2-yl)carbamoyl)-3-azabicyclo[3.1.0]hexane-carboxylate 6H (410 mg, 0.922 mmol) was dissolved in a hydrochloric acid-methanol solution (10 mL) and reacted at room temperature for 3 hours. The reaction solution was spun dry in vacuo to remove the solvent, and then lyophilized to obtain the target compound (1*R*,5*S,*6*R*)-*N*-(2-(8-(ethylthio)imidazo[1,5-a]pyridine-3-yl)propan-2-yl)-3-azabicyclo[3.1.0]hexane-6-carboxamide hydrochloride (Compound 6) (240 mg, yield: 68.4%, yellowish solid).

¹H NMR (400 MHz, DMSO-d₆) δ 9.16 (s, 1H), 8.32 (t, 1H), 7.89 (s, 1H), 6.98 (d, 2H), 3.27 (m, 4H), 3.16 (q, 2H), 1.91 (m, 1H), 1.80 (m, 2H), 1.75 (s, 6H), 1.32 (t, 3H).

MS (ESI): m/z = 345.1 [M + H]⁺

### Example 7

### (1R,5S,6R)-N-(2-(8-(Isopropylthio)imidazo[1,5-a]pyridin-3-yl)prop-2-yl)-3-azabicyclo[3.1.0]hexane-6-carboxamide (Compound 7)

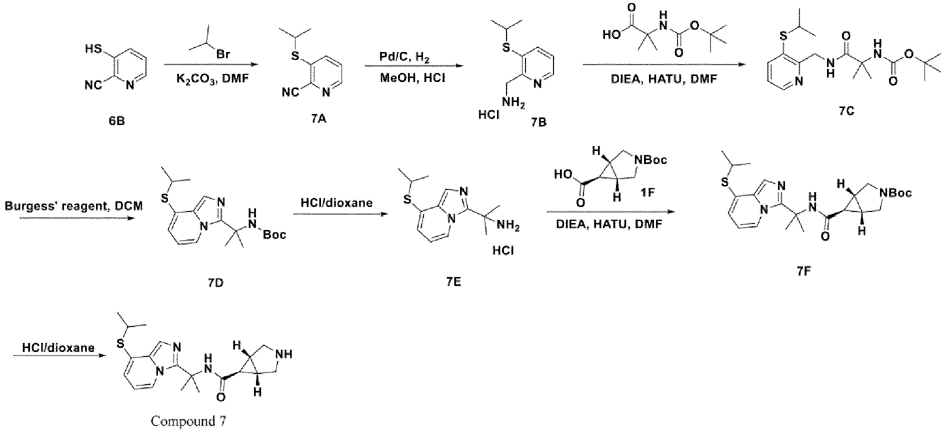

### Step 1: 3-(isopropylthio)pyridine-2-carbonitrile (7A)

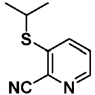

After 6B was prepared as described above, 2-bromopropane (0.97 g, 7.9 mmol) and potassium carbonate (1.83 g, 13.2 mmol) were added to the reaction solution, and the mixture was stirred at room temperature overnight. The reaction solution was poured into water, the aqueous phase was extracted with ethyl acetate, and the merged organic phases were dried over anhydrous sodium sulfate, and dried in vacuo to obtain 3-(isopropylthio)pyridine-2-carbonitrile 7A (crude product). The crude product was separated and purified by silica gel column chromatography (petroleum ether/ethyl acetate = 3/1) to obtain 3-(isopropylthio)pyridine-2-carbonitrile 7A (680 mg, yield: 52.7%, yellow oily substance).

MS (ESI): m/z = 179.1 [M + H]⁺

### Step 2: (3-(isopropylthio)pyridin-2-yl)methanamine hydrochloride (7B)

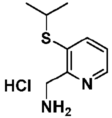

3-(Isopropylthio)pyridine-2-carbonitrile 7A (0.68 g, 3.8 mmol) was dissolved in methanol (15 mL) and 1 mL of concentrated hydrochloric acid was added, followed by addition of 10% palladium/charcoal (catalyst) amount) and reacted at room temperature overnight. The reaction solution was filtered by suction using celite to obtain (3-(isopropylthio)pyridin-2-yl)methanamine hydrochloride 7B (0.6 g, crude product), which was directly used in the next step.

MS (ESI): m/z = 183.1 [M + H]⁺

### Step 3: tert-butyl (1-(((3-(isopropylthio)pyridin-2-yl)methyl)amino)-2-methyl-1-oxoprop-2-yl)carbamate (7C)

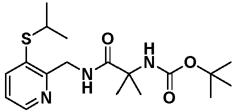

2-((*tert*-Butoxycarbonyl)amino)-2-methylpropionic acid (0.80 g, 3.96 mmol) and HATU (1.50 g, 3.96 mmol) were dissolved in DMF (15 mL) and reacted at room temperature for 20 min, then (3-(isopropylthio)pyridin-2-yl)methanamine 7B (0.60 g, 3.29 mmol) and DIEA (1.27 g, 9.87 mmol) were added, and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain crude *tert-*butyl (1-(((3-(isopropylthio)pyridin-2-yl)methyl)amino)-2-methyl-1-oxoprop-2-yl)carbamate 7C. After separation and purification by silica gel column chromatography (petroleum ether/ethyl acetate = 2/1), *tert-*butyl (1-(((3-(isopropylthio)pyridin-2-yl)methyl)amino)-2-methyl-1-oxoprop-2-yl)carbamate 7C (740 mg, yield: 61.1%, pale yellow oily substance) was obtained.

MS (ESI): m/z = 368.1 [M + H]⁺

### Step 4: tert-butyl (2-(8-(ethylthio)imidazo[1,5-a]pyridin-3-yl)propan-2-yl)carbamate (7D)

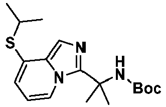

*tert-*Butyl (1-(((3-(isopropylthio)pyridin-2-yl)methyl)amino)-2-methyl-1-oxoprop-2-yl)carbamate 7C (0.74 g, 2.02 mmol) was dissolved in dichloromethane (10 mL), Burgess reagent (1.92 g, 8.08 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and dried in vacuo to obtain the crude *tert*-butyl (2-(8-(ethylthio)imidazo[1,5-a]pyridin-3-yl)prop-2-yl)carbamate 7D. After separation and purification by silica gel column chromatography (petroleum ether/ethyl acetate = 2/1), *tert-*butyl (2-(8-(ethylthio)imidazo[1,5-a]pyridin-3-yl)propan-2-yl)carbamate 7D (400 mg, yield: 56.9%, pale yellow oily substance) was obtained.

MS (ESI): m/z = 350.1 [M + H]⁺

### Step 5: 2-(8-(isopropylthio)imidazo[1,5-a]pyridin-3-yl)propan-2-amine hydrochloride (7E)

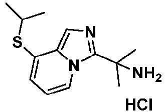

*tert*-Butyl (2-(8-(ethylthio)imidazo[1,5-a]pyridin-3-yl)propan-2-yl)carbamate 7D (100 mg, 0.29 mmol) was dissolved in a hydrochloric acid-dioxane solution (8 mL), and reacted at room temperature for 2 hours. The reaction solution was spun dry in vacuo to obtain 2-(8-(isopropylthio)imidazo[1,5-a]pyridin-3-yl)propan-2-amine hydrochloride 7E (70 mg, crude product), which was directly used in the next step.

MS (ESI): m/z = 250.1 [M + H]⁺

### Step 6: tert-butyl (1R,5S,6R)-6-((2-(8-(isopropylthio)imidazo[1,5-a]pyridin-3-yl)prop-2-yl)carbamoyl)-3-azabicyclo[3.1.0]-3-carboxylate (7F)

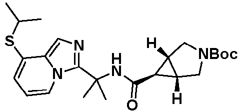

(1*R*,5*S*,6*R*)-3-(*tert*-Butoxycarbonyl)-3-azabicyclo[3.1.0]hexane-6-carboxylic acid 1F (109.4 mg, 0.48 mmol) and HATU (183.1 mg, 0.48 mmol) were dissolved in DMF (5 mL) and reacted at room temperature for half an hour. 2-(8-(Isopropylthio)imidazo[1,5-a]pyridin-3-yl)propan-2-amine 7E (70 mg, 0.28 mmol) and DIEA (108.8 mg, 0.84 mmol) were then added mmol) and the mxiture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain crude *tert-*butyl (1*R*,5*S*,6*R*)-6-((2-(8-(Isopropylthio)imidazo[1,5-a]pyridin-3-yl)prop-2-yl)carbamoyl)-3-azabicyclo[3.1.0]-3-carboxylate 7F (150 mg, crude product).

MS (ESI): m/z = 459.1 [M + H]⁺

### Step 7: (1R,5S,6R)-N-(2-(8-(isopropylthio)imidazo[1,5-a]pyridin-3-yl)propan-2-yl)-3-azabicyclo[3.1.0]hexane-6-carboxamide (Compound 7)

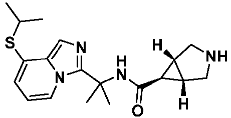

*tert-*Butyl (1*R*,5*S*,6*R*)-6-((2-(8-(isopropylthio)imidazo[1,5-a]pyridin-3-yl)propan-2-yl)carbamoyl)-3-azabicyclo[3.1.0]-3-carboxylate 7F (150 mg) was dissolved in a hydrochloric acid-dioxane solution (5 mL) and reacted at room temperature for 1 hour. The reaction solution was subjected to rotary evaporation to remove the solvent to obtain a crude product. The crude product was purified by preparative HPLC to give the target compound (1*R*,5*S*,6*R*)-N-(2-(8-(isopropylthio)imidazo[1,5-a]pyridine-3-yl)propan-2-yl)-3-azabicyclo[3.1.0]hexane-6-carboxamide (Compound 7) (60 mg, yield: 76.9%, white solid).

¹H NMR (400 MHz, DMSO-d₆) δ 8.38 (s, 1H), 8.17 (d, 1H), 7.23 (s, 1H), 6.72 (d, 1H), 6.60 (t, 1H), 3.67 (m, 1H), 2.87 (d, 2H), 2.68 (d, 2H), 1.64 (s, 6H), 1.57 (t, 1H), 1.47 (m, 2H), 1.31 (d, 6H).

MS (ESI): m/z = 359.1 [M + H]⁺

### Example 8

### (1R,5S,6R)-N-(2-(8-((Cyclopropylmethyl)thio)))thio)imidazo[1,5-a]pyridin-3-yl)propane-2-yl)-3-azabicyclo[3.1.0]hexane-6-carboxamide (Compound 8)

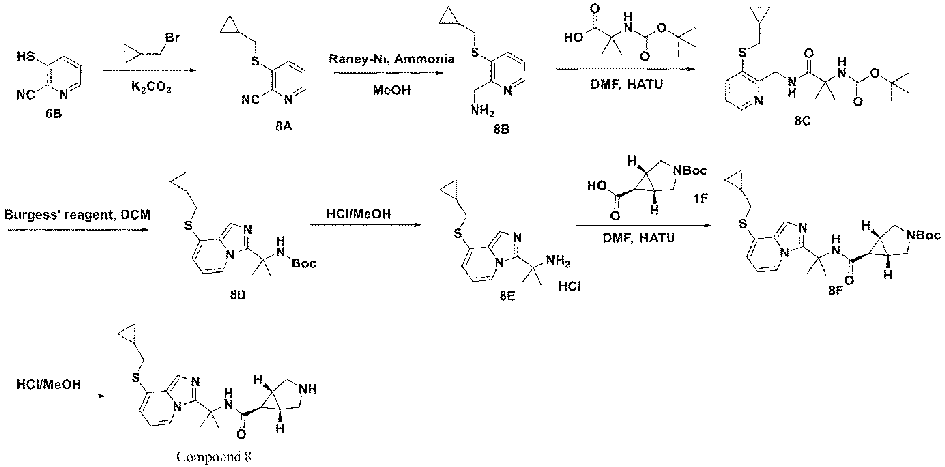

### Step 1: 3-((cyclopropylmethyl)thio)pyridine-2-carbonitrile (8A)

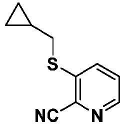

After 6B was prepared as described above, (bromomethyl)cyclopropane (964 mg, 7.2 mmol) and potassium carbonate (2.98 g, 21.6 mmol) were added to the reaction solution, and the mixture was stirred at room temperature overnight. The reaction solution was poured into water, the aqueous phase was extracted with ethyl acetate, and the merged organic phases were dried over anhydrous sodium sulfate, and dried in vacuo to obtain 3-((cyclopropylmethyl)sulfanyl)pyridine-2-carbonitrile 8A (crude product) (1.1 g, yield: 80.3%, yellow oily substance).

MS (ESI): m/z = 191.0 [M + H]⁺

### Step 2: (3-((cyclopropylmethyl)thio)pyridin-2-yl)methanamine (8B)

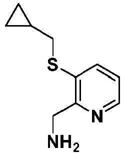

3-((Cyclopropylmethyl)thio)pyridine-2-carbonitrile 8A (1.1 g, 5.7 mmol) was dissolved in methanol (30 mL), followed by addition of Raney nickel (catalytic amount) and ammonia (3 mL) and reacted at room temperature for 18 hours. After suction filtration of the reaction solution, (3-((cyclopropylmethyl)sulfanyl)pyridin-2-yl)methanamine 8B (crude product) (1.0 g, yield: 89.3%, yellow oily substance) was obtained.

MS (ESI): m/z = 195.0 [M + H]⁺

### Step 3: tert-butyl (1-(((3-((cyclopropylmethyl)thio)pyridin-2-yl)methyl)amino)-2-methyl-1-oxoprop-2-yl)carbamate (8C)

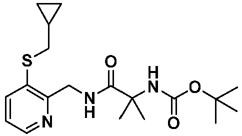

2-((*tert*-Butoxycarbonyl)amino)-2-methylpropionic acid (1.04 g, 5.15 mmol) and HATU (2.54 g, 6.69 mmol) were dissolved in *N,N*-dimethylformamide (15 mL)) at room temperature for half an hour. (3-((Cyclopropylmethyl)sulfanyl)pyridin-2-yl)methanamine 8B (1.0 g, 5.15 mmol) and triethylamine (1.56 g, 15.45 mmol) were then added and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude *tert*-butyl (1-(((3-((cyclopropylmethyl)sulfanyl)pyridin-2-yl)methyl)amino)-2-methyl-1-oxopropan-2-yl)carbamate 8C was separated and purified by silica gel column chromatography (petroleum ether/ethyl acetate = 3/1) to obtain *tert-*butyl (1-(((3-((cyclopropylmethyl)sulfanyl)pyridin-2-yl)methyl)amino)-2-methyl-1-oxopropan-2-yl)carbamate 8C (900 mg, yield: 46.1%, pale yellow oily substance).

MS (ESI): m/z = 380.2 [M + H]⁺

### Step 4: tert-butyl (2-(8-((cyclopropylmethyl)sulfanyl)imidazo[1,5-a]pyridin-3-yl)propan-2-yl)carbamate (8D)

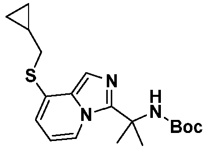

*tert-*Butyl (1-(((3-((cyclopropylmethyl)sulfanyl)pyridin-2-yl)methyl)amino)-2-methyl-1-oxoprop-2-yl)carbamate 8C (900 mg, 4.24 mmol) was dissolved in dichloromethane (10 mL), Burgess reagent (1.51 g, 6.36 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and dried in vacuo to obtain crude *tert*-butyl (2-(8-((cyclopropylmethyl)thio)imidazo[1,5-a]pyridin-3-yl)prop-2-yl)carbamate. The crude product was separated and purified by silica gel column chromatography (petroleum ether/ethyl acetate = 5/1) to obtain *tert*-butyl (2-(8-((cyclopropylmethyl)thio)imidazo[1,5-a]pyridine-3-yl)propan-2-yl)carbamate 8D (600 mg, yield: 70.0%, pale yellow oily substance).

MS (ESI): m/z = 362.2 [M + H]⁺

### Step 5: 2-(8-((cyclopropylmethyl)thio)imidazo[1,5-a]pyridin-3-yl)propan-2-amine hydrochloride (8E)

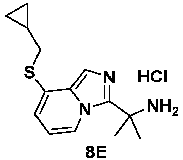

*tert-*Butyl (2-(8-((cyclopropylmethyl)sulfanyl)imidazo[1,5 -a]pyridin-3 -yl)propan-2-yl)carbamate 8D (600 mg, 1.7 mmol) was dissolved in a hydrochloric acid-methanol solution (10 mL) and reacted at room temperature for 2 hours. The reaction solution was spun dry in vacuo to obtain 2-(8-((cyclopropylmethyl)sulfanyl)imidazo[1,5-a]pyridin-3-yl)propan-2-amine hydrochloride 8E (500 mg, crude product), which was directly used in the next step.

MS (ESI): m/z = 262.1 [M + H]⁺

### Step 6: tert-butyl (1R,5S,6R)-6-((2-(8-((cyclopropylmethyl)thio)imidazo[1,5-a]pyridin-3-yl)propan-2-yl)carbamoyl)-3-azabicyclo[3.1.0]hexane-3-carboxylate (8F)

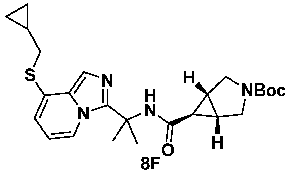

(1*R*,5*S*,6*R*)-3-(*tert*-Butoxycarbonyl)-3-azabicyclo[3.1.0]hexane-6-carboxylic acid 1F (60.8 mg, 0.27 mmol) and HATU (133 mg, 0.35 mmol) were dissolved in *N,N*-dimethylformamide (2 mL) and reacted at room temperature for half an hour. 2-(8-((Cyclopropylmethyl)sulfanyl)imidazo[1,5-a]pyridin-3-yl)propan-2-amine 8E (70 mg, 0.27 mmol) and triethylamine (81.8 mg, 0.81 mmol) were added and stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude *tert-*butyl (1*R*,5*S*,6*R*)-6-((2-(8-((cyclopropylmethyl)thio)imidazo[1,5-a]pyridin-3-yl)propane-2-yl)carbamoyl)-3-azabicyclo[3.1.0]hexane-3-carboxylate 8Fwas separated and purified by silica gel column chromatography (dichloromethane/methanol = 20/1) to obtain *tert-*butyl (1*R*,5*S*,6*R*)-6-((2-(8-((cyclopropylmethyl)thio))imidazo[1,5-a]pyridin-3-yl)propan-2-yl)carbamoyl)-3-azabicyclo[3.1.0]hexane-3-carboxylate 8F (60 mg, yield: 47.6%, yellow oily substance).

MS (ESI): m/z = 471.2 [M + H]⁺

### Step 7: (1R,5S,6R)-N-(2-(8-((cyclopropylmethyl)thio)imidazo[1,5-a]pyridin-3-yl)propan-2-yl)-3-azabicyclo[3.1.0]hexane-6-carboxamide (Compound 8)

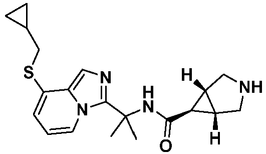

*tert-*Butyl (1*R*,5*S*,6*R*)-6-((2-(8-((cyclopropylmethyl)sulfanyl)imidazo[1,5-a]pyridin-3-yl)propan-2-yl)carbamoyl)-3-azabicyclo[3.1.0]hexane-3-carboxylate 8F (60 mg, 0.127 mmol) was dissolved in a hydrochloric acid-methanol solution (5 mL) and reacted at room temperature for 3 hours. The reaction solution was spun dry in vacuo to remove the solvent to obtain a crude product. The crude product was purified by preparative HPLC to give (1*R*,5*S*,6*R*)-*N*-(2-(8-((cyclopropylmethyl)sulfanyl)imidazo[1,5-a]pyridine-3-yl)propan-2-yl)-3-azabicyclo[3.1.0]hexane-6-carboxamide (Compound 8) (20 mg, yield: 42.5%, white solid).

¹H NMR (400 MHz, CD₃OD) δ 8.50 (s, 1H), 8.13 (d, 1H), 7.34 (s, 1H), 6.74 (d, 1H), 6.61 (t, 1H)), 3.35 (s, 2H), 3.28-3.25 (m, 2H), 2.98 (d, 2H), 1.92 (m, 2H), 1.78 (s, 6H), 1.66 (m, 1H)), 1.09 (m, 1H), 0.59 (dd, 2H), 0.29 (dd, 2H).

MS (ESI): m/z = 371.2 [M + H]⁺

### Example 9

### (1R,5S,6R)-N-(2-(8-(Benzylthio)imidazo[1,5-a]pyridin-3-yl)prop-2-yl)-3-azabicyclo[3.1.0]hexane-6-carboxamide (Compound 9)

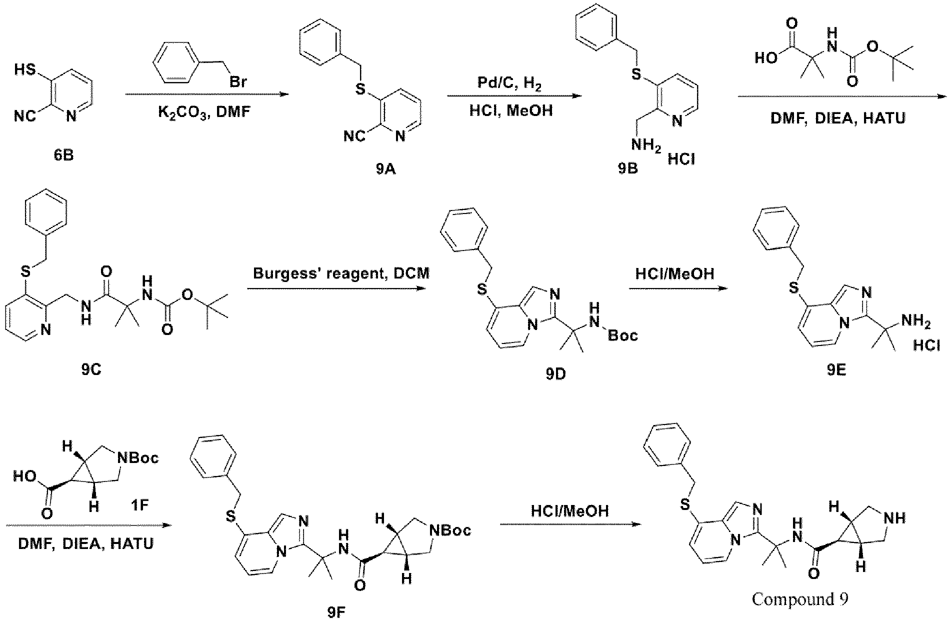

### Step 1: 3-(benzylthio)pyridine-2-carbonitrile (9A)

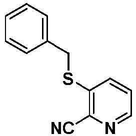

After 6B was prepared as described above, (bromomethyl)benzene (1.5 g, 8.8 mmol) and potassium carbonate (4.1 g, 29.4 mmol) were added to the reaction solution, and the mixture was stirred at room temperature overnight. The reaction solution was poured into water, the aqueous phase was extracted with ethyl acetate, and the merged organic phases were dried over anhydrous sodium sulfate, and dried in vacuo to obtain 3-(benzylthio)pyridine-2-carbonitrile 9A (1.3 g, yellow oily substance), and the crude product was directly used in the next step.

MS (ESI): m/z = 227.1 [M + H]⁺

### Step 2: (3-(benzylthio)pyridin-2-yl)methanamine hydrochloride (9B)

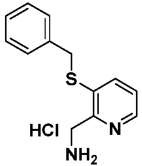

3-(Benzylthio)pyridine-2-carbonitrile 9A (1.3 g, 5.75 mmol) was dissolved in methanol (30 mL), hydrochloric acid (2 mL) was added, followed by addition of 10% palladium/charcoal (catalytic amount) and reacted at room temperature for three days (during which the palladium/charcoal needed to be replaced with a fresh one once). After suction filtration of the reaction solution, (3-(benzylthio)pyridin-2-yl)methanamine hydrochloride 9 B (1.2 g, crude product) was obtained, which was directly used in the next reaction.

MS (ESI): m/z = 231.1 [M + H]⁺

### Step 3: tert-butyl (1-(((3-(benzylthio)pyridin-2-yl)methyl)amino)-2-methyl-1-oxopropan-2-yl)carbamate (9C)

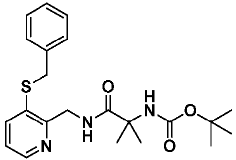

2-((*tert*-Butoxycarbonyl)amino)-2-methylpropionic acid (1.06 g, 5.2 mmol) and HATU (2.15 g, 5.65 mmol) were dissolved in *N,N*-dimethylformamide (30 mL)) at room temperature for half an hour. (3-(Benzylthio)pyridin-2-yl)methanamine 9B (1.20 g, 5.2 mmol) and DIEA (2.1 mL) were then added and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product 9C. The crude product was separated and purified by silica gel column chromatography (petroleum ether/ethyl acetate = 3/1) to obtain *tert-*butyl (1-(((3-(benzylthio)pyridin-2-yl)methyl)amino)-2-methyl-1-oxoprop-2-yl)carbamate 9C (600 mg, yield: 27.9%, pale yellow oily substance).

MS (ESI): m/z = 416.1 [M + H]⁺

### Step 4: tert-butyl (2-(8-(benzylthio)imidazo[1,5-a]pyridin-3-yl)propan-2-yl)carbamate (9D)

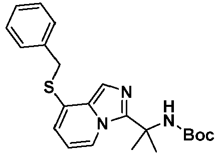

*tert-*Butyl (1-(((3-(benzylthio)pyridin-2-yl)methyl)amino)-2-methyl-1-oxopropan-2-yl)carbamate 9C (600 mg, 1.45 mmol) was dissolved in dichloromethane (20 mL), Burgess reagent (1.38 g, 5.8 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and dried in vacuo to obtain crude (2-(8-(benzylthio)imidazolium)[1,5-a]pyridin-3-yl)propan-2-yl)carbamate 9D. The crude product was separated and purified by silica gel column chromatography (petroleum ether/ethyl acetate = 5/1) to obtain *tert-*butyl 2-(8-(benzylthio)imidazo[1,5-a]pyridin-3-yl)propane-2-yl)carbamate 9D (230 mg, yield: 40%, pale yellow oily substance).

MS (ESI): m/z = 398.1 [M + H]⁺

### Step 5: 2-(8-(benzylthio)imidazo[1,5-a]pyridin-3-yl)propan-2-amine hydrochloride (9E)

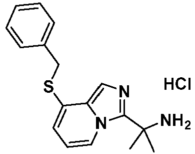

*tert*-Butyl (2-(8-(benzylthio)imidazo[1,5-a]pyridin-3-yl)propan-2-yl)carbamate 9D (230 mg, 0.58 mmol) was dissolved in a hydrochloric acid-methanol solution (10 mL) and reacted at room temperature overnight. The reaction solution was spun dry in vacuo to obtain 2-(8-(benzylthio)imidazo[1,5-a]pyridin-3-yl)propan-2-amine hydrochloride 9E (200 mg, crude product), which was directly used in the next step.

MS (ESI): m/z = 298.1 [M + H]⁺

### Step 6: tert-butyl (1R,5S,6R)-6-((2-(8-(benzylthio)imidazo[1,5-a]pyridin-3-yl)prop-2-yl)carbamoyl)-3-azabicyclo[3.1.0]hexane-3-carboxylate (9F)

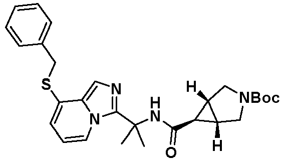

(1*R*,5*S*,6*R*)-3-(*tert*-Butoxycarbonyl)-3-azabicyclo[3.1.0]hexane-6-carboxylic acid 1F (182 mg, 0.8 mmol) and HATU (304 mg, 0.8 mmol) were dissolved in *N,N*-dimethylformamide (10 mL) and reacted at room temperature for half an hour. 2-(8-(Benzylthio)imidazo[1,5-a]pyridin-3-yl)propan-2-amine 9E (200 mg, 0.67 mmol) and DIEA (0.5 mL) were then added, and the mixture was stirred at room temperature overnight. The reaction solution was poured into water, the aqueous phase was extracted with ethyl acetate, and the merged organic phases were washed with saturated brine, dried over anhydrous sodium sulfate, and spun dry in vacuo to obtain a crude product 9F. The crude product was separated and purified by silica gel column chromatography (dichloromethane/methanol = 15/1) to obtain *tert-*butyl (1*R*,5*S*,6*R*)-6-((2-(8-(benzylthio)imidazo[1,5-a]pyridin-3-yl)propan-2-yl)carbamoyl)-3-azabicyclo[3.1.0]hexane-3-carboxylate 9F (170 mg, yield: 50.1%, yellow oily substance).

MS (ESI): m/z = 507.1 [M + H]⁺

### Step 7: (1R,5S,6R)-N-(2-(8-(benzylthio)imidazo[1,5-a]pyridin-3-yl)propan-2-yl)-3-azabicyclo[3.1.0]hexane-6-carboxamide (Compound 9)

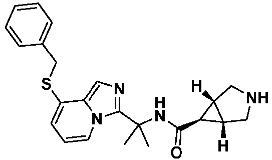

*tert*-Butyl (1*R*,5*S*,6*R*)-6-((2-(8-(benzylthio)imidazo[1,5-a]pyridin-3-yl)prop-2-yl)carbamoyl)-3-azabicyclo[3.1.0]hexane-3-carboxylate 9F (170 mg, 0.336 mmol) was dissolved in a hydrochloric acid-methanol solution (10 mL) and reacted at room temperature for 3 hours. The reaction solution was spun dry in vacuo to remove the solvent to obtain a crude product. The crude product was purified by preparative HPLC to give a white solid product (Compound 9) (43 mg, yield: 31.5%).

¹H NMR (400 MHz, DMSO-d₆) δ 8.38 (s, 1H), 8.12 (d, 1H), 7.40 (d, 2H), 7.32 (t, 2H), 7.27 (d, 1H), 7.23 (s, 1H), 6.67 (d, 1H), 6.56 (t, 1H), 4.35 (s, 2H), 2.88 (d, 2H), 2.70 (d, 2H), 1.64 (s, 6H), 1.56 (t, 1H), 1.49 (m, 2H).

MS (ESI): m/z = 407.0 [M + H]⁺

### Example 10

### (1R,5S,6R)-N-(2-(8-(Isobutylthio)imidazo[1,5-a]pyridin-3-yl)prop-2-yl)-3-azabicyclo[3.1.0]hexane-6-carboxamide (Compound 10)

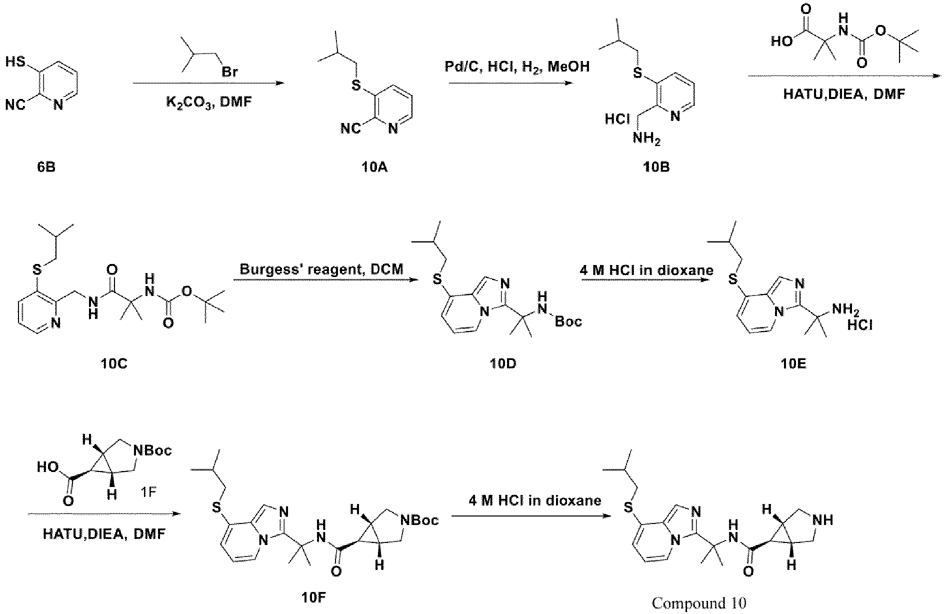

### Step 1: 3-(isobutylthio)pyridine-2-carbonitrile (10A)

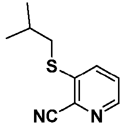

After 6B was prepared as described above, 1-bromo-2-methylpropane (1.48 mg, 10.87 mmol) and potassium carbonate (3 g, 21.74 mmol) were added to the reaction solution, and the mixture was stirred at room temperature overnight. The reaction solution was poured into water, the aqueous phase was extracted with ethyl acetate, and the merged organic phases were dried over anhydrous sodium sulfate, and dried in vacuo to obtain a crude product. The crude product was separated and purified by silica gel column chromatography (petroleum ether/ethyl acetate = 3/1) to obtain a yellow oily substance 3-(isobutylthio)pyridine-2-carbonitrile 10A (800 mg, yield: 57.5%).

MS (ESI): m/z = 193.1 [M + H]⁺

### Step 2: (3-(isobutylthio)pyridin-2-yl)methanamine hydrochloride (10B)

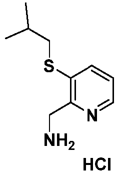

3-(Isobutylthio)pyridine-2-carbonitrile 10A (800 mg, 4.17 mmol) was dissolved in methanol (30 mL), and 1 mL of concentrated hydrochloric acid was added, followed by addition of 10% palladium/charcoal (catalytic amount) and reacted at room temperature overnight. After suction filtration of the reaction solution, crude (3-(isobutylthio)pyridin-2-yl)methanamine hydrochloride 10B (1.0 g) was obtained.

MS (ESI): m/z = 197.1 [M + H]⁺

### Step 3: tert-butyl (1-(((3-(isobutylthio)pyridin-2-yl)methyl)amino)-2-methyl-1-oxopropan-2-yl)carbamate (10C)

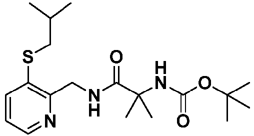

2-((*tert*-Butoxycarbonyl)amino)-2-methylpropionic acid (1.01 g, 5.0 mmol) and HATU (1.9 g, 5.0 mmol) were dissolved in DMF (30 mL) and reacted at room temperature for 15 min, then (3-(isobutylthio)pyridin-2-yl)methanamine 10B (1 g, 4.17 mmol) and DIEA (1.27 g, 9.87 mmol) were added, and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude product was separated and purified by silica gel column chromatography (petroleum ether/ethyl acetate = 2/1) to obtain a pale yellow solid *tert*-butyl (1-(((3-(isobutylthio)pyridin-2-yl)methyl)amino)-2-methyl-1-oxopropan-2-yl)carbamate 10C (680 mg, yield: 61.1%).

MS (ESI): m/z = 382.1 [M + H]⁺

### Step 4: tert-butyl (2-(8-(isobutylthio)imidazo[1,5-a]pyridin-3-yl)propan-2-yl)carbamate (10D)

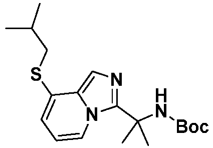

*tert-*Butyl (1-(((3-(isobutylthio)pyridin-2-yl)methyl)amino)-2-methyl-1-oxoprop-2-yl)carbamate 10C (680 mg, 1.78 mmol) was dissolved in dichloromethane (20 mL), Burgess reagent (1.68 g, 7.14 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and dried in vacuo to obtain a crude product. The crude product was separated and purified by silica gel column chromatography (petroleum ether/ethyl acetate = 2/1) to obtain *tert*-butyl (2-(8-(isobutylthio)imidazo[1,5-a]pyridin-3-yl)prop-2-yl)carbamate 10D (280 mg, yield: 56.9%, pale yellow oily substance).

MS (ESI): m/z = 364.1 [M + H]⁺

### Step 5: 2-(8-(isobutylthio)imidazo[1,5-a]pyridin-3-yl)propan-2-amine hydrochloride (10E)

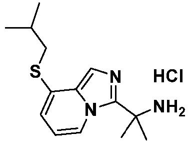

*tert*-Butyl (2-(8-(isobutylthio)imidazo[1,5-a]pyridin-3-yl)propan-2-yl)carbamate 10D (280 mg, 0.77 mmol) was dissolved in a hydrochloric acid-dioxane solution (8 mL) and reacted at room temperature overnight. The reaction solution was spun dry in vacuo to obtain 2-(8-(isobutylthio)imidazo[1,5-a]pyridin-3-yl)propan-2-amine hydrochloride 10E (250 mg, crude product), which was directly used in the next step.

MS (ESI): m/z = 264.1 [M + H]⁺

### Step 6: tert-butyl (1R,5S,6R)-6-((2-(8-(isobutylthio)imidazo[1,5-a]pyridin-3-yl)propan-2-yl)carbamoyl)-3-azabicyclo[3.1.0]hexane-3-carboxylate (10F)

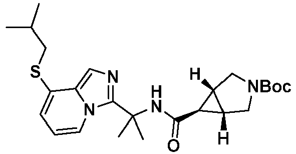

(1*R*,5*S*,6*R*)-3-(*tert*-Butoxycarbonyl)-3-azabicyclo[3.1.0]hexane-6-carboxylic acid 1F (258 mg, 1.14 mmol) and HATU (433 mg, 1.14 mmol) were dissolved in DMF (10 mL) and reacted at room temperature for half an hour. 2-(8-(Isobutylthio)imidazo[1,5-a]pyridin-3-yl)propan-2-amine 10E (250 mg, 0.95 mmol) and DIEA (368 mg, 2.85 mmol) were then added and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude product was separated and purified by silica gel column chromatography (dichloromethane/methanol = 15/1) to obtain *tert-*butyl (1*R*,5*S*,6*R*)-6-((2-(8-(isobutylthio)imidazolium)[1,5-a]pyridin-3-yl)propan-2-yl)carbamoyl)-3-azabicyclo[3.1.0]hexane-3-carboxylate 10F (300 mg, yield: 77.7%, yellow oily substance).

MS (ESI): m/z = 473.1 [M + H]⁺

### Step 7: (1R,5S,6R)-N-(2-(8-(isobutylthio)imidazo[1,5-a]pyridin-3-yl)propan-2-yl)-3-azabicyclo[3.1.0]hexane-6-carboxamide (Compound 10)

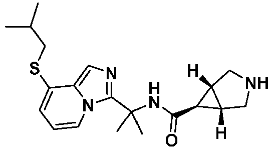

*tert-*Butyl (1*R*,5*S*,6*R*)-6-((2-(8-(isobutylthio)imidazo[1,5-a]pyridin-3-yl)prop-2-yl)amino Formyl)-3-azabicyclo[3.1.0]hexane-3-carboxylate 10F (300 mg, 0.63 mmol) was dissolved in hydrochloric acid-dioxane solution (5 mL) and reacted at room temperature for 1 hour. The reaction solution was spun dry in vacuo to remove the solvent, and lyophilized to obtain the target compound (1*R*,5*S*,6*R*)-*N*-(2-(8-(isobutylthio) imidazo[1,5-a]pyridine-3-yl)propan-2-yl)-3-azabicyclo[3.1.0]hexane-6-carboxamide (Compound 10) (60 mg, yield: 76.9%, white solid).

¹H NMR (400 MHz, DMSO-d₆) δ 8.40 (s, 1H), 8.12 (d, 1H), 7.22 (s, 1H), 6.64 (d, 1H), 6.58 (t, 1H), 2.95 (d, 2H), 2.89 (d, 2H), 2.72 (d, 2H), 1.88 (m, 1H), 1.64 (s, 6H), 1.57 (m, 1H), 1.50 (m, 2H), 1.03 (d, 6H).

MS (ESI): m/z = 373.1 [M + H]⁺

### Example 11

### (1R,5S,6R)-N-(2-(8-((Cyclobutylmethyl)thio)imidazo[1,5-a]pyridin-3-yl)propan-2-yl)-3-Azabicyclo[3.1.0]hexane-6-carboxamide (Compound 11)

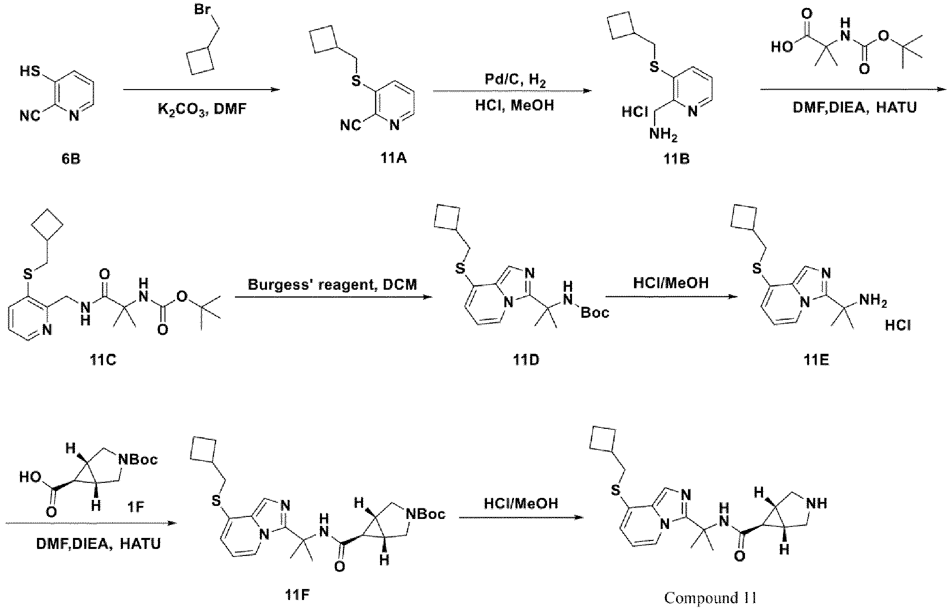

### Step 1: 3-((cyclobutylmethyl)thio)pyridine-2-carbonitrile (11A)

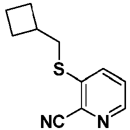

After 6B was prepared as described above, cyclobutylbromomethane (1.31 g, 8.80 mmol) and potassium carbonate (3.05 g, 22.0 mmol) were added to the reaction solution, and the mixture was stirred at room temperature overnight. The reaction solution was poured into water, the aqueous phase was extracted with ethyl acetate, the merged organic phases were dried over anhydrous sodium sulfate, and dried in vacuo to obtain 3-((cyclobutylmethyl)sulfanyl)pyridine-2-carbonitrile 11A (crude product). The crude product was separated and purified by silica gel column chromatography (petroleum ether/ethyl acetate = 3/1) to obtain 3-((cyclobutylmethyl)thio)pyridine-2-carbonitrile 11A (840 mg, yield: 56%, yellow oily substance).

MS (ESI): m/z = 205.1 [M + H]⁺

### Step 2: (3-((cyclobutylmethyl)sulfanyl)pyridin-2-yl)methanamine hydrochloride (11B)

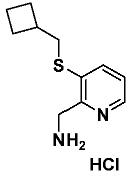

3-((Cyclobutylmethyl)sulfanyl)pyridine-2-carbonitrile 11A (840 mg, 4.12 mmol) was dissolved in methanol (20 mL), hydrochloric acid (1 mL) was added, followed by addition of 10% palladium/charcoal (catalytic amount) and reacted for three days in a hydrogen atmosphere at room temperature (during which the palladium/charcoal needed to be replaced with fresh ones). After suction filtration of the reaction solution, (3-((cyclobutylmethyl)sulfanyl)pyridin-2-yl)methanamine hydrochloride 11B (1.07 g, crude product) was obtained.

MS (ESI): m/z = 209.1 [M + H]⁺

### Step 3: tert-butyl (1-(((3-((cyclobutylmethyl)sulfanyl)pyridin-2-yl)methyl)amino)-2-methyl-1-oxoprop-2-yl)carbamate (11C)

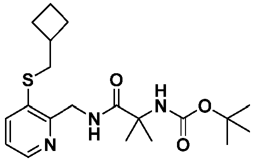

2-((*tert*-Butoxycarbonyl)amino)-2-methylpropionic acid (1.05 g, 5.14 mmol) and HATU (2.15 g, 5.65 mmol) were dissolved in *N,N*-dimethylformamide (30 mL)), and reacted at room temperature for half an hour, then (3-((cyclobutylmethyl)sulfanyl)pyridin-2-yl)methanamine 11B (1.07 g, 5.14 mmol) and DIEA (2.1 mL) were added, and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product. The crude *tert-*butyl (1-(((3-((cyclobutylmethyl)sulfanyl)pyridin-2-yl)methyl)amino)-2-methyl-1-oxoprop-2-yl)carbamate 11Cwas separated and purified by silica gel column chromatography (petroleum ether/ethyl acetate = 3/1) to obtain *tert-*butyl (1-(((3-((cyclobutylmethyl)sulfanyl)pyridin-2-yl)methyl)amino)-2-methyl-1-oxoprop-2-yl)carbamate 11C (800 mg, yield: 39.6%, pale yellow oily substance).

MS (ESI): m/z = 394.2 [M + H]⁺

### Step 4: tert-butyl (2-(8-((cyclobutylmethyl)sulfanyl)imidazo[1,5-a]pyridin-3-yl)propan-2-yl)carbamate (11D)

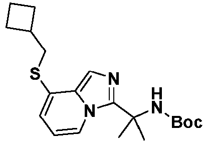

*tert-*Butyl (1-(((3-((cyclobutylmethyl)sulfanyl)pyridin-2-yl)methyl)amino)-2-methyl-1-oxoprop-2-yl)carbamate 11C (800 mg, 2.04 mmol) was dissolved in dichloromethane (20 mL), Burgess reagent (971 mg, 4.07 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and dried in vacuo to obtain the crude product. The crude *tert-*butyl (2-(8-((cyclobutylmethyl)sulfanyl)imidazo[1,5-a]pyridin-3-yl)propan-2-yl)carbamate 11D was separated and purified by column silica gel chromatography (petroleum ether/ethyl acetate = 5/1) to obtain *tert*-butyl (2-(8-((cyclobutylmethyl)thio)imidazo[1,5-a]pyridin-3-yl)prop-2-yl)carbamate 11D (290 mg, yield: 38%, pale yellow oily substance).

MS (ESI): m/z = 376.2 [M + H]⁺

### Step 5: 2-(8-((cyclobutylmethyl)sulfanyl)imidazo[1,5-a]pyridin-3-yl)propan-2-amine hydrochloride (11E)

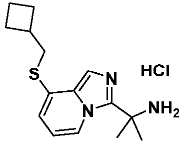

*tert-*Butyl (2-(8-((cyclobutylmethyl)sulfanyl)imidazo[1,5-a]pyridin-3-yl)propan-2-yl)carbamate 11D (290 mg, 0.773 mmol) was dissolved in a hydrochloric acid-methanol solution (10 mL) and reacted at room temperature overnight. The reaction solution was spun dry in vacuo to obtain 2-(8-((cyclobutylmethyl)sulfanyl)imidazo[1,5-a]pyridin-3-yl)propan-2-amine hydrochloride 11E (215 mg, crude product), which was directly used in the next step.

MS (ESI): m/z = 276.1 [M + H]⁺

### Step 6: tert-butyl (1R,5S,6R)-6-((2-(8-((cyclobutylmethyl)thio)imidazo[1,5-a]pyridin-3-yl)propan-2-yl)carbamoyl)-3-azabicyclo[3.1.0]hexane-3-carboxylate (11F)

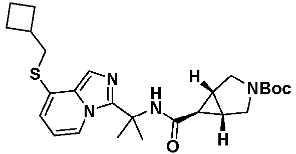

(1*R*,5*S*,6*R*)-3-(*tert*-Butoxycarbonyl)-3-azabicyclo[3.1.0]hexane-6-carboxylic acid 1F (213 mg, 0.938 mmol) and HATU (357 mg, 0.938 mmol) were dissolved in *N*,*N-*dimethylformamide (10 mL) and reacted at room temperature for half an hour. 2-(8-((Cyclobutylmethyl)sulfanyl)imidazo[1,5-a]pyridin-3-yl)propan-2-amine 11E (215 mg, 0.782 mmol) and DIEA (0.5 mL) were then added and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain crude *tert*-butyl (1*R*,5*S*,6*R*)-6-((2-(8-((cyclobutylmethyl)sulfanyl)imidazo[1,5-a]pyridin-3-yl)propan-2-yl)carbamoyl)-3-azabicyclo [3.1.0]hexane-3-carboxylate 11F. The crude product was separated and purified by silica gel column chromatography (dichloromethane/methanol= 15/1) to obtain *tert-*butyl (1*R*,5*S*,6*R*)-6-((2-(8-((cyclobutylmethyl)thio)imidazole [1,5-a]pyridin-3-yl)propan-2-yl)carbamoyl)-3-azabicyclo[3.1.0]hexane-3-carboxylate 11F (180 mg, yield: 47.6%, yellow oily substance).

MS (ESI): m/z = 485.1 [M + H]⁺

### Step 7: (1R,5S,6R)-N-(2-(8-((cyclobutylmethyl)thio)imidazo[1,5-a]pyridin-3-yl)propan-2-yl)-3-azabicyclo[3.1.0]hexane-6-carboxamide (Compound 11)

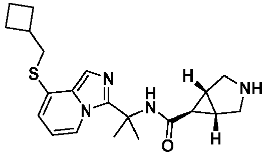

*tert*-Butyl (1*R*,5*S*,6*R*)-6-((2-(8-((cyclobutylmethyl)sulfanyl)imidazo[1,5-a]pyridin-3-yl)prop-2-yl)carbamoyl)-3-azabicyclo[3.1.0]hexane-3-carboxylate 11F (180 mg, 0.372 mmol) was dissolved in a hydrochloric acid-methanol solution (10 mL) and reacted at room temperature for 3 hours. The reaction solution was spun dry in vacuo to remove the solvent to obtain a crude product. The crude product was purified by preparative HPLC to give a white solid (Compound 11) (12 mg, yield: 83.9%).

¹H NMR (400 MHz, DMSO-d₆) δ 8.60 (s, 1H), 8.38 (s, 1H), 8.18 (d, 1H), 7.26 (s, 1H), 6.69 (d, 1H), 6.64 (t, 1H), 3.19 (d, 2H), 3.08 (d, 2H), 2.98 (d, 2H), 2.59-2.56 (m, 3H), 2.13 (m, 2H), 1.94-1.78 (m, 4H), 1.70 (s, 6H), 1.67 (m, 1H).

MS (ESI): m/z = 385.1 [M + H]⁺

### Example 12

### (1R,5S,6R)-N-(2-(8-(Cyclobutylthio)imidazo[1,5-a]pyridin-3-yl)prop-2-yl)-3-azabicyclo[3.1.0]hexane-6-carboxamide (Compound 12)

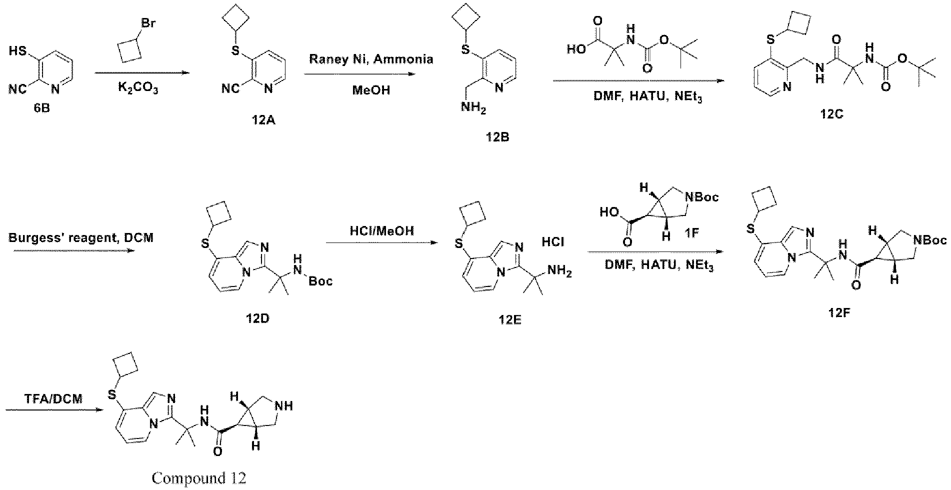

### Step 1: 3-(cyclobutylthio)pyridine-2-carbonitrile (12A)

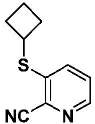

After 6B was prepared as described above, bromocyclobutane (964 mg, 7.2 mmol) and potassium carbonate (2.98 g, 21.6 mmol) were added to the reaction solution, and the mixture was stirred at room temperature overnight. The reaction solution was poured into water, the aqueous phase was extracted with ethyl acetate, and the merged organic phases were dried over anhydrous sodium sulfate, and dried in vacuo to obtain 3-(cyclobutylthio)pyridine-2-carbonitrile 12 A (crude product) (1.1 g, yield: 80.3%, yellow oily substance).

MS (ESI): m/z = 191.0 [M + H]⁺

### Step 2: (3-(cyclobutylthio)pyridin-2-yl)methanamine (12B)

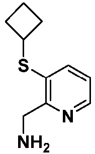

3-(Cyclobutylthio)pyridine-2-carbonitrile 12A (1.1 g, 5.7 mmol) was dissolved in methanol (30 mL), and then Raney nickel (catalytic amount) and ammonia (3 mL) were added and reacted at room temperature for 18 hours. The reaction solution was filtered by suction to obtain (3-(cyclobutylthio)pyridin-2-yl)methanamine 12B (crude product) (1.0 g, yield: 89.3%, yellow oily substance).

MS (ESI): m/z = 195.1 [M + H]⁺

### Step 3: tert-butyl (1-(((3-(cyclobutylthio)pyridin-2-yl)methyl)amino)-2-methyl-1-oxopropan-2-yl)carbamate (12C)

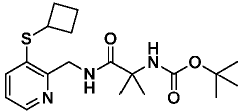

(3-(Cyclobutylthio)pyridin-2-yl)methanamine 12B (400 mg, 2.06 mmol) was dissolved in dry DMF (20 mL), then HATU (1.175 g, 3.09 mmol) was added, and the mixture was stirred at room temperature for 0.5 hours. 2-((*tert*-Butoxycarbonyl)amino)-2-methylpropionic acid (0.502 g, 2.47 mmol) and triethylamine (1.67 g, 16.5 mmol) were added, and the mixture was stirred at room temperature for 2 hours. The reaction solution was quenched by adding water, and the organic phase was extracted with ethyl acetate, dried over anhydrous sodium sulfate, and spun dry in vacuo to obtain a crude oily product. The crude product was purified through a silica gel column (petroleum ether/ethyl acetate = 1/1) to obtain *tert*-butyl (1-(((3-(cyclobutylthio)pyridin-2-yl)methyl)amino)-2-methyl-1-oxopropan-2-yl)carbamate 12C (300 mg, yield: 38.4%).

MS (ESI): m/z = 380.1 [M + H]⁺

### Step 4: tert-butyl (2-(8-(cyclobutylthio)imidazo[1,5-a]pyridin-3-yl)propan-2-yl)carbamate (12D)

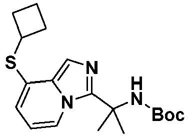

*tert-*Butyl (1-(((3-(cyclobutylthio)pyridin-2-yl)methyl)amino)-2-methyl-1-oxoprop-2-yl)carbamate 12C (300 mg, 0.792 mmol) was dissolved in dry dichloromethane (10 mL), then Burgess reagent (283.1 mg, 1.188 mmol) was added, and the mixture was stirred at room temperature overnight. The reaction solution was spun dry in vacuo to obtain a crude product. The crude product was purified through a silica gel column (petroleum ether/ethyl acetate = 3/1) to obtain *tert-*butyl (2-(8-(cyclobutylthio)imidazo[1,5-a]pyridin-3-yl)propan-2-yl)carbamate 12D (100 mg, yield: 35.1%).

MS (ESI): m/z = 362.1 [M + H]⁺

### Step 5: 2-(8-(cyclobutylthio)imidazo[1,5-a]pyridin-3-yl)propan-2-amine hydrochloride (12E)

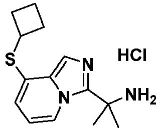

*tert*-Butyl (2-(8-(cyclobutylthio)imidazo[1,5-a]pyridin-3-yl)propan-2-yl)carbamate 12D (100 mg, 0.277 mmol) was dissolved in a solution of HCl in methanol (10 mL), and the solution was stirred at room temperature for 2 hours. The solvent was then spun dry to obtain crude 2-(8-(cyclobutylthio)imidazo[1,5-a]pyridin-3-yl)propan-2-amine hydrochloride 12E (90 mg), which was directly cast to the next step without purification.

MS (ESI): m/z = 262.1 [M + H]⁺

### Step 6: tert-butyl (1R,5S,6R)-6-((2-(8-(cyclobutylthio)imidazo[1,5-a]pyridin-3-yl)prop-2-yl)aminomethane acyl)-3-azabicyclo[3.1.0]hexane-3-carboxylate (12F)

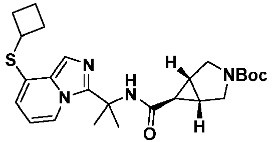

2-(8-(Cyclobutylthio)imidazo[1,5-a]pyridin-3-yl)propan-2-amine 12E (90 mg, 0.344 mmol) was dissolved in dry DMF (5 mL), then HATU (197 mg, 0.518 mmol) was added, and the mixture was stirred at room temperature for 0.5 h. (1*R*,5*S*,6*R*)-3-(*tert*-Butoxycarbonyl)-3-azabicyclo[3.1.0]hexane-6-carboxylic acid 1F (95 mg, 0.416 mmol) and triethylamine (278 mg, 2.75 mmol) were then added and the mixture was stirred at room temperature for 2 hours. The reaction solution was quenched by adding water, and extracted with ethyl acetate. The organic phase was dried over anhydrous sodium sulfate and spun dry in vacuo to obtain a crude oily product. The crude product was purified through a silica gel column (petroleum ether/ethyl acetate = 2/1) to obtain *tert*-butyl (1*R*,5*S*,6*R*)-6-((2-(8-(cyclobutylthio)imidazo[1, 5-a]Pyridin-3-yl)prop-2-yl)carbamoyl)-3-azabicyclo[3.1.0]hexane-3-carboxylate 12F (65 mg, yield:40.1%).

MS (ESI): m/z = 471.1 [M + H]⁺

### Step 7: (1R,5S,6R)-N-(2-(8-(cyclobutylthio)imidazo[1,5-a]pyridin-3-yl)propan-2-yl)-3-azabicyclo[3.1.0]hexane-6-carboxamide (Compound 12)

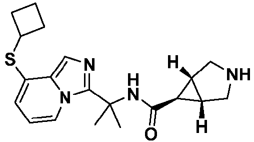

*tert-*Butyl (1*R*,5*S*,6*R*)-6-((2-(8-(cyclobutylthio)imidazo[1,5-a]pyridin-3-yl)prop-2-yl)carbamoyl)-3-azabicyclo[3.1.0]hexane-3-carboxylate 12F (65 mg, 0.138 mmol) was dissolved in dichloromethane (2 mL) and trifluoroacetic acid (0.5 mL) and stirred at room temperature for 3 hours. The reaction solution was spun dry to remove the solvent to obtain a crude product. The crude product was purified by preparative HPLC to give (1*R*,5*S*,6*R*)-*N*-(2-(8-(cyclobutylthio)imidazo[1,5-a]pyridin-3-yl)propane-2-yl)-3-azabicyclo[3.1.0]hexane-6-carboxamide (Compound 12) (11.7 mg, yield: 22.9%).

¹H NMR (400 MHz, CD₃OD) δ 8.13 (d, 1H), 7.29 (s, 1H), 6.60 (t, 1H), 6.56 (d, 1H), 4.12-4.05 (m, 1H), 3.02 (d, 2H), 2.85 (d, 2H), 2.59-2.51 (m, 2H), 2.13-2.01 (m, 4H), 1.77 (s, 6H), 1.70 (m, 2H), 1.48 (t, 1H).

MS (ESI): m/z = 371.0 [M + H]⁺

### Example 13

### (1R,5S,6R)-N-(2-(8-((Cyclopentylmethyl)thio)imidazo[1,5-a]pyridin-3-yl)propan-2-yl)-3-azabicyclo[3.1.0]hexane-6-carboxamide (Compound 13)

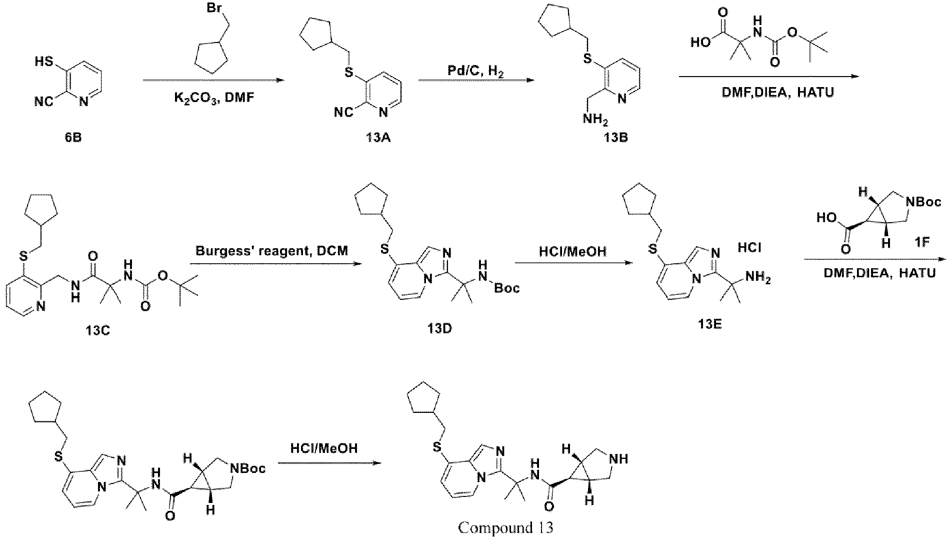

### Step 1: 3-((cyclopentylmethyl)sulfanyl)pyridine-2-carbonitrile (13A)

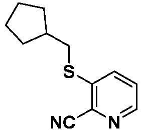

After 6B was prepared as described above, cyclopentyl bromide (1 g, 6.13 mmol) and potassium carbonate (2.5 g, 18.1 mmol) were added to the reaction solution, and the mixture was stirred at room temperature overnight. The reaction solution was poured into water, the aqueous phase was extracted with ethyl acetate, the merged organic phases were dried over anhydrous sodium sulfate, and dried in vacuo to obtain 3-((cyclopentylmethyl)sulfanyl)pyridine-2-carbonitrile 13 A (crude product). The crude product was separated and purified by silica gel column chromatography (petroleum ether/ethyl acetate = 3/1) to obtain 3-((cyclopentylmethyl)thio)pyridine-2-carbonitrile 13A (850 mg, yield: 56%, yellow oily substance).

MS (ESI): m/z = 219.1 [M + H]⁺

### Step 2: (3-((cyclopentylmethyl)sulfanyl)pyridin-2-yl)methanamine (13B)

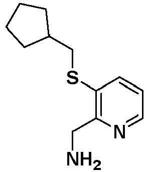

3-((Cyclopentylmethyl)sulfanyl)pyridine-2-carbonitrile 13A (850 mg, 5.5 mmol) was dissolved in methanol (30 mL), hydrochloric acid (2 mL) was added, followed by 10% palladium/charcoal (catalyst amount), and reacted for three days under hydrogen atmosphere at room temperature (during which the palladium/charcoal needed to be replaced with fresh ones). After suction filtration of the reaction solution, (3-((cyclopentylmethyl)sulfanyl)pyridin-2-yl)methanamine 13B (800 mg, crude product) was obtained.

MS (ESI): m/z = 223.1 [M + H]⁺

### Step 3: tert-butyl (1-(((3-((cyclopentylmethyl)sulfanyl)pyridin-2-yl)methyl)amino)-2-methyl-1-oxopropan-2-yl)carbamate (13C)

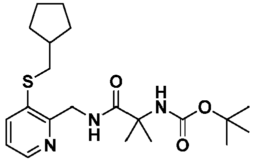

2-((*tert*-Butoxycarbonyl)amino)-2-methylpropionic acid (900 mg, 3.96 mmol) and HATU (1.5 g, 3.96 mmol) were dissolved in *N,N*-dimethylformamide (15 mL)) and reacted at room temperature for half an hour. (3-((Cyclopentylmethyl)sulfanyl)pyridin-2-yl)methanamine 13B (800 mg, 3.6 mmol) and DIEA (1.4 g, 10.9 mmol) were then added and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product 13C. The crude product was separated and purified by silica gel column chromatography (petroleum ether/ethyl acetate = 3/1) to obtain *tert*-butyl (1-(((3-((cyclopentylmethyl)sulfanyl)pyridin-2-yl)methyl)amino)-2-methyl-1-oxopropan-2-yl)carbamate 13C (500 mg, yield: 34.2%, pale yellow oily substance).

MS (ESI): m/z = 408.1 [M + H]⁺

### Step 4: tert-butyl (2-(8-((cyclopentylmethyl)sulfanyl)imidazo[1,5-a]pyridin-3-yl)propan-2-yl)carbamate (13D)

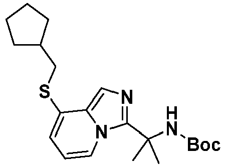

*tert-*Butyl (1-(((3-((cyclopentylmethyl)sulfanyl)pyridin-2-yl)methyl)amino)-2-methyl-1-oxoprop-2-yl)carbamate 13C (500 mg, 1.22 mmol) was dissolved in dichloromethane (10 mL), Burgess reagent (732 mg, 3.07 mmol) was added, and the mixture was stirred at room temperature overnight. The reaction solution was poured into water, the aqueous phase was extracted with dichloromethane, and the merged organic phases were washed with saturated brine, dried over anhydrous sodium sulfate, and dried in vacuo to obtain a crude product 13D. The crude product was separated and purified by silica gel column chromatography (petroleum ether/ethyl acetate = 3/1) to obtain tert-butyl (2-(8-((cyclopentylmethyl)sulfanyl)imidazo[1,5-a]pyridine-3-yl)propan-2-yl)carbamate 13D (260 mg, yield: 54%, pale yellow oily substance).

MS (ESI): m/z = 390.1 [M + H]⁺

### Step 5: 2-(8-((cyclopentylmethyl)sulfanyl)imidazo[1,5-a]pyridin-3-yl)propan-2-amine hydrochloride (13E)

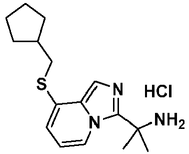

*tert-*Butyl (2-(8-((cyclopentylmethyl)sulfanyl)imidazo[1,5-a]pyridin-3-yl)propan-2-yl)carbamate 13D (260 mg, 0.668 mmol) was dissolved in a hydrochloric acid-methanol solution (10 mL) and reacted at room temperature overnight. The reaction solution was spun dry in vacuo to obtain 2-(8-((cyclopentylmethyl)sulfanyl)imidazo[1,5-a]pyridin-3-yl)propan-2-amine hydrochloride 13E (200 mg, crude product), which was directly used in the next step.

MS (ESI): m/z = 290.1 [M + H]⁺

### Step 6: tert-butyl (1R,5S,6R)-6-((2-(8-((cyclopentylmethyl)thio)imidazo[1,5-a]pyridin-3-yl)propan-2-carbamoyl)-3-azabicyclo[3.1.0]hexane-3-carboxylate (13F)

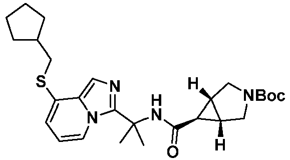

(1*R*,5*S*,6*R*)-3-(*tert*-Butoxycarbonyl)-3-azabicyclo[3.1.0]hexane-6-carboxylic acid 1F (173 mg, 0.761 mmol) and HATU (289 mg, 0.761 mmol) were dissolved in *N,N-*dimethylformamide (10 mL) and reacted at room temperature for half an hour, and then 2-(8-((cyclopentylmethyl)thio)imidazo[1,5-a]pyridin-3-yl)propan-2-amine 13E (200 mg, 0.692 mmol) and DIEA (0.5 mL) were added, and the mixture was stirred at room temperature overnight. The reaction solution was poured into water, the aqueous phase was extracted with ethyl acetate, and the merged organic phases were washed with saturated brine, dried over anhydrous sodium sulfate, and dried in vacuo to obtain a crude product 13D. The crude product was separated and purified by silica gel column chromatography (dichloromethane/methanol= 15/1) to obtain *tert-*butyl (1*R*,5*S*,6*R*)-6-((2-(8-((cyclopentylmethyl)sulfur yl)imidazo[1,5-a]pyridin-3-yl)prop-2-yl)carbamoyl)-3-azabicyclo[3.1.0]hexane-3-carboxylate 13F (200 mg, yield: 58%, yellow oily substance).

MS (ESI): m/z = 499.1 [M + H]⁺

### Step 7: (1R,5S,6R)-N-(2-(8-((cyclopentylmethyl)thio)imidazo[1,5-a]pyridin-3-yl)propane-2-yl)-3-azabicyclo[3.1.0]hexane-6-carboxamide (Compound 13)

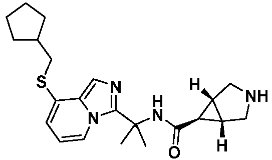

*tert-*Butyl (1*R*,5*S*,6*R*)-6-((2-(8-((cyclopentylmethyl)sulfanyl)imidazo[1,5-a]pyridin-3-yl)propan-2-yl)carbamoyl)-3-azabicyclo[3.1.0]hexane-3-carboxylate 13F (200 mg, 0.401 mmol) was dissolved in a hydrochloric acid-methanol solution (10 mL) and reacted at room temperature for 3 hours. The reaction solution was spun dry in vacuo to remove the solvent to obtain a crude product (Compound 13). The crude product was purified by preparative HPLC to give a white solid (50 mg, yield: 31.3%).

¹H NMR (400 MHz, DMSO-d₆) δ 8.37 (s, 1H), 8.12 (d, 1H), 7.21 (s, 1H), 6.64 (d, 1H), 6.58 (t, 1H), 3.05 (d, 2H), 2.87 (d, 2H), 2.69 (d, 2H), 2.17-2.09 (m, 1H), 2.03-1.97 (m, 1H), 1.84-1.78 (m, 2H), 1.64 (s, 6H), 1.56 (t, 1H), 1.53-1.48 (m, 4H), 1.35-1.28 (m, 3H).

MS (ESI): m/z = 399.2 [M + H]⁺

### Example 14

### (1R,5S,6R)-N-(2-(1-Methyl-7-(methylthio)-1H-indazol-3-yl)prop-2-yl)-3-azabicyclo[3.1.0]hexane-6-carboxamide (Compound 14)

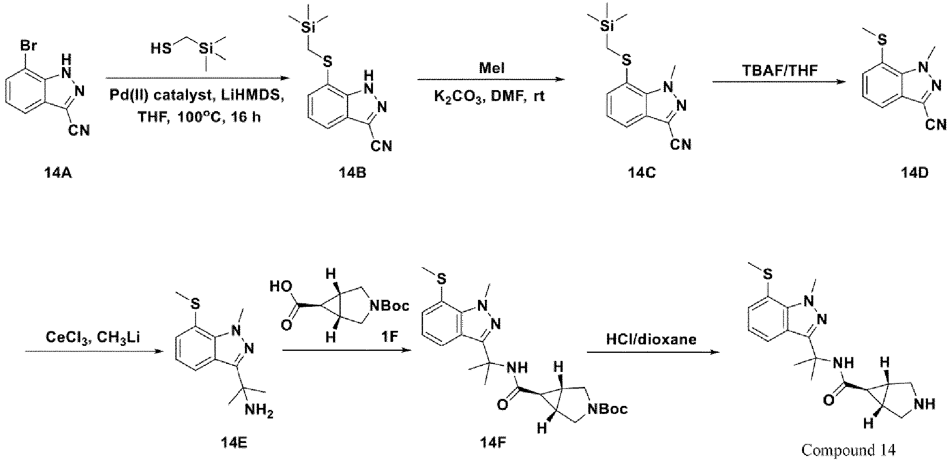

### Step 1: 7-(((trimethylsilyl)methyl)sulfanyl)-1H-indazole-3-carbonitrile (14B)

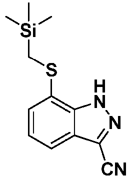

Under the protection of nitrogen, in a sealed tube, 7-bromo-1*H*-indazole-3-carbonitrile 14A (500 mg, 2.26 mmol) and a metal palladium catalyst (CAS No.: 2230788-58-4) (40 mg) were dissolved in THF (15 mL), and a 1 M/L LiHMDS/THF solution (9 mL, 9.05 mmol) was added, followed by addition of (trimethylsilyl)methanethiol (353 mg, 2.94 mmol) and reacted at 100°C overnight. Upon completion of the reaction, the reaction solution was quenched by adding water (500 mL), and then extracted with ethyl acetate (30 mL x 3). The organic phase was washed once with 50 mL of a saturated aqueous NaCl solution and dried over anhydrous sodium sulfate. The organic phase was filtered by suction and spun dry. The resulting residue was purified using a a silica gel column (petroleum ether/ethyl acetate = 1/1) to obtain a pale yellow solid 7-(((trimethylsilyl)methyl)thio)-1*H*-indazole-3-carbonitrile 14B (396 mg, yield: 67.17%).

MS (ESI): m/z = 262.1 [M + H]⁺

### Step 2: 1-methyl-7-(((trimethylsilyl)methyl)sulfanyl)-1H-indazole-3-carbonitrile (14C)

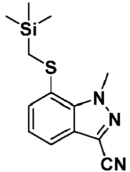

7-(((Trimethylsilyl)methyl)sulfanyl)-1*H*-indazole-3-carbonitrile 14B (1 g, 3.83 mmol) and K₂CO₃ (1.58 g, 11.49 mmol) were dissolved in DMF (20 mL), and then iodomethane (0.95 g, 6.88 mmol) was added and reacted for 1 h. Upon completion of the reaction, water (50 mL) was added for quenching, and then the reaction solution was extracted with ethyl acetate (30 mL × 3). The organic phase was washed once with 50 mL of a saturated aqueous NaCl solution and dried over anhydrous sodium sulfate. The organic phase was spun dry, and resulting residue was purified using a silica gel column (petroleum ether/ethyl acetate = 2/1) to obtain an off-white solid, which was 1-methyl-7-(((trimethylsilyl)methyl))thio)-1*H*-indazole-3-carbonitrile 14C (0.65 g, yield: 61.7%).

MS (ESI): m/z = 276.1 [M + H]⁺

### Step 3: 1-methyl-7-(methylthio)-1H-indazole-3-carbonitrile (14D)

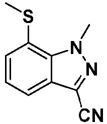

To 1-methyl-7-(((trimethylsilyl)methyl)sulfanyl)-1*H*-indazole-3-carbonitrile 14C (0.65 g, 2.36 mmol) was added a 1 M/L TBAF/THF solution (50 mL) and reacted for 2H. Upon completion of the reaction, water (100 mL) was added for quenching, and the reaction solution was extracted with ethyl acetate (50 mL × 3). The organic phase was washed once with 150 mL of a saturated aqueous NaCl solution and dried over anhydrous sodium sulfate. The organic phase was filtered by suction and spun dry. The resulting residue was purified using a silica gel column (petroleum ether/ethyl acetate = 1/1) to obtain a faint yellow solid 1-methyl-7-(methylthio)-1H-indazole-3-carbonitrile 14D (0.38 g, yield: 79.20%).

MS (ESI): m/z = 204.1 [M + H]⁺

### Step 4: 2-(1-methyl-7-(methylthio)-1H-indazol-3-yl)propan-2-amine (14E)

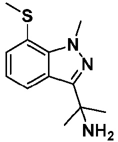

Cerium trichloride (869 mg, 3.55 mmol) was added to DMF (30 mL), and the mixture was stirred for 0.5 h and then cooled to -60°C, then a 1.3 M/L MeLi/THF solution (2.73 mL, 3.55 mmol) under the condition of a temperature not exceeding -40°C, and the mixture was stirred for 0.5 h. 1-Methyl-7-(methylthio)-1*H*-indazol-3-carbonitrile 14D was the added (180 mg, 0.89 mmol), and the solution was slowly warmed to room temperature and reacted at room temperature for 4 h. Upon completion of the reaction, a saturated aqueous NaOH solution (10 mL) was added to the reaction solution for quenching, and then water (50 mL) was added. The reaction solution was filtered and extracted with ethyl acetate (30 mL x 3). The organic phases were washed once with 100 mL of a saturated aqueous NaCl and dried over anhydrous sodium sulfate. The organic phases were filtered and spun dry, and the resulting residue was purified using a silica gel column (petroleum ether/ethyl acetate = 2/1) to obtain 2-(1-methyl-7-(methylthio)-1*H*-indazol-3-yl)propan-2-amine 14E (65 mg, yield: 31.2%).

MS (ESI): m/z = 236.1 [M + H]⁺

### Step 5: tert-butyl (1R,5S,6R)-6-((2-(1-methyl-7-(methylthio)-1H-indazol-3-yl)prop-2-yl)carbamoyl)-3-azabicyclo[3.1.0]hexane-3-carboxylate (14F)

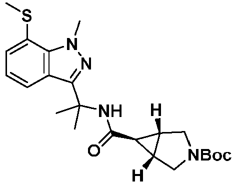

(1*R*,5*S*,6*R*)-3-(*tert*-Butoxycarbonyl)-3-azabicyclo [3.1.0]hexane-6-carboxylic acid 1F (75 mg, 0.33 mmol) and HATU (126 mg, 0.33 mmol) were dissolved in DMF (4 mL), and then 2-(1-methyl-7-(methylthio)-1*H*-indazol-3-yl)propan-2-amine 14E (65 mg, 0.28 mmol) and DIEA (107 mg, 0.99 mmol) were added and reacted for 1 h. Upon completion of the reaction, water (10 mL) was added for quenching, and then the reaction solution was extracted with ethyl acetate (5 mL × 3). The organic phases were washed once with 20 mL of a saturated aqueous NaCl and dried over anhydrous sodium sulfate. The organic phases were filtered and spun dry, and the resulting residue was purified with a preparative plate (petroleum ether:ethyl acetate = 2:1) to give a viscous gray crude solid, which was *tert*-butyl (1*R*,5*S*,6*R*)-6-((2-(1-methyl-7-(methylthio)-1*H*-indazol-3-yl)propan-2-yl)carbamoyl)-3-azabicyclo[3.1.0]hexane-3-carboxylate 14F (110 mg, yield: 88.5%).

MS (ESI): m/z = 445.1 [M + H]⁺

### Step 6: (1R,5S,6R)-N-(2-(1-methyl-7-(methylthio)-1H-indazol-3-yl)propan-2-yl)-3-azabicyclo[3.1.0]hexane-6-carboxamide (Compound 14)

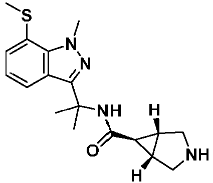

To the crude *tert*-butyl(1*R*,5*S*,6*R*)-6-((2-(1-methyl-7-(methylthio)-1*H*-indazol-3-yl)propan-2-yl)carbamoyl)-3-azabicyclo[3.1.0]-3-carboxylic acid n-hexane 14F (110 mg) was added 4 M/L HCl/dioxane (3 mL) and the mixture was stirred for 30 min. Upon completion of the reaction, the reaction solution was spun dry in vacuo, and subjected to prep-HPLC to give(1*R*,5*S*,6*R*)-*N*-(2-(1-methyl-7-(methylthio)-1H-indazole-3) was obtained by prep-HPLC-yl)propan-2-yl)-3-azabicyclo[3.1.0]hexane-6-carboxamide (Compound 14) (24.7 mg, yield: 78.6%).

¹H NMR (400 MHz, DMSO-d₆) δ 8.33 (s, 1H), 7.71 (d, 1H), 7.23 (d, 1H), 7.03 (t, 1H), 4.29 (s, 3H), 2.86 (d, 2H), 2.68 (d, 2H), 2.54 (s, 3H), 1.63 (s, 6H), 1.58 (t, 1H), 1.45 (m, 2H).

MS (ESI): m/z = 345.1 [M + H]⁺

### Example 15

### (1R,5S,6R)-N-(2-(7-(Methylthio)-1H-indazol-3-yl)propan-2-yl)-3-azabicyclo[3.1.0]hexane-6-carboxamide (Compound 15)

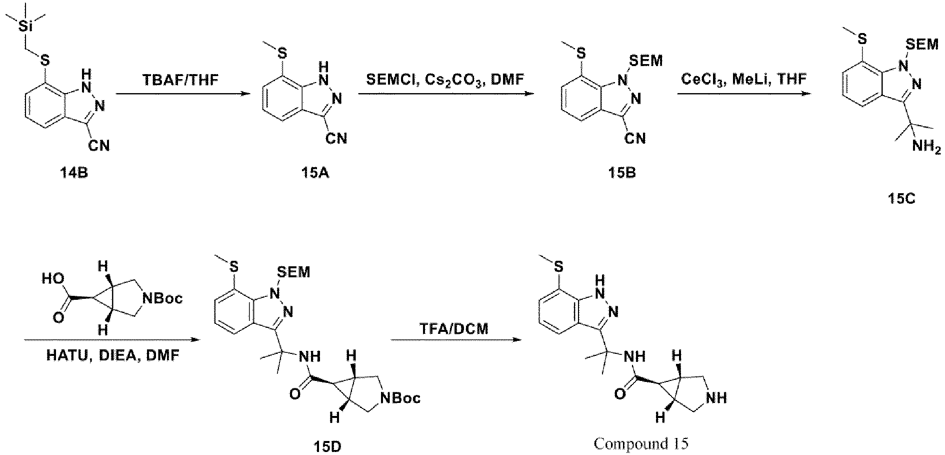

### Step 1: 7-(methylthio)-1H-indazole-3-carbonitrile (15A)

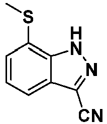

To 7-(((trimethylsilyl)methyl)sulfanyl)-1*H*-indazole-3-carbonitrile 14B (0.85 g, 2.36 mmol) was added a 1 M/L solution of TBAF/THF (10 mL) and reacted for 24 h. Upon completion of the reaction, water (20 mL) was added for quenching, and the reaction solution was extracted with ethyl acetate (20 mL × 3). The organic phases were washed once with 50 mL of a saturated aqueous NaCl solution and dried over anhydrous sodium sulfate. The organic phases were filtered by suction and spun dry, and the resulting residue was purified using a silica gel column (petroleum ether/ethyl acetate = 1/1) to obtain white 7-(methylthio)-1*H*-indazole-3-carbonitrile 15A (0.51 g, yield: 82.8%).

MS (ESI): m/z = 190.1 [M + H]⁺

### Step 2: 7-(methylthio)-1-((2-(trimethylsilyl)ethoxy)methyl)-1H-indazole-3-carbonitrile (15B)

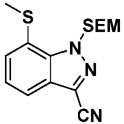

7-(Methylthio)-1H-indazole-3-carbonitrile 15A (490 mg, 2.59 mmol) and Cs2CO3 (2.54 g, 7.78 mmol) were added to DMF (10 mL) and the mixture was stirred for 0.5 h. 2-(Trisilyl)ethoxymethyl chloride (649 mg, 3.89 mmol) was then added and reacted for 1 h. Upon completion of the reaction, water (50 mL) was added for quenching, and then the reaction solution was extracted with ethyl acetate (20 mL × 3). The organic phases were washed once with 50 mL of a saturated aqueous NaCl solution and dried over anhydrous sodium sulfate. The organic phases were filtered by suction and spun dry, and the resulting residue was purified using a silica gel column (petroleum ether/ethyl acetate = 5/1) to obtain an off-white solid 7-(methylthio)-1-((2-(trimethylmethylmethane)silyl)ethoxy)methyl)-1*H*-indazole-3-carbonitrile 15B (750 mg, yield: 90.7%).

MS (ESI): m/z = 320.1 [M + H]⁺

### Step 3: 2-(7-(methylthio)-1-((2-(trimethylsilyl)ethoxy)methyl)-1H-indazol-3-yl)propan-2-amine (15C)

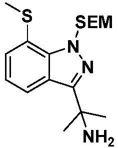

Cerium trichloride (2.30 g, 9.40 mmol) was dissolved in DMF (40 mL) and the mixture was stirred for 0.5 h and then cooled to-60°C, and then a 1.3 M/L MeLi /THF solution (7.23 mL, 9.40 mmol) was added and the mixture was stirred for 0.5 h. 7-(Methylthio)-1-((2-(trimethylsilyl)ethoxy)methyl)-1*H*-indazole-3-carbonitrile 15B (750 mg, 2.35 mmol) was then added, and the solution was slowly warmed to room temperature and reacted at room temperature for 4 h. Upon completion of the reaction, a saturated aqueous NaOH (20 mL) solution was added for quenching, followed by addition of water (50 mL). The reaction solution was filtered and extracted with ethyl acetate (20 mL × 3). The organic phase was washed once with 50 mL of a saturated aqueous NaCl solution and dried over anhydrous sodium sulfate. The organic phase was filtered by suction and spun dry, and the resulting residue was purified using a silica gel column (petroleum ether/ethyl acetate = 2/1) to obtain a white solid 2-(7-(methylthio)-1-((2-(tris)methylsilyl)ethoxy)methyl)-1*H*-indazol-3-yl)propan-2-amine 15C (260 mg, yield: 31.5%).

MS (ESI): m/z = 352.1 [M + H]⁺

### Step 4: tert-butyl (1R,5S,6R)-6-((2-(7-(methylthio)-1-((2-(trimethylsilyl)ethoxy)methyl)-1H-indazol-3-yl)propan-2-yl)carbamoyl)-3-azabicyclo[3.1.0]hexane-3-carboxylate (15D)

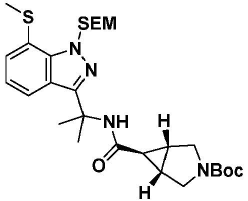

(1*R*,5*S*,6*R*)-3-(*tert*-Butoxycarbonyl)-3-azabicyclo[3.1.0]hexane-6-carboxylic acid (201 mg, 0.89 mmol) and HATU (338 mg, 0.89 mmol) were dissolved in DMF (10 mL), then 2-(7-(methylthio)-1-((2-(trimethylsilyl)ethoxy)methyl)-1*H*-indazol-3-yl)propan-2-amine 15C (260 mg, 0.74 mmol) and DIEA (287 mg, 2.22 mmol) were added and reacted for 1 h. Upon completion of the reaction, water (20 mL) was added for quenching, and then the reaction solution was extracted with ethyl acetate (15 mL × 3). The organic phases were washed once with 40 mL of a saturated aqueous NaCl solution and dried over anhydrous sodium sulfate. The organic phases were filtered by suction and spun dry, and the resulting residue was purified on a preparative plate (petroleum ether:ethyl acetate = 2:1). A crude viscous grey semi-solid was obtained, which was *tert-*butyl (1*R*,5*S*,6*R*)-6-((2-(7-(methylthio)-1-((2-(trimethylsilyl))ethoxy)methyl)-1*H*-indazol-3-yl)propan-2-yl)carbamoyl)-3-azabicyclo[3.1.0]hexane-3-carboxylate 15D (200 mg, yield: 48.2%).

MS (ESI): m/z = 561.1 [M+H]⁺

### Step 5: (1R,5S,6R)-N-(2-(7-(methylthio)-1H-indazol-3-yl)propan-2-yl)-3-aza Bicyclo[3.1.0]hexane-6-carboxamide (Compound 15)

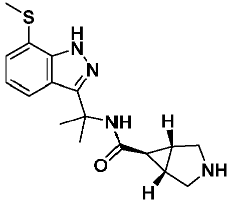

To the crude *tert*-butyl (1*R*,5*S*,6*R*)-6-((2-(7-(methylthio)-1-((2-(trimethylsilyl)ethoxy)methyl)-1*H*-Indazol-3-yl)prop-2-yl)carbamoyl)-3-azabicyclo[3.1.0]hexane-3-carboxylate 15D (200 mg) was added a TFA:DCM = 1:3 solution (10 mL) and the mixture was stirred for 30 min. Upon completion of the reaction, the reaction solution was dried in vacuo and purified by Pre-HPLC to obtain (1*R*,5*S,*6*R*)-*N*-(2-(7-(methylthio)-1*H*-indazol-3-yl)propane-2-yl)-3-azabicyclo[3.1.0]hexane-6-carboxamide (Compound 15) (20 mg, yield: 17%).

¹H NMR (400 MHz, DMSO-d₆) δ 12.74 (s, 1H), 8.30 (s, 1H), 7.70 (d, 1H), 7.22 (d, 1H), 7.02 (d, 1H), 2.86 (d, 2H), 2.68 (d, 2H), 2.54 (s, 3H), 1.66 (s, 6H), 1.59 (t, 1H), 1.46 (m, 2H).

MS (ESI): m/z = 331.1 [M + H]⁺

### Example 16

### (1R,5S,6R)-N-(2-(2-(Methylthio)phenyl)propan-2-yl)-3-azabicyclo[3.1.0]hexane-6-carboxamide (Compound 16)

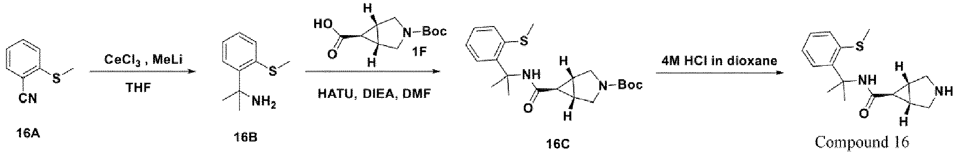

### Step 1: 2-(2-(methylthio)phenyl)propan-2-amine (16B)

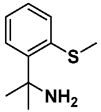

Under the protection of nitrogen, cerium trichloride (1.45 g, 5.90 mmol) was added to THF (10 mL), and the mixture was stirred and cooled to -60°C, and then methyllithium (4.54 mL, 1.3 mmol/mL) was slowly added under the condition of a temperature not exceeding -40°C, followed by stirring for 0.5 h. 2-(Methylthio)benzonitrile 16A (220 mg, 1.48 mmol) was then dissolved in THF (2 mL), and then the solution was slowly added to the reaction solution, and stirring was continued for 4 h after the addition. Upon completion of the reaction, a saturated aqueous sodium hydroxide solution (10 mL) was added for quenching, and then the reaction solution was filtered by suction. The filter cake was washed with ethyl acetate (5 mL). Water (20 mL) was then added to the filtrate and the the filtrate was extracted with ethyl acetate (20 mL × 3). The merged organic phases were washed once with 50 mL of a saturated aqueous sodium chloride solution and dried over anhydrous sodium sulfate. The organic phases were spun dry to obtain a grey solid 2-(2-(methylthio)phenyl)propan-2-amine 16B (300 mg).

MS (ESI): m/z = 182.1 [M + H]⁺

### Step 2: tert-butyl (1R,5S,6R)-6-((2-(2-(2-(methylthio)phenyl)propan-2-yl)carbamoyl)-3-azabicyclo[3.1.0]hexane-3-carboxylate (16C)

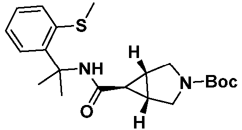

(1*R*,5*S*,6*R*)-3-(*tert*-Butoxycarbonyl)-3-azabicyclo[3.1.0]hexane-6-carboxylic acid 1F (180 mg, 0.80 mmol) and HATU (302 mg, 0.80 mmol) were dissolved in DMF (5 mL), and then 2-(2-(methylthio)phenyl)propan-2-amine 16B (120 mg, 0.66 mmol) and DIEA (256 mg, 1.99 mmol) were added and reacted for 1 h. Upon completion of the reaction, water (10 mL) was added for quenching, and then the reaction solution was extracted with ethyl acetate (5 mL × 3). The merged organic phases were washed once with 20 mL of a saturated aqueous sodium chloride solution and dried over anhydrous sodium sulfate. The organic phases were filtered and spun dry, and the resulting residue was purified using a preparative plate (petroleum ether:ethyl acetate = 2:1) to obtain a pale yellow solid, which was *tert*-butyl (1*R*,5*S*,6*R*)-6-((2-(2-(2-(methylthio)phenyl)propan-2-yl)carbamoyl)-3-azabicyclo[3.1.0]hexane-3-carboxylate 16C (60 mg, yield: 23.3%).

MS (ESI): m/z = 391.1 [M + H]⁺

### Step 3: (1R,5S,6R)-N-(2-(2-(methylthio)phenyl)propan-2-yl)-3-azabicyclo[3.1.0]hexane-6-carboxamide (Compound 16)

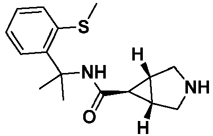

To *tert-*butyl (1*R*,5*S*,6*R*)-6-((2-(2-(2-(methylthio)phenyl)propan-2-yl)carbamoyl)-3-azabicyclo[3.1.0]hexane-3-carboxylate 16C (60 mg, 0.153 mmol) was added 4 M/L HCl/dioxane (5 mL), and the mixture was stirred for 30 min. Upon completion of the reaction, the reaction soltuion was spun dry in vacuo and purified by prep-HPLC to give (1*R*,5*S*,6*R*)-*N-*(2-(2-(methylthio)phenyl)propan-2-yl)-3-azabicyclo[3.1.0]hexane-6-carboxamide (Compound 16) (30 mg, yield: 67.2%).

¹H NMR (400 MHz, DMSO-d₆) δ 7.97 (s, 1H), 7.25 (d, 1H), 7.20 (d, 1H), 7.16 (t, 1H), 7.06 (t, 1H), 2.86 (d, 2H), 2.70 (d, 2H), 2.40 (s, 3H), 1.62 (s, 6H), 1.56-1.53 (m, 3H).

MS (ESI): m/z = 290.9 [M + H]⁺

### Example 17

### (1R,5S,6R)-N-(2-(8-Methyl-1-(methylthio)imidazo[1,5-a]pyridin-3-yl)propan-2-yl)-3-azabicyclo[3.1.0]hexane-6-carboxamide (Compound 17)

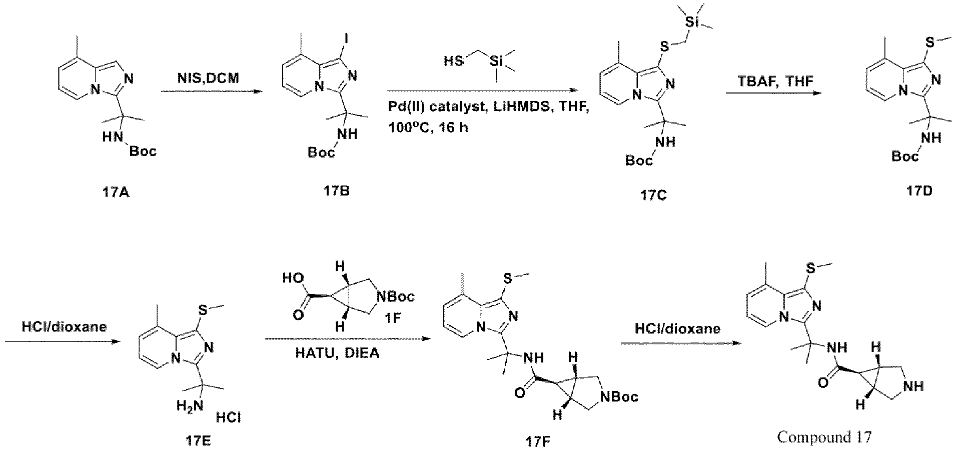

### Step 1: tert-butyl (2-(1-iodo-8-methylimidazo[1,5-a]pyridin-3-yl)propan-2-yl)carbamate (17B)

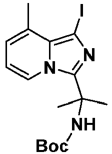

*tert*-Butyl (2-(8-methylimidazo[1,5-a]pyridin-3-yl)prop-2-yl)carbamate 17A (2.0 g, 6.9 mmol) and NIS (2.4 g, 10.7 mmol) were dissolved in dichloromethane (20 mL) and the mixture was stirred at room temperature for 2 h. LCMS monitoring showed that the reaction was complete. The reaction solution was poured into water, the aqueous phase was extracted with ethyl acetate, and the merged organic phases were dried over anhydrous sodium sulfate, and spun dry in vacuo. The sample was purified by column chromatography (petroleum ether/ethyl acetate = 2/1) to finally give *tert-*butyl (2-(1-iodo-8-methylimidazo[1,5-a]pyridine-3-yl)propan-2-yl)carbamate 17B (1.6 g, yield: 55.8%, green solid).

MS (ESI): m/z = 416.0 [M + H]⁺

### Step 2: tert-butyl (2-(8-methyl-1-(((trimethylsilyl)methyl)sulfanyl)imidazo[1,5-a]pyridin-3-yl)prop-2-yl)carbamate (17C)

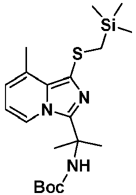

*tert*-Butyl (2-(1-iodo-8-methylimidazo[1,5-a]pyridin-3-yl)propan-2-yl)carbamate 17B (1.23 g, 3.0 mmol), trismethylsilylmethanethiol (428 mg, 3.6 mmol), and a metallic palladium catalyst (CAS: 2230788-58-4) (61 mg, 0.07 mmol) were taken to dissolve in THF, and then LiHMDS (1.0 M in THF, 7.2 mL) was added dropwise to a microwave tube. Air was expelled, and the solution was sealed in the microwave tube. The reaction temperature was 100°C, and the reaction time was 16 h. The reaction solution was then quenched with methanol and the organic phase was spun dry in vacuo. The sample was purified by column chromatography (petroleum ether/ethyl acetate = 2/1) to finally obtain *tert-*butyl (2-(8-methyl-1-(((trimethylsilyl)methyl)thio)imidazol[1,5-a]pyridin-3-yl)propan-2-yl)carbamate 17C (380 mg, yield: 31.5%, white solid).

MS (ESI): m/z = 408.0 [M + H]⁺

### Step 3: tert-butyl (2-(8-methyl-1-(methylthio)imidazo[1,5-a]pyridin-3-yl)propan-2-yl)carbamic acid (17D)

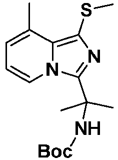

*tert*-Butyl (2-(8-methyl-1-(((trimethylsilyl)methyl)sulfanyl)imidazo[1,5-a]pyridin-3-yl)prop-2-yl)carbamate 17C (380 mg, 0.93 mmol) was dissolved in a solution of TBAF in tetrahydrofuran (20 mL), and the solution was heated to 50°C and reacted overnight. The reaction solution was dried in vacuo and purified by column chromatography (petroleum ether/ethyl acetate = 2 / 1) to obtain *tert*-butyl (2-(8-methyl-1-(methylthio)imidazo[1,5-a])pyridin-3-yl)propan-2-yl)carbamate 17D (153 mg, yield: 48.8%, white solid).

MS (ESI): m/z = 336.0 [M + H]⁺

### Step 4: 2-(8-methyl-1-(methylthio)imidazo[1,5-a]pyridin-3-yl)propan-2-amine hydrochloride (17E)

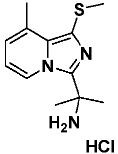

*tert*-Butyl (2-(8-methyl-1-(methylthio)imidazo[1,5-a]pyridin-3-yl)propan-2-yl)carbamate 17D (153 mg, 0.65 mmol) was dissolved in HCl/dioxane (5 mL) and reacted at room temperature for 1 h. The reaction solution was then spun dry in vacuo to obtain a crude product, which was 2-(8-methyl-1-(methylthio)imidazo[1,5-a]pyridin-3-yl)propan-2-amine hydrochloride 17E (110 mg, crude product, white solid).

MS (ESI): m/z = 236.1 [M + H]⁺

### Step 5: tert-butyl (1R,5S,6R)-6-((2-(8-methyl-1-(methylthio)imidazo[1,5-a]pyridin-3-yl)prop-2-yl)carbamoyl)-3-azabicyclo[3.1.0]hexane-3-carboxylate (17F)

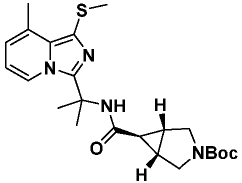

(1*R*,5*S*,6*R*)-3-(*tert*-Butoxycarbonyl)-3-azabicyclo[3.1.0]hexane-6-carboxylic acid 1F (127 mg, 0.56 mmol) and HATU (213 mg, 0.56 mmol) were dissolved in DMF (6 ml) and the mixture was stirred for 30 min. The crude 2-(8-methyl-1-(methylthio)imidazo[1,5-a]pyridin-3-yl)propan-2-amine 17E (110 mg, 0.46 mmol) obtained as above) and DIEA (181 mg, 1.4 mmol) were added to the reaction solution and the mixture was stirred for 2 hours. The reaction solution was then extracted with ethyl acetate (50 mL × 3), and the organic phases were dried over anhydrous sodium sulfate and the solvent was removed in vacuo. The organic phases were purified by column chromatography (petroleum ether/ethyl acetate = 2 / 1) to obtain *tert*-butyl (1*R*,5*S*,6*R*)-6-((2-(8-methyl-1-(methylthio)imidazolium [1,5-a]pyridin-3-yl)propan-2-yl)carbamoyl)-3-azabicyclo[3.1.0]hexane-3-carboxylate 17F (120 mg, yield: 57.8%, white solid).

MS (ESI): m/z = 445.1 [M + H]⁺

### Step 6: (1R,5S,6R)-N-(2-(8-methyl-1-(methylthio)imidazo[1,5-a]pyridin-3-yl)propane-2-yl)-3-azabicyclo[3,1,0]hexane-6-carboxamide (Compound 17)

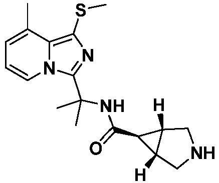

*tert-*Butyl (1*R*,5*S*,6*R*)-6-((2-(8-methyl-1-(methylthio)imidazo[1,5-a]pyridin-3-yl)propan-2-(yl)carbamoyl)-3-azabicyclo[3.1.0]hexane-3-carboxylate 17F (120 mg, 0.27 mmol) was dissolved in HCl/dioxane (6 mL) and reacted at room temperature for 1 h. The reaction solution was then spun dry in vacuo, and the crude product was purified by preparative HPLC to give (1*R*,5*S*,6*R*)-*N*-(2-(8-methyl-1-(methylthio)imidazo[1, 5-a]pyridin-3-yl)propan-2-yl)-3-azabicyclo[3,1,0]hexane-6-carboxamide (Compound 17) (58 mg, yield: 62.4%, white solid).

¹H NMR (400 MHz, DMSO-d₆) δ 8.39 (s, 1H), 8.11 (d, 1H), 6.55-6.48 (m, 2H), 2.91 (m, 2H), 2.75-2.67 (m, 2H), 2.61 (s, 3H), 2.39 (s, 3H), 1.63 (s, 6H), 1.56-1.51 (m, 3H).

MS (ESI): m/z = 345.0 [M + H]⁺

### Test examples:

### Test Example 1. Determination of human somatostatin type IV receptor SSTR 4 agonist activity

Purpose of test: A cell-based human SSTR4 cAMP assay was used to determine the agonistic effect of the test compound on an SSTR 4 receptor.

Cell culture and reagent preparation: cell line: Flp-In-CHO-SSTR4 stable pool; complete medium: Ham's F-12 K + 10% FBS + 1 x penicillin-streptomycin (PS) + 600 µg/ml hygromycin B; cell seeding medium: Ham's F-12K + 10% FBS; assay buffer: 1 x HBSS + 20 mM HEPES + 0.1%BSA + 500 uM IBMX.

Test operation:
(1) The Flp-In-CHO-SSTR 4 stable pool cell line was cultured in a complete medium at 37°C, 5% CO₂ until a confluency of 70% to 90%.
(2) After TrypLE digestion, the cells were resuspended in the seeding medium and seeded in a 384-well cell culture plate (384 PE culture plate), with 7,000 cells per well, and were cultured overnight at 37°C, 5% CO₂.
(3) Preparation of positive control compound and test compound working solutions (8x).
(4) The cell culture plate was taken out, inverted at 200 g at room temperature for 5 s to remove the medium, then quickly filled with 15 µl of an experimental buffer to each experimental well, and centrifuged at 200 g at room temperature for 5 s.
(5) 2.5 µl of the 8x compound working solution diluted in step 3 was added to corresponding test wells, which were centrifuged at 200 g for 5 s at room temperature, and incubated at 37°C for 10 min.
(6) A 4 uM Forskolin working solution (8x) was prepared.
(7) The cell plate was taken out and equilibrated to room temperature, then filled with 2.5 µl of the 8x Forskolin working solution prepared in step 6 to corresponding test wells, followed by RT at 200 g for 5 s, and left to stand at 37°C for 30 min.
(8) A Eu-cAMP tracer and Uliaght-anti-cAMP were freeze-thawed, the Eu-cAMP tracer was diluted 50-fold with a detection buffer, and the Uliaght-anti-cAMP was dilutd 150-fold.
(9) 10 µl of the Eu-cAMP tracer was added to all experimental wells, then 10 µl of the detection buffer was added to NC wells, and 10 µl of the Uliaght-anti-cAMP was added to the remaining experimental wells.
(10) The reaction plate was centrifuged at 200 g for 30 s at room temperature, and after standing at 25°C for 1 h, data was collected using Envision.

Data analysis $Z ′ factor = 1 - 3 * \left(SDMax+SDMin\right) / \left(AverageMax-AverageMin\right)$ $CVMax = \left(SDMax/AverageMax\right) * 100 %$ $CVMin = \left(SDMin/Average Min\right) * 100 %$ $S / B = signal / background$
Vehicle control (Min): assay buffer
Positive control (Max): 1,000 nM Somatostatin 14
Compound EC50 was calculated using a GraphPad nonlinear fitting formula: $Y = Bottom + \left(Top-Bottom\right) / \left(1+{10}^{∧}\left(\left(LogEC50-X\right)*HillSlope\right)\right)$
X: Compound concentration log value; Y: HTRF ratio

**Table 1. Results of assay of human somatostatin type IV receptor SSTR 4 agonist activity**

| Compound number | SSTR4, EC₅₀ (nM) |
|---|---|
| Positive control compound | 0.51 |
| Compound 1 | 0.15 |
| Compound 2 | 6.77 |
| Compound 3 | 10.54 |
| Compound 4 | 36.73 |
| Compound 5-P1 | 1.25 |
| Compound 5-P2 | 0.41 |
| Compound 6 | 0.73 |
| Compound 7 | 3.29 |
| Compound 8 | 0.50 |
| Compound 9 | 3.42 |
| Compound 10 | 7.89 |
| Compound 11 | 2.47 |
| Compound 12 | 1.83 |
| Compound 13 | 7.58 |
| Compound 14 | 0.057 |
| Compound 15 | 0.046 |
| Compound 16 | 1.25 |
| Compound 17 | 0.068 |

Conclusion: The compounds of the present invention have significant agonistic activity on human growth hormone type IV receptor SSTR4.

### Test Example 2. Metabolic stability of liver microsomes

Metabolic degradation of a test compound was analyzed using pooled human liver microsomes and male rat liver microsomes, respectively, at 37 °C.

A final incubation reaction system solution contained a phosphate buffer (pH 7.4), a positive control compound (dextromethorphan) or test compound (200 µM, 1.5 µL), and liver microsomes (0.5 mg/mL, 238.5 µL). After pre-incubation at 37°C for 5 min, NADPH (5 mM, 60 µL) was added to initiate a reaction. A fixed volume of the reaction mixture (30 µL) was sampled into the solution at fixed time points (0, 5, 15, 30, 60 min) to quench the reaction. After centrifugation (4000 rpm, 15 min), the supernatant (100 µL) was mixed with distilled water (100 µL) and then subjected to LC-MS/MS analysis to test the amount of the compound. A half-life was calculated using a first-order reaction kinetic equation (Cₜ = C₀ × e^{-ket}, T_{1/2} = Ln2/ke).

**Table 2. Analysis and test results of human liver microsomes and male rat liver microsomes**

| Compound number | Human | Rat |
|---|---|---|
| | T_{1/2} (min) | T_{1/2} (min) |
| Compound 1 | > 186.4 | > 186.4 |
| Compound 14 | > 186.4 | > 186.4 |
| Compound 15 | > 186.4 | > 186.4 |
| Compound 17 | > 186.4 | > 186.4 |

Conclusion: The compounds of the present invention have good metabolic stability in liver microsomes.

### Test Example 3. Pharmacokinetic evaluation

Using rats as test animals, an LC/MS/MS method was used to determine the drug concentrations in plasma at different times after oral and intravenous administration of Compound 1a and Compound 14 in the rats. The pharmacokinetic behavior of the compounds of the present invention in rats was studied, and pharmacokinetic characteristics thereof were evaluated.

### Test drug:

Compound 1a and Compound 14.

### Test animals:

For each compound, 6 healthy adult SD (Sprague-Dawley) rats, male, were divided into oral and intravenous administration groups, 3 rats in each group, purchased from Shanghai SIPPR/BK Laboratory Animal Co., Ltd., animal production license number: SCXK (Shanghai) 2008-0016.

### Drug preparation:

A specific amount of the drug was weighed and dissolved in 5% dimethylacetamide (DMA) + 5% polyethylene glycol-15 hydroxystearate (solutol) + 90% saline solution to prepare a 0.2 mg/mL solution.

### Dosing:

The SD rats were fasted overnight and were administered orally and intravenously. The oral dose was 10.0 mg/kg, and the intravenous dose was 1.0 mg/kg.

### Test operation:

Compound 1a and Compound 14 were administered orally and intravenously to the rats. At 0.083, 0.25, 0.5, 1, 2, 4, 8, and 24 hours after administration, 0.2 mL of blood was collected from the submandibular vein or other suitable blood vessels, placed in K2-EDTA tubes, and then stored on ice. Within one hour, the plasma was separated by centrifugation at 6,800 g for 6 minutes at 2-8°C, and stored at -80°C for LC/MS/MS analysis. The rats were fed 4 hours after administration.

**Table 3. Pharmacokinetic parameters in rats**

| Compound number | Pharmacokinetic parameters | | | | | |
|---|---|---|---|---|---|---|
| | Blood concentration | Curve area | Half-life | Residence time | Clearance rate | Apparent volume of distribution |
| | Cmax (ng/mL) | AUC₍₀₋ₜ₎ (h*ng/mL) | T_{1/2} (h) | MRT ₍₀₋ₜ₎ (h) | CL (mL/min/kg) | Vz (mL/kg) |
| Compound 1a | 368.96 ± 82.98 | 1246.88 ± 431.50 | 2.58 ± 1.07 | 2.92 ± 0.39 | 53.02 ± 1.76 | 5122.23 ± 1138.86 |
| Compound 14 | 875.49 ± 114.52 | 2890.26 ± 78.06 | 2.58 ± 0.50 | 2.51 ± 0.17 | 48.66 ± 5.01 | 7091.73 ± 1083.29 |

Conclusion: The compounds of the present invention are well absorbed in pharmacokinetics.

### Test Example 4, efficacy evaluation-1

### Experimental design

Each male SD rat (7-8 weeks old) was injected with 50 microliters of CFA in the center of the left hind paw. A PWT basal value test was conducted 24 hours after modeling, and the drugs were administered after randomization.

### Test drug

Compound 1a, Compound 14, indomethacin, and Control Compound A with the following structure (refer to patent WO 2014184275 for the synthesis method of Control Compound A)

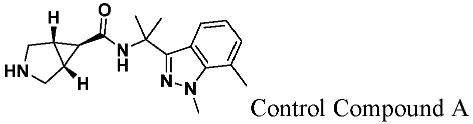

### Animal grouping

There were 8 animals in each group, and the animals were weighed and administered according to Table 4.

**Table 4 Test group and dosage table**

| Group | Model | Dosing group | Solvent | Dosage (mg/kg) | Route of administration | Test time point |
|---|---|---|---|---|---|---|
| 1 | CFA | Medium | Tween 80 + 0.5% MC (v:v = 1:99) | NA | I.P. | 1 h, 3 h |
| 2 | CFA | Indomethacin | Tween 80 + 0.5% MC (v:v = 1:99) | 30 | P.O. | 1 h, 3 h |
| 3 | CFA | Compound 1a | Tween 80 + 0.5% MC (v:v = 1:99) | 10 | I.P. | 1 h, 3 h |
| 4 | CFA | Control Compound A | Tween 80 + 0.5% MC (v:v = 1:99) | 10 | I.P. | 1 h, 3 h |
| 5 | CFA | Compound 14 | Tween 80 + 0.5% MC (v:v = 1:99) | 10 | I.P. | 1 h, 3 h |

| | | | | | | |
|---|---|---|---|---|---|---|
| Note: I.P. is intraperitoneal injection, P.O. is oral administration. | | | | | | |

### Compound efficacy testing:

For all groups of animals, paw withdrawal thresholds (PWTs) of the animals were tested using Von Frey after 1 h and 3 h of single administration.

### Experimental results

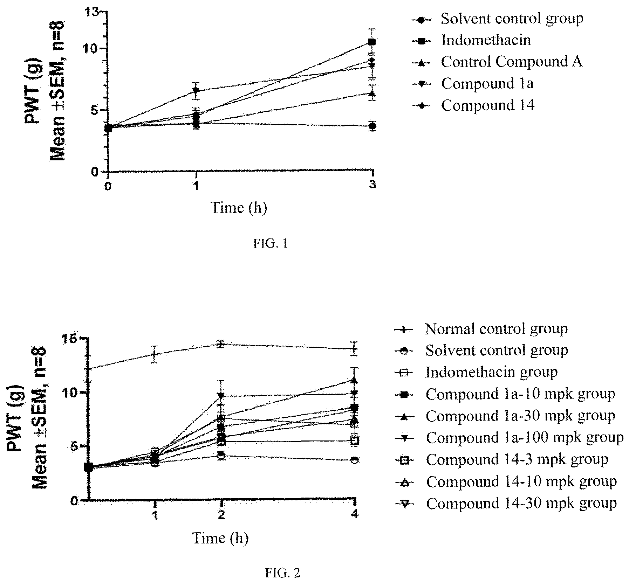

As shown in FIG. 1, 24 hours after the rats were injected with CFA, the PWT decreased to about 3.6 g, which was significantly lower than the PWTs of normal rats, indicating that the mechanical allodynia model was successfully established. The positive control compound indomethacin could significantly inhibit CFA-induced mechanical allodynia in the rats 3 hours after administration. Control Compound A, Compound 1a, and Compound 14 all inhibited CFA-induced mechanical allodynia in the rats. Furthermore, the efficacy of Compound 1a at 1 hour after administration and 3 hours after administration and Compound 14 at 3 hours after administration was significantly better than that of Control Compound A.

**Table 5 Single-dose pharmacodynamic study test results of compounds in rat CFA pain model**

| Group | Mechanical allodynia test (PWT (g), mean) | | |
|---|---|---|---|
| | Baseline | 1 hour | 3 hours |
| Solvent control group | 3.559 | 3.893 | 3.545 # |
| Indomethacin group | 3.554 | 4.448 | 10.360 **** #### |
| Control Compound A group | 3.765 | 3.813 | 6.237 * |
| Compound 1a group | 3.554 | 6.484 * # | 8.371 **** |
| Compound 14 group | 3.566 | 4.628 | 8.886 **** # |

| | | | |
|---|---|---|---|
| Note: ** p* < 0.05, ***** p <* 0.0001 as compared with the solvent control group, using Two-way ANOVA to collect statistics; *# p* < 0.05, *#### p* < 0.0001 as compared with Control Compound A group, using Two-way ANOVA to collect statistics. | | | |

### Test Example 5, efficacy evaluation-2

### Multi-dose oral (PO) pharmacodynamics study of Compound 1a and Compound 14 in rat CFA model

### 1. Summary

The efficacy of Compound 1a and Compound 14 in the rat CFA pain model was evaluated using a mechanical allodynia method.

### 2. Test scheme

### 2.1 Test drug

### Indomethacin, Compound 1a, and Compound 14

### 2.2 Experimental animals

SD rats, 72 males, divided into 9 groups, were purchased from Beijing Vital River Laboratory Animal Technology Co., Ltd.

### 2.3 Test method

The experimental animals were adaptively reared for 3 to 7 days, and 8 of them were selected as a normal control group, and the other animals were injected with 50 µL of CFA in the center of the left hind paw for modeling. 24 hours after injection, the animals were tested for paw withdrawal threshold (PWT) basal values, and were divided into groups according to the basal values, which were solvent control group, indomethacin group, Compound 1a, 10 mpk group, Compound 1a, 30 mpk group, Compound 1a, 100 mpk group, Compound 14, 3 mpk group, Compound 14, 10 mpk group, and Compound 14, 30 mpk group, with 8 animals in each group. Subsequently, the animals were weighed and administered orally according to Table 6. All the animals were tested for paw withdrawal thresholds (PWT) using Von Frey 1, 2, and 4 hours after administration to further evaluate the efficacy of the compounds of the present invention.

**Table 6 Test group and dosage table**

| Group No. | Group | Number of animals | Dosage (mg/kg) | Route of administration (5 mL/kg) | Test time point |
|---|---|---|---|---|---|
| 1 | Normal control group | 8 | - | - | 1 h, 2 h, 4 h |
| 2 | Solvent control group | 8 | - | Oral | 1 h, 2 h, 4 h |
| 3 | Indomethacin group | 8 | 30 | Oral | 1 h, 2 h, 4 h |
| 4 | Compound 1a-10 mpk group | 8 | 10 | Oral | 1 h, 2 h, 4 h |
| 5 | Compound 1a-30 mpk group | 8 | 30 | Oral | 1 h, 2 h, 4 h |
| 6 | Compound 1a-100 mpk group | 8 | 100 | Oral | 1 h, 2 h, 4 h |
| 7 | Compound 14-3 mpk group | 8 | 3 | Oral | 1 h, 2 h, 4 h |
| 8 | Compound 14-10 mpk group | 8 | 10 | Oral | 1 h, 2 h, 4 h |
| 9 | Compound 14-30 mpk group | 8 | 30 | Oral | 1 h, 2 h, 4 h |

### 2.4 Results:

As shown in FIG. 2, 24 hours after the rats were injected with CFA, PWT was significantly down-regulated compared with the normal control group, indicating that the mechanical allodynia model was successfully established. The positive control compound indomethacin significantly inhibited CFA-induced mechanical allodynia in the rats 2 and 4 hours after administration. Compound 1a could dose-dependently inhibit CFA-induced mechanical hyperalgesia in the rats 2 and 4 hours after administration, with an onset dose of 10 mg/kg, and the efficacy in the 30 mpk and 100 mpk groups 4 hours after administration was significantly better than that of the positive control compound indomethacin. Compound 14 could dose-dependently inhibit CFA-induced mechanical hyperalgesia in the rats 4 hours after administration, with an onset dose of 10 mg/kg.

**Table 7 Test results of multi-dose pharmacodynamic studies of Compounds 1a and 14 in rat CFA pain model**

| Group | Paw withdrawal threshold (PWT(g)) (average) | | | |
|---|---|---|---|---|
| | 0 hours | 1 hour | 2 hours | 4 hours |
| Normal control group | 12.124 **** #### | 13.440 **** #### | 14.331 **** #### | 13.808 **** #### |
| Solvent control group | 2.969 | 3.436 | 4.072 ## | 3.545 ## |
| Indomethacin group | 3.012 | 4.461 | 7.486 ** | 6.813 ** |
| Compound 1a-10 mpk group | 3.016 | 4.020 | 6.722 * | 8.438 **** |
| Compound 1a-30 mpk group | 3.118 | 4.173 | 7.637 ** | 10.958 **** ### |
| Compound 1a-100 mpk group | 3.118 | 3.855 | 9.549 **** | 9.687 **** # |
| Compound 14-3 mpk group | 3.118 | 3.535 | 5.369 | 5.335 |
| Compound 14-10 mpk group | 3.118 | 4.155 | 5.789 | 7.308 ** |
| Compound 14-30 mpk group | 3.079 | 4.051 | 5.683 | 8.213 **** |

| | | | | |
|---|---|---|---|---|
| Note: *p< 0.05, **p< 0.01, ****p< 0.0001 as compared with the solvent control group, using Two-way ANOVA to collect statistics; #p< 0.05, ##p< 0.01, ###p< 0.001, ####p< 0.0001 as compared with the indomethacin group, using Two-way ANOVA to collect statistics. | | | | |

### Test Example 6, selective test

### 1. Summary

Selective activity of the compounds on human SSTR4 was assessed using a radioligand binding assay.

### 2. Test operation

At 25°C, different concentrations of the compounds gradiently diluted were incubated together with a radioligand [¹²⁵I]Somatostatin-14 in CHO-K1 cell membrane homogenates expressing human SSTR1, 2, 3, 4, and 5, respectively, for 2 hours. Cell membranes were then filtered and washed, and protein-bound radioactivity was measured with a suitable instrument. The Ki values of corresponding targets were calculated using a Cheng-Prusoff formula.

### 3. Test results

The binding selectivity of different compounds to SSTRs 1-5 were shown in Table 1. The results showed that the SSTR4 selectivity of Compound 1a was the best, which was better than that of Control Compound A.

**Table 8 In vitro human SSTR selectivity of compounds**

| Target | Binding Ki (nM) | | | |
|---|---|---|---|---|
| | Compound 1a | Control Compound A | Compound 14 | Somatostatin-14 |
| SSTR1 | > 8276 | 1804 | 799 | 0.26 |
| SSTR2 | > 5313 | > 5313 | > 5313 | 0.01 |
| SSTR3 | > 3421 | > 3421 | > 3421 | 0.06 |
| SSTR4 | 5.52 | 1.41 | 0.51 | 0.51 |
| SSTR5 | > 5652 | > 5652 | > 5652 | 0.36 |
| SSTR4 /SSTR 1 | > 1500 | 1276.8 | 879.5 | 0.5 |

The technical features of the examples described above can be combined arbitrarily. For the sake of brevity, all possible combinations of the technical features in the examples described above are not described. However, provided that there is no contradiction between the combinations of these technical features, all should be regarded as being within the scope encompassed by this description.

The above examples only represent several examples of the present invention, and the description thereof is specific and detailed, but should not be construed as a limitation to the scope of the present invention. It should be noted that for those of ordinary skill in the art, several modifications and improvements can further be made without departing from the concept of the present invention, which all belong to the scope of protection of the present invention. Therefore, the scope of protection of the present invention shall be subject to the appended claims.

## Claims

1. A compound of Formula (I), or a stereoisomer, an *N*-oxide, a hydrate, a solvate, a pharmaceutically acceptable salt, or a polymorph thereof: 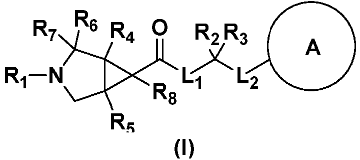
wherein R₁ is selected from -H and a C₁₋₆ alkyl;
L₁ is selected from -NH- and -O-;
R₂ and R₃ are each independently selected from -H, a C₁₋₆ alkyl, and a C₃₋₆ cycloalkyl, wherein R₂ and R₃ are not both -H; or R₂ and R₃ together form a 3- to 6-membered saturated cyclic group comprising 0 to 1 group selected from -O-, -NR₉-, -SO-, and -SO₂-;
R₄, R₅, R₆, R₇, and R₈ are each independently selected from -H, a C₁₋₆ alkyl, a C₁₋₆ alkoxy, a C₁₋₆ alkoxy C₁₋₆ alkyl, a -(CH₂)ₘ-C₃₋₁₀ carbocyclyl, a -(CH₂)ₘ-(3- to 10-membered heterocyclyl), a -(CH₂)ₘ-O-C₃₋₁₀ carbocyclyl, a -(CH₂)ₘ-O-(3- to 10-membered heterocyclyl), phenyl, and a 5- to 6-membered heteroaryl, the heterocyclyl or the heteroaryl contains 1 to 4 heteroatoms selected from N, O, and S, and the alkyl, the alkoxy, the carbocyclyl, the phenyl, the heteroaryl, or the heterocyclyl in R₄, R₅, R₆, R₇, and R₈ is each independently optionally further substituted with 0 to 4 substituents selected from -H, -F, -Cl, -Br, -I, hydroxy, sulfydryl, cyano, amino, a C₁₋₄ alkyl, and a C₁₋₄ alkoxy;
R₉ is selected from -H, a C₁₋₆ alkyl, a C₁₋₄ alkoxy C₁₋₆ alkyl, a halogen, hydroxy, cyano, and a C₃₋₆ cycloalkyl;
A is selected from a C₆₋₁₄ aryl, a 5- to 14-membered heteroaryl, a 5- to 14-membered heterocyclyl, and a 5- to 14-membered cycloalkyl, and the aryl, the heteroaryl, the heterocyclyl, and the cycloalkyl in A are optionally further substituted with 1 to 4 R₁₀, wherein the heteroaryl or the heterocyclyl contains 1 to 4 heteroatoms selected from N, O, and S;
when L₂ is selected from a single bond, -(CRₐR_{b})ₙ-, and -(CRₐR_{b})ₙO-, then R₁₀ is independently selected from -H, -F, -Cl, -Br, -I, hydroxy, cyano, amino, a C₁₋₆ alkyl, a C₁₋₄ alkoxy C₁₋₆ alkyl, a C₃₋₆ cycloalkyl, and -SR₁₁, and then at least one R₁₀ is -SR₁₁, wherein R₁₁ is each independently selected from -H, a C₁₋₆ alkyl, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a C₆₋₁₀ aryl, and a 5- to 6-membered heteroaryl, the heterocyclyl and the heteroaryl contain 1 to 4 heteroatoms selected from N, O, and S, the alkyl, and the alkoxy, the aryl, the heteroaryl, the carbocyclyl, or the heterocyclyl is each independently optionally further substituted with 0 to 4 substituents selected from -H, a halogen, hydroxy, cyano, a C₁₋₄ alkyl, and a C₁₋₄ alkoxy;
when L₂ is selected from -(CRₐR_{b})ₙS-, then R₁₀ is independently selected from -H, -F, -Cl, - Br, -I, hydroxy, cyano, amino, a C₁₋₄ alkyl, a C₁₋₄ alkoxy, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -O-(CH₂)ₙ-C₃₋₁₀ carbocyclyl or -O-(CH₂)ₙ-(3- to 10-membered heterocyclyl), and -SR₁₁, wherein R₁₁ is each independently selected from -H, a C₁₋₆ alkyl, a C₁₋₄ alkoxy C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3-to 10-membered heterocyclyl), a C₆₋₁₀ aryl, and a 5- to 6-membered heteroaryl, the heterocyclyl or the heteroaryl contains 1 to 4 heteroatoms selected from N, O, and S, and the alkyl, the alkoxy, the aryl, the heteroaryl, the carbocyclyl, or the heterocyclyl is each independently optionally further substituted with 0 to 4 substituents selected from H, a halogen, hydroxy, cyano, a C₁₋₄ alkyl, and a C₁₋₄ alkoxy;
Rₐ and R_{b} are each independently selected from -H and a C₁₋₆ alkyl;
m is independently selected from 0, 1, 2, and 3 at each occurrence;
n is independently selected from 0, 1, 2, and 3 at each occurrence.

2. The compound, or the stereoisomer, the *N*-oxide, the hydrate, the solvate, the pharmaceutically acceptable salt, or the polymorph thereof according to claim 1, wherein
L₁ is -NH-;
R₁ is -H.

3. The compound, or the stereoisomer, the *N*-oxide, the hydrate, the solvate, the pharmaceutically acceptable salt, or the polymorph thereof according to claim 1, wherein the compound has structural features of Formula (VI): 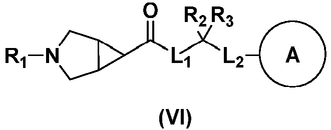
L₁ is selected from -NH- and -O-;
L₂ is selected from a single bond and -(CRₐR_{b})ₙ- or -(CRₐR_{b})ₙO-, wherein Rₐ and R_{b} are independently selected from -H and a C₁₋₆ alkyl;
n is independently selected from 0, 1, 2, and 3 at each occurrence.

4. The compound, or the stereoisomer, the *N*-oxide, the hydrate, the solvate, the pharmaceutically acceptable salt, or the polymorph thereof according to claim 1, wherein the compound has structural features of Formula (II): 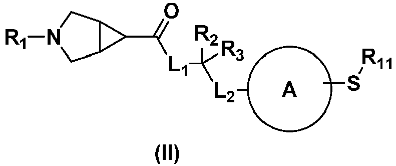
L₁ is selected from -NH- and -O-;
L₂ is selected from a single bond, -(CRₐR_{b})ₙ-, and -(CRₐR_{b})ₙO-, wherein Rₐ and R_{b} are each independently selected from -H and a C₁₋₆ alkyl;
n is independently selected from 0, 1, 2, and 3 at each occurrence; optionally wherein either:
(a) R₁ is -H; L₁ is -NH-; L₂ is selected from a single bond, -CH₂-, -CH₂CH₂-, - CH₂CH₂CH₂-, -CH₂O-, and -CH₂CH₂O-; or
(b) R₂ and R₃ are each independently selected from a C₁₋₆ alkyl and a C₃₋₆ cycloalkyl; or R₂ and R₃ together form a 3- to 6-membered saturated cyclic group comprising 0 to 1 group selected from -O-, -SO-, and -SO₂-; or
(c) R₁₁ is each independently selected from -H, a C₁₋₆ alkyl, a C₁₋₄ alkoxy C₁₋₆ alkyl, a -(CH₂)ₙ-alkenyl, a -(CH₂)ₙ-alkynyl, a -(CH₂)ₙ-C₃₋₁₀ carbocyclyl, a -(CH₂)ₙ-(3- to 10-membered heterocyclyl), a C₆₋₁₀ aryl, and a 5- to 6-membered heteroaryl, the heterocyclyl or the heteroaryl contains 1 to 4 heteroatoms selected from N, O, and S, and the alkyl, the alkoxy, the aryl, the heteroaryl, the carbocyclyl, or the heterocyclyl is independently optionally further substituted with 0 to 4 substituents selected from -H, a halogen, hydroxy, cyano, a C₁₋₄ alkyl, and a C₁₋₄ alkoxy; optionally wherein R₁₁ is each independently selected from methyl, ethyl, isopropyl, trifluoromethyl, cyclopropyl, cyclobutyl, cyclopentyl, cyclohexyl, phenyl, imidazolyl, pyrrolyl, furyl, thiophenyl, and pyridyl.

5. The compound, or the stereoisomer, the *N*-oxide, the hydrate, the solvate, the pharmaceutically acceptable salt, or the polymorph thereof according to claim 1, wherein the compound has structural features of Formula (III): 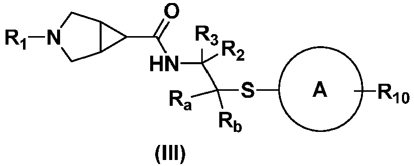
R₁₀ is each independently selected from -H, -F, -Cl, -Br, -I, hydroxy, cyano, amino, a C₁₋₄ alkyl, a C₁₋₄ alkoxy, a C₁₋₄ alkoxy C₁₋₆ alkyl, a -(CH₂)ₙ-alkenyl, a -(CH₂)ₙ-alkynyl, a -O-(CH₂)ₙ-C₃₋₁₀ carbocyclyl, a -O-(CH₂)ₙ-(3- to 10-membered heterocyclyl), a -O-(CH₂)ₙ-C₃₋₁₀ carbocyclyl, a -O-(CH₂)ₙ-(3- to 10-membered heterocyclyl), and -SR₁₁, wherein R₁₁ is each independently selected from -H, a C₁₋₆ alkyl, a C₁₋₄ alkoxy C₁₋₆ alkyl, a -(CH₂)ₙ-alkenyl, a - (CH₂)ₙ-alkynyl, a -(CH₂)ₙ-C₃₋₁₀ carbocyclyl, a -(CH₂)ₙ-(3- to 10-membered heterocyclyl), a C₆₋₁₀ aryl, and a 5- to 6-membered heteroaryl, the heterocyclyl or the heteroaryl contains 1 to 4 heteroatoms selected from N, O, and S, and the alkyl, the alkoxy, the aryl, the heteroaryl, the carbocyclyl, or the heterocyclyl is each independently optionally further substituted with 0 to 4 substituents selected from -H, a halogen, hydroxy, cyano, a C₁₋₄ alkyl, and a C₁₋₄ alkoxy;
Rₐ and R_{b} are each independently selected from -H and a C₁₋₆ alkyl;
optionally wherein R₁₀ is each independently selected from -H, -F, -Cl, -Br, -I, hydroxy, cyano, amino, methyl, ethyl, isopropyl, trifluoromethyl, cyclopropyl, cyclobutyl, cyclopentyl, cyclobutoxy, tetrahydrofuranyl, and -SR₁₁, wherein R₁₁ is each independently selected from methyl, ethyl, isopropyl, trifluoromethyl, cyclopropyl, cyclobutyl, cyclopentyl, cyclohexyl, phenyl, imidazolyl, pyrrolyl, furyl, thienyl, and pyridyl; Rₐ and R_{b} are each independently selected from -H, methyl, ethyl, and isopropyl.

6. The compound, or the stereoisomer, the N-oxide, the hydrate, the solvate, the pharmaceutically acceptable salt, or the polymorph thereof according to claim 1, wherein the compound has structural features of Formula (IV): 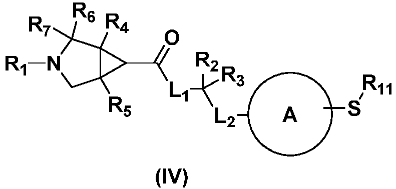 R₄ is selected from a C₁₋₆ alkyl, a C₁₋₆ alkoxy, a C₁₋₆ alkoxy C₁₋₆ alkyl, a -(CH₂)ₘ-C₃₋₁₀ carbocyclyl, a -(CH₂)ₘ-(3- to 10-membered heterocyclyl), a -(CH₂)ₘ-O-C₃₋₁₀ carbocyclyl, a - (CH₂)ₘ-O-(3- to 10-membered heterocyclyl), phenyl, and a 5- to 6-membered heteroaryl, the heterocyclyl or the heteroaryl contains 1 to 4 heteroatoms selected from N, O, and S, and the alkyl, the alkoxy, the carbocyclyl, the phenyl, the heteroaryl, or the heterocyclyl in R₄ is each independently optionally further substituted with 0 to 4 substituents selected from -H, -F, -Cl, -Br, -I, hydroxy, sulfydryl, cyano, amino, a C₁₋₄ alkyl, and a C₁₋₄ alkoxy.

7. The compound, or the stereoisomer, the *N*-oxide, the hydrate, the solvate, the pharmaceutically acceptable salt, or the polymorph thereof according to claim 1, wherein the compound has structural features of Formula (V): 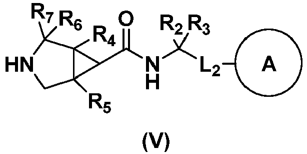
R₂ and R₃ are each independently selected from a C₁₋₆ alkyl and a C₃₋₆ cycloalkyl, wherein R₂ and R₃ are not both -H; or R₂ and R₃ together form a 3- to 6-membered saturated cycloalkyl;
R₄, R₅, R₆, and R₇ are each independently selected from -H, a C₁₋₆ alkyl, a C₁₋₆ alkoxy, and a C₁₋₆ alkoxy C₁₋₆ alkyl, and the alkyl and the alkoxy in R₄, R₅, R₆, and R₇ are each independently optionally further substituted with 0 to 4 substituents selected from -H, -F, -Cl, -Br, -I, hydroxy, sulfydryl, cyano, amino, a C₁₋₄ alkyl, and a C₁₋₄ alkoxy;
A is selected from a C₆₋₁₄ aryl, a 5- to 14-membered heteroaryl, a 5- to 14-membered heterocyclyl, and a 5- to 14-membered cycloalkyl, and the aryl, the heteroaryl, the heterocyclyl, and the cycloalkyl in A are optionally further substituted with 1 to 4 R₁₀, wherein the heteroaryl or the heterocyclyl contains 1 to 4 N atoms;
when L₂ is selected from a single bond, R₁₀ is independently selected from -H, -F, -Cl, -Br, -I, hydroxy, cyano, amino, a C₁₋₆ alkyl, a C₁₋₄ alkoxy C₁₋₆ alkyl, a C₃₋₆ cycloalkyl, and - SR₁₁, and at least one R₁₀ is -SR₁₁, wherein R₁₁ is each independently selected from -H, a C₁₋₆ alkyl, a C₁₋₄ alkoxy C₁₋₆ alkyl, a -(CH₂)ₙ-C₃₋₁₀ carbocyclyl, a -(CH₂)ₙ-(3- to 10-membered heterocyclyl), and a 5- to 6-membered heteroaryl, the heterocyclyl or the heteroaryl contains 1 to 4 heteroatoms selected from O, S, and N, and the alkyl, the alkoxy, the carbocyclyl, or the heterocyclyl is each independently optionally further substituted with 0 to 4 substituents selected from -H, a halogen, hydroxy, cyano, a C₁₋₄ alkyl, and a C₁₋₄ alkoxy;
when L₂ is selected from -(CRₐR_{b})ₙS-, R₁₀ is each independently selected from -H, -F, -Cl, -Br, -I, hydroxy, cyano, amino, a C₁₋₄ alkyl, a C₁₋₄ alkoxy, and a C₁₋₄ alkoxy C₁₋₆ alkyl, the alkyl and the alkoxy are each independently optionally further substituted with 0 to 4 substituents selected from H, a halogen, hydroxy, cyano, a C₁₋₄ alkyl, and a C₁₋₄ alkoxy;
Rₐ and R_{b} are each independently selected from -H and a C₁₋₆ alkyl;
n is independently selected from 0, 1, 2, and 3 at each occurrence.

8. The compound, or the stereoisomer, the *N*-oxide, the hydrate, the solvate, the pharmaceutically acceptable salt, or the polymorph thereof according to claim 1, wherein A is selected from the following structures: 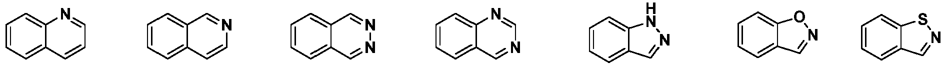 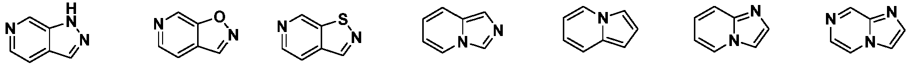 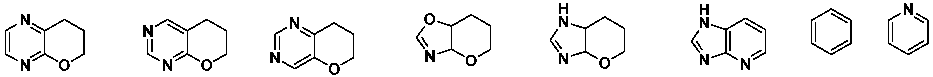 ; optionally wherein A is selected from one of the following structures: 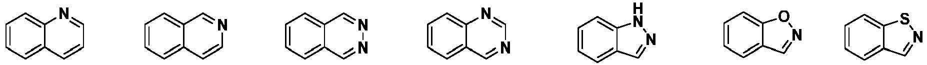 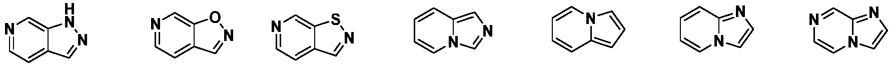 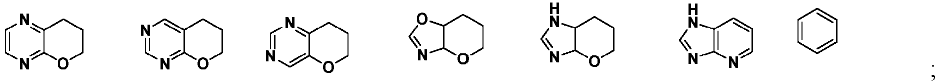 optionally wherein A is selected from the following structures:

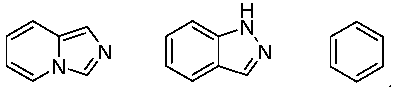

9. The compound, or the stereoisomer, the N-oxide, the hydrate, the solvate, the pharmaceutically acceptable salt, or the polymorph thereof according to claim 1, wherein the compound has structural features of Formula (a): 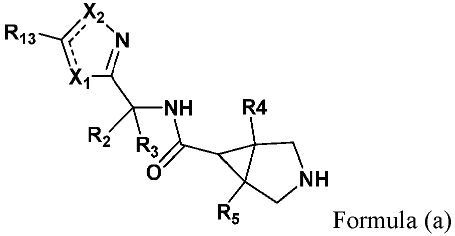 wherein
-̅ -̅ -̅ represents a single or double bond, provided that one and only one of two -̅ -̅ -̅ represents a double bond;
X₁ is CR₁₃ or NR₁₂;
X₂ is CR₁₃ or NR₁₂;
alternatively, X₁ and a substituent thereof together with an adjacent carbon atom and a substituent R₁₃ thereof form a benzene ring or a 5- to 6-membered heteroaromatic ring, and the benzene ring or the 5- to 6-membered heteroaromatic ring is optionally substituted with 1 to 4 R₁₃;
R₁₃ is each independently selected from -H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁;
R₁₂ is selected from H, a C₁₋₆ alkyl, and a halogenated C₁₋₆ alkyl;
R₁₁ is selected from -H, a C₁₋₆ alkyl, a halogenated C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
R₂ and R₃ are each independently selected from H, a C₁₋₆ alkyl, a halogenated C₁₋₆ alkyl, and -(CH₂)ₙ-OR;
R₄ and R₅ are each independently selected from H, a C₁₋₆ alkyl, a halogenated C₁₋₆ alkyl, and -(CH₂)ₙ-OR;
n is 0, 1, 2, or 3,
provided that at least one R₁₃ is an -SR₁₁ substituent;
optionally wherein either:
(a)
X₂ is CR₁₃;
X₁ and the substituent thereof together with the adjacent carbon atom and the substituent R₁₃ thereof form a benzene ring or a 5- to 6-membered heteroaromatic ring, and the benzene ring or the 5- to 6-membered heteroaromatic ring is substituted with 1 to 4 R₁₃;
R₁₃ is each independently selected from -H, halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁;
R₁₁ is selected from -H, a C₁₋₆ alkyl, a halogenated C₁₋₆ alkyl, a -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
R₂ and R₃ are each independently selected from H, a C₁₋₆ alkyl, a halogenated C₁₋₆ alkyl, and -(CH₂)ₙ-OR;
R₄ and R₅ are each independently selected from H, a C₁₋₆ alkyl, a halogenated C₁₋₆ alkyl, and -(CH₂)ₙ-OR;
n is 0, 1, 2, or 3,
provided that at least one R₁₃ is an -SR₁₁ substituent;
or
(b)
X₂ is NR₁₂;
X₁ and the substituent thereof together with the adjacent carbon atom and the substituent R₁₃ thereof form a benzene ring or a 5- to 6-membered heteroaromatic ring, and the benzene ring or the 5- to 6-membered heteroaromatic ring is substituted with 1 to 4 R₁₃;
R₁₃ is each independently selected from -H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁;
R₁₁ is selected from -H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
R₂ and R₃ are each independently selected from H, a C₁₋₆ alkyl, a halogenated C₁₋₆ alkyl, and -(CH₂)ₙ-OR;
R₄ and R₅ are each independently selected from H, a C₁₋₆ alkyl, a halogenated C₁₋₆ alkyl, and -(CH₂)ₙ-OR;
n is 0, 1, 2, or 3,
provided that at least one R₁₃ is -SR₁₁.

10. The compound, or the stereoisomer, the N-oxide, the hydrate, the solvate, the pharmaceutically acceptable salt, or the polymorph thereof according to claim 1, wherein the compound has structural features of Formula (b): 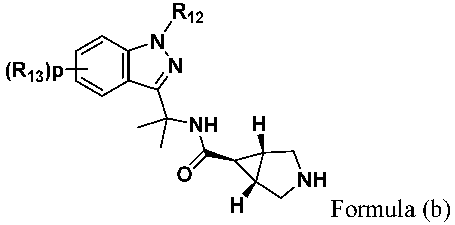 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁, and at least one R₁₃ is -SR₁₁;
R₁₂ is selected from H, a C₁₋₆ alkyl, and a halogenated C₁₋₆ alkyl;
R₁₁ is selected from H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R is selected from hydrogen, a C₁₋₆ alkyl, and a halogenated C₁₋₆ alkyl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
n is 0, 1, 2, or 3;
p is 1, 2, 3, or 4;
optionally wherein either:
(a)
p is 1, 2, or 3;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁;
R₁₁ is selected from a C₁₋₆ alkyl, a halogenated C₁₋₆ alkyl, and a -(CH₂)ₙ-C₃₋₆ carbocyclyl;
R₁₂ is selected from H, a C₁₋₆ alkyl, or a halogenated C₁₋₆ alkyl;
n is 0 or 1;
or
(b)
p is 1 or 2;
R₁₃ is each independently selected from H, a C₁₋₆ alkyl, a halogenated C₁₋₆ alkyl, and - SR₁₁, and at least one R₁₃ is -SR₁₁;
R₁₁ is selected from a C₁₋₄ alkyl and a -(CH₂)ₙ-C₃₋₄ carbocyclyl;
R₁₂ is selected from H, a C₁₋₄ alkyl, and a halogenated C₁₋₄ alkyl;
n is 0 or 1;
or
(c)
P is 1;
R₁₃ is -SMe;
R₁₂ is H or a C₁₋₄ alkyl.

11. The compound, or the stereoisomer, the *N*-oxide, the hydrate, the solvate, the pharmaceutically acceptable salt, or the polymorph thereof according to claim 1, wherein the compound has structural features of Formula (b-1): 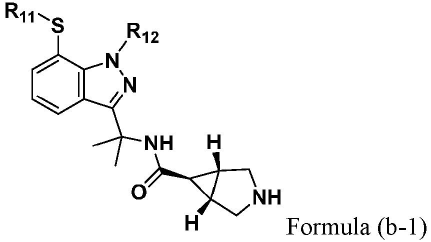 wherein
R₁₁ is selected from H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R₁₂ is selected from H, a C₁₋₆ alkyl, and a halogenated C₁₋₆ alkyl; and
n is 0, 1, 2, or 3;
optionally wherein either:
(a)
R₁₁ is selected from a C₁₋₆ alkyl, a halogenated C₁₋₆ alkyl, and a -(CH₂)ₙ-C₃₋₆ carbocyclyl;
R₁₂ is selected from H, a C₁₋₆ alkyl, and a halogenated C₁₋₆ alkyl; and
n is 0 or 1; or
(b)
R₁₁ is selected from a C₁₋₄ alkyl and a -(CH₂)ₙ-C₃₋₄ carbocyclyl;
R₁₂ is selected from H, a C₁₋₄ alkyl, and a halogenated C₁₋₄ alkyl;
n is 0 or 1; or
(c)
R₁₁ is methyl;
R₁₂ is H or a C₁₋₄ alkyl.

12. The compound, or the stereoisomer, the *N*-oxide, the hydrate, the solvate, the pharmaceutically acceptable salt, or the polymorph thereof according to claim 1, wherein the compound has structural features of Formula (c) or Formula (c-1) or Formula (c-2): 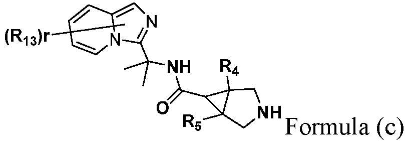 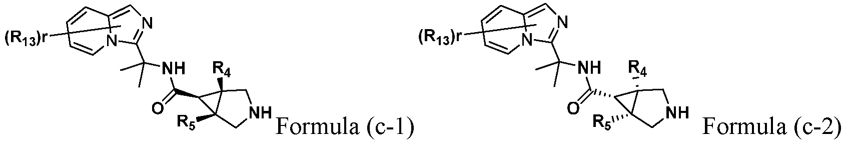 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁, and at least one R₁₃ is -SR₁₁;
R₁₁ is selected from H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R₄ and R₅ are each independently selected from H, a C₁₋₆ alkyl, a halogenated C₁₋₆ alkyl, and -(CH₂)ₙ-OR;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
r is 1, 2, 3, 4, or 5;
optionally wherein either:
(a)
r is 1, 2 or 3;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁;
R₁₁ is selected from a C₁₋₆ alkyl, a halogenated C₁₋₆ alkyl, and a -(CH₂)ₙ-C₃₋₆ carbocyclyl;
R₄ and R₅ are each independently selected from H and a C₁₋₆ alkyl;
n is 0 or 1; or
(b)
r is 1 or 2;
R₁₃ is each independently selected from H, a C₁₋₆ alkyl, a halogenated C₁₋₆ alkyl, and - SR₁₁, and at least one R₁₃ is -SR₁₁;
R₁₁ is selected from a C₁₋₄ alkyl and a -(CH₂)ₙ-C₃₋₄ carbocyclyl;
R₄ and R₅ are each independently selected from H and a C₁₋₆ alkyl;
n is 0 or 1; or
(c)
r is 2;
one of R₁₃ is -SMe, and another one is selected from H and a C₁₋₄ alkyl;
R₄ and R₅ are each independently selected from H and a C₁₋₄ alkyl; or
(d)
r is 1;
R₁₃ is -SMe;
R₄ and R₅ are each independently selected from H and a C₁₋₄ alkyl.

13. The compound, or the stereoisomer, the *N*-oxide, the hydrate, the solvate, the pharmaceutically acceptable salt, or the polymorph thereof according to claim 1, wherein the compound has structural features of Formula (d): 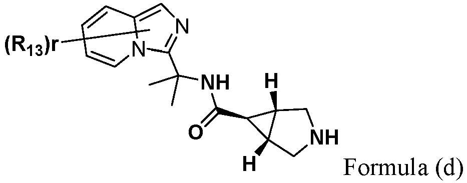 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁, and at least one R₁₃ is -SR₁₁;
R₁₁ is selected from H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
r is 1, 2, 3, 4, or 5;
optionally wherein either:
(a)
r is 1, 2 or 3;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁;
R₁₁ is selected from a C₁₋₆ alkyl, a halogenated C₁₋₆ alkyl, and a -(CH₂)ₙ-C₃₋₆ carbocyclyl;
n is 0 or 1;
or
(b)
r is 1 or 2;
R₁₃ is each independently selected from H, a C₁₋₆ alkyl, a halogenated C₁₋₆ alkyl, and - SR₁₁, and at least one R₁₃ is -SR₁₁;
R₁₁ is selected from a C₁₋₄ alkyl and a -(CH₂)ₙ-C₃₋₄ carbocyclyl;
n is 0 or 1;
or
(c)
r is 2;
one of R₁₃ is -SMe and another one is selected from H and a C₁₋₄ alkyl;
or
(d)
r is 1;
R₁₃ is -SMe.

14. The compound, or the stereoisomer, the *N*-oxide, the hydrate, the solvate, the pharmaceutically acceptable salt, or the polymorph thereof according to claim 1, wherein the compound has structural features of Formula (d-1): 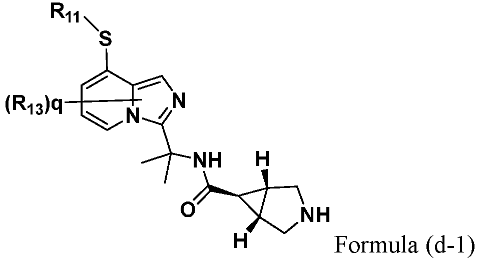 wherein
R₁₁ is selected from H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
q is 0, 1, 2, 3, or 4;
optionally wherein either:
(a)
q is 0, 1 or 2;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁;
R₁₁ is selected from a C₁₋₆ alkyl, a halogenated C₁₋₆ alkyl, and a -(CH₂)ₙ-C₃₋₆ carbocyclyl;
n is 0 or 1;
or
(b)
q is 0 or 1;
R₁₁ is selected from a C₁₋₄ alkyl and a -(CH₂)ₙ-C₃₋₄ carbocyclyl;
R₁₃ is selected from H, a C₁₋₄ alkyl, and a halogenated C₁₋₄ alkyl;
n is 0 or 1;
or
(c)
q is 0;
R₁₁ is a C₁₋₄ alkyl.

15. The compound, or the stereoisomer, the *N*-oxide, the hydrate, the solvate, the pharmaceutically acceptable salt, or the polymorph thereof according to claim 1, wherein the compound has structural features of Formula (d-2): 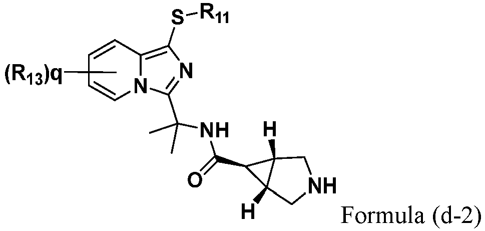 wherein
R₁₁ is selected from H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
q is 0, 1, 2, 3, or 4;
optionally wherein either:
(a)
q is 0, 1, or 2;
R₁₁ is selected from a C₁₋₆ alkyl, a halogenated C₁₋₆ alkyl, and a -(CH₂)ₙ-C₃₋₆ carbocyclyl;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁;
n is 0 or 1;
or
(b)
q is 0 or 1;
R₁₁ is selected from a C₁₋₄ alkyl, a halogenated C₁₋₄ alkyl, and a -(CH₂)ₙ-C₃₋₄ carbocyclyl;
R₁₃ is each independently selected from H, a C₁₋₆ alkyl, and a halogenated C₁₋₆ alkyl; n is 0 or 1;
or
(c)
q is 1;
R₁₁ is methyl;
R₁₃ is H or a C₁₋₄ alkyl.

16. The compound, or the stereoisomer, the *N*-oxide, the hydrate, the solvate, the pharmaceutically acceptable salt, or the polymorph thereof according to claim 1, wherein the compound has structural features of Formula (d-3): 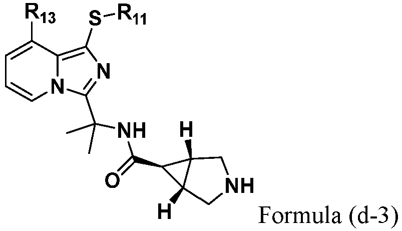 wherein
R₁₁ is selected from H, a C₁₋₆ alkyl, a halogenated C₁₋₆ alkyl, -(CH₂)ₙ-C₂₋₆ alkenyl, a - (CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₂₋₆ alkenyl, a -(CH₂)ₙ-C₂₋₆ alkynyl, a - (CH₂)ₙ-C₃₋₁₀ carbocyclyl, a -(CH₂)ₙ-(3- to 10-membered heterocyclyl), a -(CH₂)ₙ-C₆₋₁₀ aryl, a - (CH₂)ₙ-5- to 6-membered heteroaryl, and -SR₁₁;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
optionally wherein either
(a)
R₁₁ is selected from a C₁₋₆ alkyl, a halogenated C₁₋₆ alkyl, and a -(CH₂)ₙ-C₃₋₆ carbocyclyl;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁;
n is 0 or 1;
or
(b)
R₁₁ is selected from a C₁₋₄ alkyl, a halogenated C₁₋₄ alkyl, and a -(CH₂)ₙ-C₃₋₄ carbocyclyl;
R₁₃ is each independently selected from H, a C₁₋₄ alkyl, and a halogenated C₁₋₄ alkyl; n is 0 or 1;
or
(c)
R₁₁ is methyl;
R₁₃ is H or a C₁₋₄ alkyl.

17. The compound, or the stereoisomer, the *N*-oxide, the hydrate, the solvate, the pharmaceutically acceptable salt, or the polymorph thereof according to claim 1, wherein the compound has structural features of Formula (e): 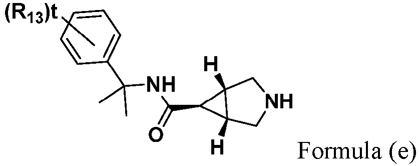 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₃₋₁₀ cycloaryl, and -SR₁₁, and at least one R₁₃ is -SR₁₁;
R₁₁ is selected from H, a C₁₋₆ alkyl, a halogenated C₁₋₆ alkyl, -(CH₂)ₙ-OR, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
t is 1, 2, 3, 4, or 5;
optionally wherein either
(a)
t is 1, 2, or 3;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁, and at least one R₁₃ is -SR₁₁;
R₁₁ is selected from a C₁₋₆ alkyl, a halogenated C₁₋₆ alkyl, and a -(CH₂)ₙ-C₃₋₆ carbocyclyl;
n is 0 or 1;
or
(b)
t is 1 or 2;
R₁₃ is each independently selected from H, a C₁₋₆ alkyl, a halogenated C₁₋₆ alkyl, and - SR₁₁, and at least one R₁₃ is -SR₁₁;
R₁₁ is selected from a C₁₋₄ alkyl and a -(CH₂)ₙ-C₃₋₄ carbocyclyl;
n is 0 or 1;
or
(c)
t is 1;
R₁₁ is a C₁₋₄ alkyl.

18. The compound, or the stereoisomer, the *N*-oxide, the hydrate, the solvate, the pharmaceutically acceptable salt, or the polymorph thereof according to claim 1, wherein the compound has structural features of Formula (e-1): 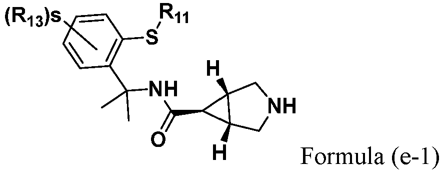 wherein
R₁₃ is each independently selected from H, a halogen, cyano, -(CH₂)ₙ-NR'R", a C₁₋₆ alkyl, a halogenated C₁₋₆ alkyl, -(CH₂)ₙ-OR, a -(CH₂)ₙ-C₃₋₁₀ cycloaryl, and -SR₁₁;
R₁₁ is selected from H, a C₁₋₆ alkyl, a halogenated C₁₋₆ alkyl, -(CH₂)ₙ-OR, -(CH₂)ₙ-C₂₋₆ alkenyl, a -(CH₂)ₙ-C₂₋₆ alkynyl, a -(CH₂)ₙ-C₃₋₁₀ carbocyclyl, a -(CH₂)ₙ-(3- to 10-membered heterocyclyl), a -(CH₂)ₙ-C₆₋₁₀ aryl, and a -(CH₂)ₙ-5- to 6-membered heteroaryl;
R' and R" are each selected from hydrogen, a C₁₋₆ alkyl, and a halogenated C₁₋₆ alkyl, or R' and R" together with a nitrogen atom attached thereto form a 3- to 10-membered heterocyclyl;
R is selected from hydrogen, a C₁₋₆ alkyl, and a halogenated C₁₋₆ alkyl;
n is 0, 1, 2, or 3;
s is 0, 1, 2, 3, or 4;
optionally wherein either
(a)
s is 0, 1, or 2;
R₁₃ is each independently selected from H, a halogen, a C₁₋₆ alkyl, a halogenated C₁₋₆ alkyl, a -(CH₂)ₙ-C₃₋₁₀ carbocyclyl, and -SR₁₁;
R₁₁ is selected from a C₁₋₆ alkyl, a halogenated C₁₋₆ alkyl, and a -(CH₂)ₙ-C₃₋₆ carbocyclyl;
n is 0 or 1;
or
(b)
s is 0 or 1;
R₁₃ is each independently selected from H, a C₁₋₆ alkyl, a halogenated C₁₋₆ alkyl, a - (CH₂)ₙ-C₃₋₁₀ carbocyclyl, and -SR₁₁;
R₁₁ is selected from a C₁₋₄ alkyl and a -(CH₂)ₙ-C₃₋₄ carbocyclyl;
n is 0 or 1;
or
(c)
s is 0;
R₁₁ is a C₁₋₄ alkyl.

19. The compound, or the stereoisomer, the *N*-oxide, the hydrate, the solvate, the pharmaceutically acceptable salt, or the polymorph thereof according to claim 1, selected from the following structures:

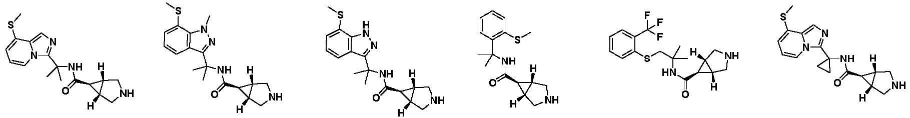

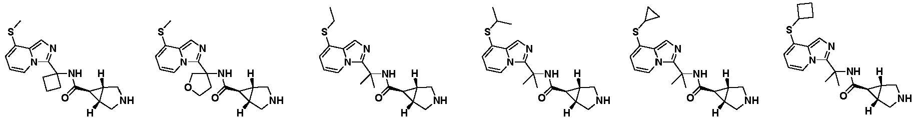

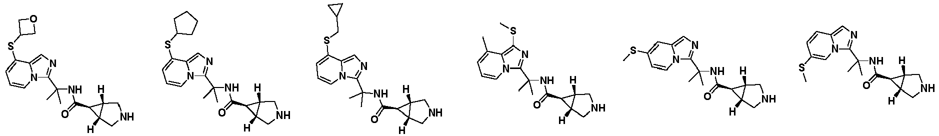

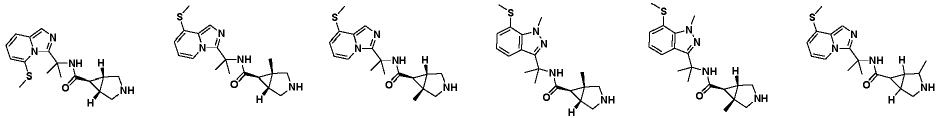

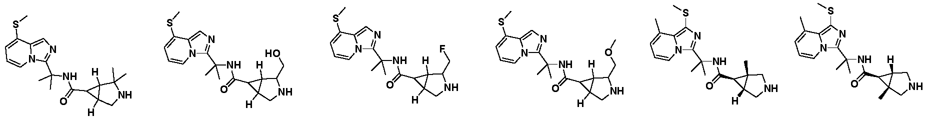

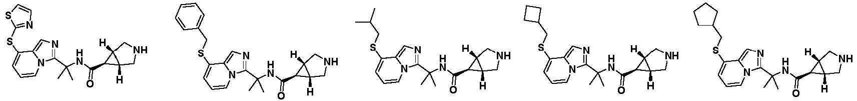

20. The compound, or the stereoisomer, the N-oxide, the hydrate, the solvate, the pharmaceutically acceptable salt, or the polymorph thereof according to any one of claims 1 to 19, wherein the pharmaceutically acceptable salt is selected from a hydrochloride, a hydrobromide, a sulfate, a nitrate, a phosphate, an acetate, a maleate, a succinate, a mandelate, a fumarate, a malonate, a malate, a 2-hydroxypropionate, an oxalate, a glycolate, a salicylate, a glucuronate, a galacturonate, a citrate, a tartrate, an aspartate, a glutamate, a benzoate, a cinnamate, a p-toluenesulfonate, a benzenesulfonate, a mesylate, an ethanesulfonate, and a trifluoromethanesulfonate, or combinations thereof.

21. A pharmaceutical composition, **characterized by** containing a therapeutically effective dose of the compound or the stereoisomer, the *N*-oxide, the hydrate, the solvate, the pharmaceutically acceptable salt, or the polymorph thereof according to any one of claims 1 to 20, and a pharmaceutically acceptable carrier or excipient.

22. The compound, or the stereoisomer, the *N*-oxide, the hydrate, the solvate, the pharmaceutically acceptable salt, or the polymorph thereof according to any one of claims 1 to 20, or the pharmaceutical composition according to claim 21, for use in therapy.

23. The compound or the stereoisomer, the *N*-oxide, the hydrate, the solvate, the pharmaceutically acceptable salt, or the polymorph thereof according to any one of claims 1 to 20 or the pharmaceutical composition according to claim 21, used to treat and/or prevent pain; optionally wherein:
(a) the pain is neuralgia; or
(b) the pain is back pain, chronic back pain, trigeminal neuralgia, type I complex regional pain syndrome, type II complex regional pain syndrome, irritable bowel syndrome, diabetic neuropathy, osteoarthritis-caused pain, tumor pain, or muscle fiber pain.

## Patentansprüche

1. Verbindung der Formel (I) oder ein Stereoisomer, N-Oxid, Hydrat, Solvat, pharmazeutisch annehmbares Salz oder Polymorph davon: 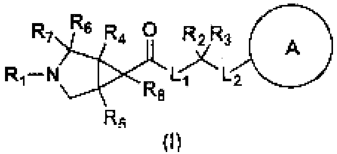 worin R₁ aus -H und einem C₁₋₆-Alkyl ausgewählt ist;
L₁ aus -NH- und -O- ausgewählt ist;
R₂ und R₃ jeweils unabhängig voneinander aus -H, einem C₁₋₆-Alkyl und einem C₃₋₆-Cycloalkyl ausgewählt sind, wobei R₂ und R₃ nicht beide -H sind; oder R₂ und R₃ zusammen eine 3- bis 6-gliedrige, gesättigte zyklische Gruppe bilden, die 0 bis 1 Gruppe umfasst, die aus -O-, NR₉-, -SO- und -SO₂- ausgewählt ist;
R₄, R₅, R₆, R₇ und R₈ jeweils unabhängig voneinander aus -H, einem C₁₋₆-Alkyl, einem C₁₋₆-Alkoxy, einem C₁₋₆-Alkoxy-C₁₋₆-alkyl, einem -(CH₂)ₘ-C₃₋₁₀-Carbocyclyl, einem -(CH₂)ₘ-(3-bis 10-gliedriges Heterocyclyl), einem -(CH₂)ₘ-O-C₃₋₁₀-Carbocyclyl, einem -(CH₂)ₘ-O-(3- bis 10-gliedriges Heterocyclyl), Phenyl und einem 5- bis 6-gliedrigem Heteroaryl ausgewählt sind, wobei das Heterocyclyl oder das Heteroaryl 1 bis 4 Heteroatome enthält, die aus N, O und S ausgewählt sind, und das Alkyl, das Alkoxy, das Carbocyclyl, das Phenyl, das Heteroaryl oder das Heterocyclyl in R₄, R₅, R₆, R₇ und R₈ jeweils unabhängig voneinander gegebenenfalls mit 0 bis 4 Substituenten, die aus -H, -F, -Cl, -Br, -I, Hydroxy, Sulfydryl, Cyano, Amino, einem C₁₋₄-Alkyl und einem C₁₋₄-Alkoxy ausgewählt sind, weiter substituiert sind;
R₉ aus -H, einem C₁₋₆-Alkyl, einem C₁₋₄-Alkoxy-C₁₋₆-alkyl, einem Halogen, Hydroxy, Cyano und einem C₃₋₆-Cycloalkyl ausgewählt ist;
A aus einem C₆₋₁₄-Aryl, einem 5- bis 14-gliedrigen Heteroaryl, einem 5- bis 14-gliedrigen Heterocyclyl und einem 5- bis 14-gliedrigen Cycloalkyl ausgewählt ist, wobei das Aryl, das Heteroaryl, das Heterocyclyl und das Cycloalkyl in A gegebenenfalls mit 1 bis 4 R₁₀ weiter substituiert sind, wobei das Heteroaryl oder das Heterocyclyl 1 bis 4 Heteroatome enthält, die aus N, O und S ausgewählt sind;
wobei, wenn L₂ aus einer Einfachbindung, -(CRₐR_{b})ₙ- und -(CRₐR_{b})ₙO- ausgewählt ist, R₁₀ dann jeweils unabhängig aus -H, -F, -Cl, -Br, -I, Hydroxy, Cyano, Amino, einem C₁₋₆-Alkyl, einem C₁₋₄-Alkoxy-C₁₋₆-alkyl, einem C₃₋₆-Cycloalkyl und -SR₁₁ ausgewählt ist und zumindest ein R₁₀ dann -SR₁₁ ist, worin R₁₁ jeweils unabhängig aus -H, einem C₁₋₆-Alkyl, einem C₁₋₄-Alkoxy-C₁₋₆-alkyl, einem -(CH₂)ₙ-C₂₋₆-Alkenyl, einem -(CH₂)ₙ-C₂₋₆-Alkinyl, einem (CH₂)ₙ-C₃₋₁₀-Carbocyclyl, einem -(CH₂)ₙ-(3- bis 10-gliedriges Heterocyclyl), einem C₆₋₁₀-Aryl und einem 5- bis 6-gliedrigem Heteroaryl ausgewählt ist, wobei das Heterocyclyl und das Heteroaryl 1 bis 4 Heteroatome enthalten, die aus N, O und S ausgewählt sind, wobei das Alkyl und das Alkoxy, das Aryl, das Heteroaryl, das Carbocyclyl oder das Heterocyclyl gegebenenfalls jeweils unabhängig mit 0 bis 4 Substituenten, die aus -H, einem Halogen, Hydroxy, Cyano, einem C₁₋₄-Alkyl und einem C₁₋₄-Alkoxy ausgewählt sind, weiter substituiert sind;
wobei, wenn L₂ aus -(CRₐR_{b})ₙS- ausgewählt ist, R₁₀ dann jeweils unabhängig aus -H, -F, -Cl, -Br, -I, Hydroxy, Cyano, Amino, einem C₁₋₄-Alkyl, einem C₁₋₄-Alkoxy, einem C₁₋₄-Alkoxy-C₁₋₆-alkyl, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -O-(CH₂)ₙ-C₃₋₁₀-Carbocyclyl oder -O-(CH₂)ₙ-(3- bis 10-gliedriges Heterocyclyl) und -SR₁₁ ausgewählt ist, worin R₁₁ jeweils unabhängig aus -H, einem C₁₋₆-Alkyl, einem C₁₋₄-Alkoxy-C₁₋₆-alkyl, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem C₆₋₁₀-Aryl und einem 5- bis 6-gliedrigem Heteroaryl ausgewählt ist, wobei das Heterocyclyl oder das Heteroaryl 1 bis 4 Heteroatome enthalten, die aus N, O und S ausgewählt sind, und wobei das Alkyl, das Alkoxy, das Aryl, das Heteroaryl, das Carbocyclyl oder das Heterocyclyl gegebenenfalls jeweils unabhängig mit 0 bis 4 Substituenten, die aus H, einem Halogen, Hydroxy, Cyano, einem C₁₋₄-Alkyl und einem C₁₋₄-Alkoxy ausgewählt sind, weiter substituiert sind;
Rₐ und R_{b} jeweils unabhängig voneinander aus -H und einem C₁₋₆-Alkyl ausgewählt sind;
m bei jedem Vorkommen unabhängig aus 0, 1, 2 und 3 ausgewählt ist;
n bei jedem Vorkommen unabhängig aus 0, 1, 2 und 3 ausgewählt ist.

2. Verbindung, Stereoisomer, N-Oxid, Hydrat, Solvat, pharmazeutisch annehmbares Salz oder Polymorph davon nach Anspruch 1, worin
L₁ -NH- ist;
R₁ -H ist.

3. Verbindung, Stereoisomer, N-Oxid, Hydrat, Solvat, pharmazeutisch annehmbares Salz oder Polymorph davon nach Anspruch 1, wobei die Verbindung Strukturmerkmale der Formel (VI) aufweist: 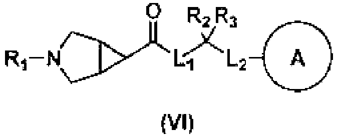 worin
L₁ aus -NH- und -O- ausgewählt ist;
L₂ aus einer Einfachbindung und -(CRₐR_{b})ₙ- oder -(CRₐR_{b})ₙO- ausgewählt ist, worin Rₐ und R_{b} jeweils unabhängig voneinander aus -H und einem C₁₋₆-Alkyl ausgewählt sind;
n bei jedem Vorkommen unabhängig aus 0, 1, 2 und 3 ausgewählt ist.

4. Verbindung, Stereoisomer, N-Oxid, Hydrat, Solvat, pharmazeutisch annehmbares Salz oder Polymorph davon nach Anspruch 1, wobei die Verbindung Strukturmerkmale der Formel (II) aufweist: 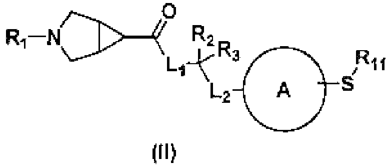 worin
L₁ aus -NH- und -O- ausgewählt ist;
L₂ aus einer Einfachbindung und -(CRₐR_{b}),- oder -(CRₐR_{b})ₙO- ausgewählt ist, worin Rₐ und R_{b} jeweils unabhängig voneinander aus -H und einem C₁₋₆-Alkyl ausgewählt sind;
n bei jedem Vorkommen unabhängig aus 0, 1, 2 und 3 ausgewählt ist;
wobei gegebenenfalls entweder:
(a) R₁ -H ist; L₁ -NH- ist; L₂ aus einer Einfachbindung, -CH₂-, -CH₂CH₂-, -CH₂CH₂CH₂-, CH₂O und -CH₂CH₂O ausgewählt ist; oder
(b) R₂ und R₃ jeweils unabhängig voneinander aus einem C₁₋₆-Alkyl und einem C₃₋₆-Cycloalkyl ausgewählt sind; oder R₂ und R₃ zusammen eine 3- bis 6-gliedrige, gesättigte zyklische Gruppe bilden, die 0 bis 1 Gruppe umfasst, die aus -O-, -SO- und -SO₂- ausgewählt ist; oder
(c) R₁₁ jeweils unabhängig aus -H, einem C₁₋₆-Alkyl, einem C₁₋₄-Alkoxy-C₁₋₆-alkyl, einem -(CH₂)ₙ-Alkenyl, einem -(CH₂)ₙ-Alkinyl, einem -(CH₂)ₙ-C₃₋₁₀-Carbocyclyl, einem -(CH₂)ₙ-(3- bis 10-gliedriges Heterocyclyl), einem C₆₋₁₀-Aryl und einem 5- bis 6-gliedrigem Heteroaryl ausgewählt ist, wobei das Heterocyclyl oder das Heteroaryl 1 bis 4 Heteroatome enthalten, die aus N, O und S ausgewählt sind, und wobei das Alkyl, das Alkoxy, das Aryl, das Heteroaryl, das Carbocyclyl oder das Heterocyclyl gegebenenfalls jeweils unabhängig mit 0 bis 4 Substituenten, die aus -H, einem Halogen, Hydroxy, Cyano, einem C₁₋₄-Alkyl und einem C₁₋₄-Alkoxy ausgewählt sind, weiter substituiert sind; wobei R₁₁ gegebenenfalls jeweils unabhängig aus Methyl, Ethyl, Isopropyl, Trifluormethyl, Cyclopropyl, Cyclobutyl, Cyclopentyl, Cyclohexyl, Phenyl, Imidazolyl, Pyrrolyl, Furyl, Thiophenyl und Pyridyl ausgewählt ist.

5. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (III) aufweist: 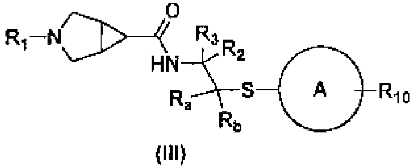 worin:
R₁₀ jeweils unabhängig aus -H, -F, -Cl, -Br, -I, Hydroxy, Sulfydryl, Cyano, Amino, einem C₁₋₄-Alkyl, einem C₁₋₄-Alkoxy, einem C₁₋₄-Alkoxy-C₁₋₆-alkyl, einem -(CH₂)ₙ-Alkenyl, einem -(CH₂)ₙ-Alkinyl, einem -O-(CH₂)ₙ-C₃₋₁₀-Carbocyclyl, einem -O-(CH₂)ₙ-(3- bis 10-gliedriges Heterocyclyl), einem -O-(CH₂)ₙ-C₃₋₁₀-Carbocyclyl, einem -O-(CH₂)ₙ-(3- bis 10-gliedriges Heterocyclyl) und -SR₁₁ ausgewählt ist, worin R₁₁ jeweils unabhängig aus -H, einem C₁₋₆-Alkyl, einem C₁₋₄-Alkoxy-C₁₋₆-alkyl, einem -(CH₂)ₙ-Alkenyl, einem -(CH₂)ₙ-Alkinyl, einem -(CH₂)ₙ-C₃₋₁₀-Carbocyclyl, einem -(CH₂)ₙ-(3- bis 10-gliedriges Heterocyclyl), einem C₆₋₁₀-Aryl und einem 5- bis 6-gliedrigem Heteroaryl ausgewählt ist, wobei das Heterocyclyl oder das Heteroaryl 1 bis 4 Heteroatome enthalten, die aus N, O und S ausgewählt sind, und wobei das Alkyl, das Alkoxy, das Aryl, das Heteroaryl, das Carbocyclyl oder das Heterocyclyl gegebenenfalls jeweils unabhängig mit 0 bis 4 Substituenten, die aus -H, einem Halogen, Hydroxy, Cyano, einem C₁₋₄-Alkyl und einem C₁₋₄-Alkoxy ausgewählt sind, weiter substituiert sind;
Rₐ und R_{b} jeweils unabhängig voneinander aus -H und einem C₁₋₆-Alkyl ausgewählt sind;
worin R₁₀ gegebenenfalls jeweils unabhängig aus -H, -F, -Cl, -Br, -I, Hydroxy, Cyano, Amino, Methyl, Ethyl, Isopropyl, Trifluormethyl, Cyclopropyl, Cyclobutyl, Cyclopentyl, Cyclobutoxy, Tetrahydrofuranyl und -SR₁₁ ausgewählt ist, worin R₁₁ jeweils unabhängig aus Methyl, Ethyl, Isopropyl, Trifluormethyl, Cyclopropyl, Cyclobutyl, Cyclopentyl, Cyclohexyl, Phenyl, Imidazolyl, Pyrrolyl, Furyl, Thienyl und Pyridyl ausgewählt ist; wobei Rₐ und R_{b} jeweils unabhängig aus -H, Methyl, Ethyl und Isopropyl ausgewählt sind.

6. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (IV) aufweist: 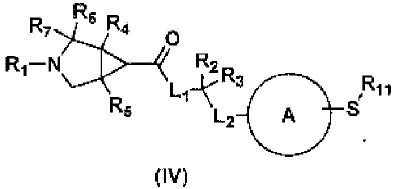 worin:
R₄ aus einem C₁₋₆-Alkyl, einem C₁₋₆-Alkoxy, einem C₁₋₆-Alkoxy-C₁₋₆-alkyl, einem (CH₂)ₘ-C₃₋₁₀-Carbocyclyl, einem -(CH₂)ₘ-(3- bis 10-gliedriges Heterocyclyl), einem -(CH₂)ₘ-O-C₃₋₁₀-Carbocyclyl, einem -(CH₂)ₘ-O-(3- bis 10-gliedriges Heterocyclyl), Phenyl und einem 5- bis 6-gliedrigem Heteroaryl ausgewählt ist, wobei das Heterocyclyl oder das Heteroaryl 1 bis 4 Heteroatome enthält, die aus N, O und S ausgewählt sind, und das Alkyl, das Alkoxy, das Carbocyclyl, das Phenyl, das Heteroaryl oder das Heterocyclyl in R₄ jeweils unabhängig gegebenenfalls mit 0 bis 4 Substituenten, die aus -H, -F, -Cl, -Br, -I, Hydroxy, Sulfydryl, Cyano, Amino, einem C₁₋₄-Alkyl und einem C₁₋₄-Alkoxy ausgewählt sind, weiter substituiert sind.

7. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (V) aufweist: 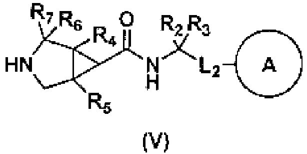 worin:
R₂ und R₃ jeweils unabhängig voneinander aus einem C₁₋₆-Alkyl und einem C₃₋₆-Cycloalkyl ausgewählt sind, worin R₂ und R₃ nicht beide -H sind; oder R₂ und R₃ zusammen ein 3- bis 6-gliedriges gesättigtes Cycloalkyl bilden;
R₄, R₅, R₆ und R₇ jeweils unabhängig voneinander aus -H, einem C₁₋₆-Alkyl, einem C₁₋₆-Alkoxy und einem C₁₋₆-Alkoxy-C₁₋₆-alkyl ausgewählt sind, und wobei das Alkyl und das Alkoxy in R₄, R₅, R₆ und R₇ gegebenenfalls jeweils unabhängig mit 0 bis 4 Substituenten weiter substituiert sind, die aus -H, -F, -Cl, -Br, -I, Hydroxy, Sulfydryl, Cyano, Amino, einem C₁₋₄-Alkyl und einem C₁₋₄-Alkoxy ausgewählt sind;
A aus einem C₆₋₁₄-Aryl, einem 5- bis 14-gliedrigen Heteroaryl, einem 5- bis 14-gliedrigen Heterocyclyl und einem 5- bis 14-gliedrigen Cycloalkyl ausgewählt ist, wobei das Aryl, das Heteroaryl, das Heterocyclyl und das Cycloalkyl in A gegebenenfalls mit 1 bis 4 R₁₀ weiter substituiert sind, wobei das Heteroaryl oder das Heterocyclyl 1 bis 4 N-Atome enthält;
wobei, wenn L₂ aus einer Einfachbindung ausgewählt ist, R₁₀ dann jeweils unabhängig aus H, -F, -Cl, -Br, -I, Hydroxy, Cyano, Amino, einem C₁₋₆-Alkyl, einem C₁₋₄-Alkoxy-C₁₋₆-alkyl, einem C₃₋₆-Cycloalkyl und -SR₁₁ ausgewählt ist und zumindest ein R₁₀ -SR₁₁ ist, worin R₁₁ jeweils unabhängig aus -H, einem C₁₋₆-Alkyl, einem C₁₋₄-Alkoxy-C₁₋₆-alkyl, einem -(CH₂)ₙ-C₃₋₁₀-Carbocyclyl, einem -(CH₂)ₙ-(3- bis 10-gliedriges Heterocyclyl), und einem 5- bis 6-gliedrigen Heteroaryl ausgewählt ist, wobei das Heterocyclyl oder das Heteroaryl 1 bis 4 Heteroatome enthalten, die aus O, S und N ausgewählt sind, und wobei das Alkyl, das Alkoxy, das Carbocyclyl oder das Heterocyclyl gegebenenfalls jeweils unabhängig mit 0 bis 4 Substituenten, die aus -H, einem Halogen, Hydroxy, Cyano, einem C₁₋₄-Alkyl und einem C₁₋₄-Alkoxy ausgewählt sind, weiter substituiert sind;
wobei, wenn L₂ aus -(CRₐR_{b})ₙS- ausgewählt ist, R₁₀ dann jeweils unabhängig aus -H, -F, -Cl, -Br, -I, Hydroxy, Cyano, Amino, einem C₁₋₄-Alkyl, einem C₁₋₄-Alkoxy und einem C₁₋₄-Alkoxy-C₁₋₆-alkyl ausgewählt ist, wobei das Alkyl und das Alkoxy gegebenenfalls jeweils unabhängig mit 0 bis 4 Substituenten, die aus H, einem Halogen, Hydroxy, Cyano, einem C₁₋₄-Alkyl und einem C₁₋₄-Alkoxy ausgewählt sind, weiter substituiert sind;
Rₐ und R_{b} jeweils unabhängig voneinander aus -H und einem C₁₋₆-Alkyl ausgewählt sind;
n bei jedem Vorkommen unabhängig aus 0, 1, 2 und 3 ausgewählt ist.

8. Verbindung, Stereoisomer, N-Oxid, Hydrat, Solvat, pharmazeutisch annehmbares Salz oder Polymorph davon nach Anspruch 1, worin A aus den folgenden Strukturen ausgewählt ist: 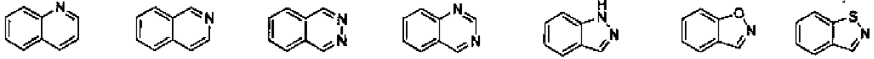 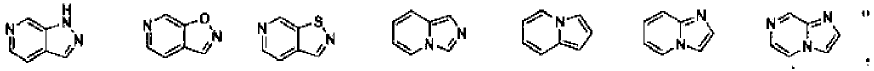 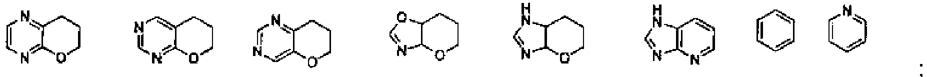 worin A gegebenenfalls aus einer der folgenden Strukturen ausgewählt ist: 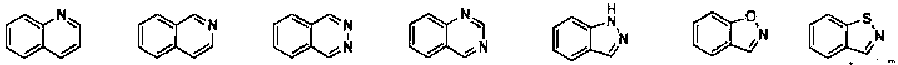 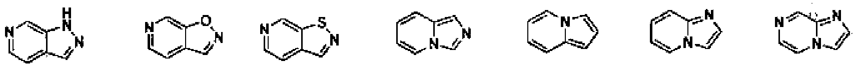 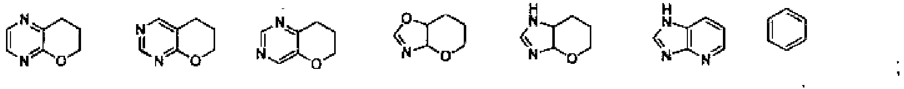 worin A gegebenenfalls aus den folgenden Strukturen ausgewählt ist:

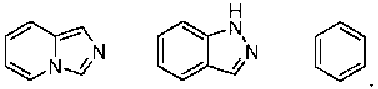

9. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (a) aufweist: 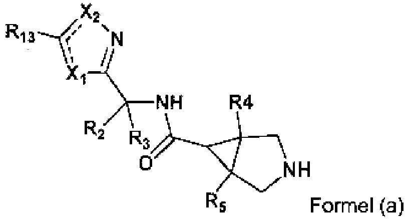 worin:
-̅ -̅ -̅ für eine Einfach- oder eine Doppelbindung steht, mit der Maßgabe, dass eines und nur eines von zwei -̅ -̅ -̅ für eine Doppelbindung steht;
X₁ CR₁₃ oder NR₁₂ ist;
X₂ CR₁₃ oder NR₁₂ ist;
wobei alternativ dazu X₁ und ein Substituent davon zusammen mit einem benachbarten Kohlenstoffatom und einem Substituenten R₁₃ davon einen Benzolring oder einen 5- bis 6-gliedrigen heteroaromatischen Ring bilden, wobei der Benzolring oder der 5- bis 6-gliedrige heteroaromatische Ring gegebenenfalls mit 1 bis 4 R₁₃ substituiert ist;
worin:
R₁₃ jeweils unabhängig aus -H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und SR₁₁ ausgewählt ist;
R₁₂ aus H, einem C₁₋₆-Alkyl und einem halogenierten C₁₋₆-Alkyl ausgewählt ist;
R₁₁ aus -H, einem C₁₋₆-Alkyl, einem halogenierten C₁₋₆-Alkyl, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl) und einem -(CH₂)ₙ-5- bis 6-gliedriges Heteroaryl ausgewählt ist;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
R₂ und R₃ jeweils unabhängig voneinander aus H, einem C₁₋₆-Alkyl, einem halogenierten C₁₋₆-Alkyl und -(CH₂)ₙ-OR ausgewählt sind;
R₄ und R₅ jeweils unabhängig voneinander aus H, einem C₁₋₆-Alkyl, einem halogenierten C₁₋₆-Alkyl und -(CH₂)ₙ-OR ausgewählt sind;
n = 0, 1, 2 oder 3 ist,
mit der Maßgabe, dass zumindest ein R₁₃ ein Substituent -SR₁₁ ist;
wobei gegebenenfalls entweder:
(a) X₂ CR₁₃ ist;
X₁ und der Substituent davon zusammen mit dem benachbarten Kohlenstoffatom und dem Substituenten R₁₃ davon einen Benzolring oder einen 5- bis 6-gliedrigen heteroaromatischen Ring bilden, wobei der Benzolring oder der 5- bis 6-gliedrige heteroaromatische Ring mit 1 bis 4 R₁₃ substituiert ist;
R₁₃ jeweils unabhängig aus -H, Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und SR₁₁ ausgewählt ist;
R₁₁ aus -H, einem C₁₋₆-Alkyl, einem halogenierten C₁₋₆-Alkyl, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3-bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
R₂ und R₃ jeweils unabhängig voneinander aus H, einem C₁₋₆-Alkyl, einem halogenierten C₁₋₆-Alkyl und -(CH₂)ₙ-OR ausgewählt sind;
R₄ und R₅ jeweils unabhängig voneinander aus H, einem C₁₋₆-Alkyl, einem halogenierten C₁₋₆-Alkyl und -(CH₂)ₙ-OR ausgewählt sind;
n = 0, 1, 2 oder 3 ist,
mit der Maßgabe, dass zumindest ein R₁₃ ein Substituent -SR₁₁ ist;
oder
(b) X₂ NR₁₂ ist;
X₁ und der Substituent davon zusammen mit dem benachbarten Kohlenstoffatom und dem Substituenten R₁₃ davon einen Benzolring oder einen 5- bis 6-gliedrigen heteroaromatischen Ring bilden, wobei der Benzolring oder der 5- bis 6-gliedrige heteroaromatische Ring mit 1 bis 4 R₁₃ substituiert ist;
R₁₃ jeweils unabhängig aus -H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und -SR₁₁ ausgewählt ist;
R₁₁ aus -H, einem C₁₋₆-Alkyl, einem halogenierten C₁₋₆-Alkyl, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
R₂ und R₃ jeweils unabhängig voneinander aus H, einem C₁₋₆-Alkyl, einem halogenierten C₁₋₆-Alkyl und -(CH₂)ₙ-OR ausgewählt sind;
R₄ und R₅ jeweils unabhängig voneinander aus H, einem C₁₋₆-Alkyl, einem halogenierten C₁₋₆-Alkyl und -(CH₂)ₙ-OR ausgewählt sind;
n = 0, 1, 2 oder 3 ist,
mit der Maßgabe, dass zumindest ein R₁₃ -SR₁₁ ist.

10. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (b) aufweist: 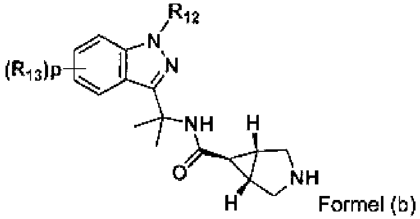 worin:
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und SR₁₁ ausgewählt ist und zumindest ein R₁₃ -SR₁₁ ist;
R₁₂ aus H, einem C₁₋₆-Alkyl und einem halogenierten C₁₋₆-Alkyl ausgewählt ist;
R₁₁ aus H, einem C₁₋₆-Alkyl, einem halogenierten C₁₋₆-Alkyl, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R aus Wasserstoff, einem C₁₋₆-Alkyl und einem halogenierten C₁₋₆-Alkyl ausgewählt ist;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
n = 0, 1, 2 oder 3 ist,
p = 1, 2, 3 oder 4 ist;
wobei gegebenenfalls entweder:
(a) p = 1, 2 oder 3 ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist und zumindest ein R₁₃ -SR₁₁ ist;
R₁₁ aus einem C₁₋₆-Alkyl, einem halogenierten C₁₋₆-Alkyl und einem -(CH₂)ₙ-C_{3 6}-Carbocyclyl ausgewählt ist;
R₁₂ aus H, einem C₁₋₆-Alkyl oder einem halogenierten C₁₋₆-Alkyl ausgewählt ist;
n = 0 oder 1 ist;
oder
(b) p = 1 oder 2 ist;
R₁₃ jeweils unabhängig aus H, einem C₁₋₆-Alkyl, einem halogenierten C₁₋₆-Alkyl und -SR₁₁ ausgewählt ist und zumindest ein R₁₃ -SR₁₁ ist;
R₁₁ aus einem C₁₋₄-Alkyl und einem -(CH₂)ₙ-C₃₋₄-Carbocyclyl ausgewählt ist;
R₁₂ aus H, einem C₁₋₄-Alkyl und einem halogenierten C₁₋₄-Alkyl ausgewählt ist;
n = 0 oder 1 ist;
oder
(c) p = 1 ist;
R₁₃ -SMe ist;
R₁₂ H oder ein C₁₋₄-Alkyl ist.

11. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (b-1) aufweist: 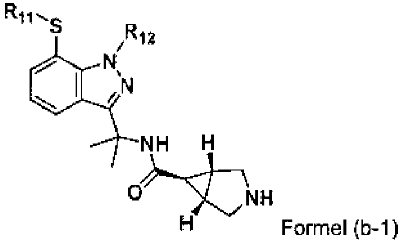 worin:
R₁₁ aus H, einem C₁₋₆-Alkyl, einem halogenierten C₁₋₆-Alkyl, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R₁₂ aus H, einem C₁₋₆-Alkyl und einem halogenierten C₁₋₆-Alkyl ausgewählt ist; und
n = 0, 1, 2 oder 3 ist;
wobei gegebenenfalls entweder:
(a) R₁₁ aus einem C₁₋₆-Alkyl, einem halogenierten C₁₋₆-Alkyl und einem -(CH₂)ₙ-C₃₋₆-Carbocyclyl ausgewählt ist;
R₁₂ aus H, einem C₁₋₆-Alkyl oder einem halogenierten C₁₋₆-Alkyl ausgewählt ist; und
n = 0 oder 1 ist;
oder
(b) R₁₁ aus einem C₁₋₄-Alkyl und einem -(CH₂)ₙ-C₃₋₄-Carbocyclyl ausgewählt ist;
R₁₂ aus H, einem C₁₋₄-Alkyl oder einem halogenierten C₁₋₄-Alkyl ausgewählt ist;
n = 0 oder 1 ist;
oder
(c) R₁₁ Methyl ist;
R₁₂ H oder ein C₁₋₄-Alkyl ist.

12. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (c) oder der Formel (c-1) oder der Formel (c-2) aufweist: 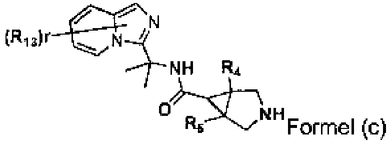 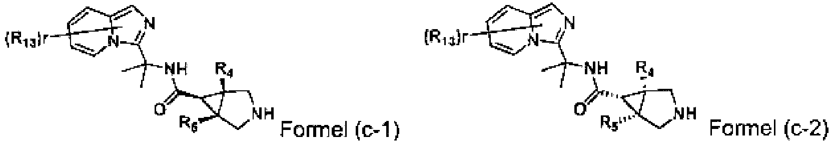 worin:
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und SR₁₁ ausgewählt ist und zumindest ein R₁₃ -SR₁₁ ist;
R₁₁ aus H, einem C₁₋₆-Alkyl, einem halogenierten C₁₋₆-Alkyl, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R₄ und R₅ jeweils unabhängig voneinander aus H, einem C₁₋₆-Alkyl, einem halogenierten C₁₋₆-Alkyl und -(CH₂)ₙ-OR ausgewählt sind;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
n = 0, 1, 2 oder 3 ist,
r = 1, 2, 3, 4 oder 5 ist;
wobei gegebenenfalls entweder:
(a) r = 1, 2 oder 3 ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist und zumindest ein R₁₃ -SR₁₁ ist;
R₁₁ aus einem C₁₋₆-Alkyl, einem halogenierten C₁₋₆-Alkyl und einem -(CH₂)ₙ-C_{3 6}-Carbocyclyl ausgewählt ist;
R₄ und R₅ jeweils unabhängig voneinander aus H und einem C₁₋₆-Alkyl ausgewählt sind;
n = 0 oder 1 ist;
oder
(b) r = 1 oder 2 ist;
R₁₃ jeweils unabhängig aus H, einem C₁₋₆-Alkyl, einem halogenierten C₁₋₆-Alkyl und -SR₁₁ ausgewählt ist und zumindest ein R₁₃ -SR₁₁ ist;
R₁₁ aus einem C₁₋₄-Alkyl und einem -(CH₂)ₙ-C₃₋₄-Carbocyclyl ausgewählt ist;
R₄ und R₅ jeweils unabhängig voneinander aus H und einem C₁₋₆-Alkyl ausgewählt sind;
n = 0 oder 1 ist;
oder
(c) r = 2 ist;
einer der R₁₃ -SMe ist und ein weiterer aus H und einem C₁₋₄-Alkyl ausgewählt ist;
R₄ und R₅ jeweils unabhängig voneinander aus H und einem C₁₋₄-Alkyl ausgewählt sind;
oder
(d) r = 1 ist;
R₁₃ -SMe ist;
R₄ und R₅ jeweils unabhängig voneinander aus H und einem C₁₋₄-Alkyl ausgewählt sind.

13. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (d) aufweist: 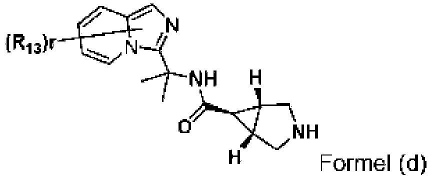 worin:
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und SR₁₁ ausgewählt ist und zumindest ein R₁₃ -SR₁₁ ist;
R₁₁ aus H, einem C₁₋₆-Alkyl, einem halogenierten C₁₋₆-Alkyl, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
n = 0, 1, 2 oder 3 ist,
r = 1, 2, 3, 4 oder 5 ist;
wobei gegebenenfalls entweder:
(a) r = 1, 2 oder 3 ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist und zumindest ein R₁₃ -SR₁₁ ist;
R₁₁ aus einem C₁₋₆-Alkyl, einem halogenierten C₁₋₆-Alkyl und einem -(CH₂)ₙ-C₃₋₆-Carbocyclyl ausgewählt ist;
n = 0 oder 1 ist;
oder
(b) r = 1 oder 2 ist;
R₁₃ jeweils unabhängig aus H, einem C₁₋₆-Alkyl, einem halogenierten C₁₋₆-Alkyl und -SR₁₁ ausgewählt ist und zumindest ein R₁₃ -SR₁₁ ist;
R₁₁ aus einem C₁₋₄-Alkyl und einem -(CH₂)ₙ-C₃₋₄-Carbocyclyl ausgewählt ist;
n = 0 oder 1 ist;
oder
(c) r = 2 ist;
einer der R₁₃ -SMe ist und ein weiterer aus H und einem C₁₋₄-Alkyl ausgewählt ist;
oder
(d) r = 1 ist;
R₁₃ -SMe ist.

14. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (d-1) aufweist: 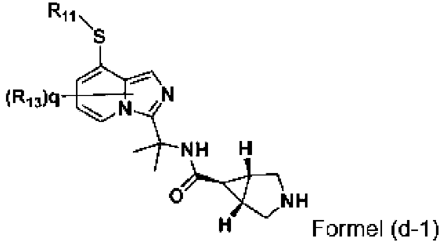 worin:
R₁₁ aus H, einem C₁₋₆-Alkyl, einem halogenierten C₁₋₆-Alkyl, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und -SR₁₁ ausgewählt ist;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
n = 0, 1, 2 oder 3 ist,
q = 0, 1, 2, 3 oder 4 ist;
wobei gegebenenfalls entweder:
(a) q = 0, 1 oder 2 ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist;
R₁₁ aus einem C₁₋₆-Alkyl, einem halogenierten C₁₋₆-Alkyl und einem -(CH₂)ₙ-C₃₋₆-Carbocyclyl ausgewählt ist;
n = 0 oder 1 ist;
oder
(b) q = 0 oder 1 ist;
R₁₁ aus einem C₁₋₄-Alkyl und einem -(CH₂)ₙ-C₃₋₄-Carbocyclyl ausgewählt ist;
R₁₃ aus H, einem C₁₋₄-Alkyl und einem halogenierten C₁₋₄-Alkyl ausgewählt ist;
n = 0 oder 1 ist;
oder
(c) q = 0 ist;
R₁₁ ein C₁₋₄-Alkyl ist.

15. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (d-2) aufweist: 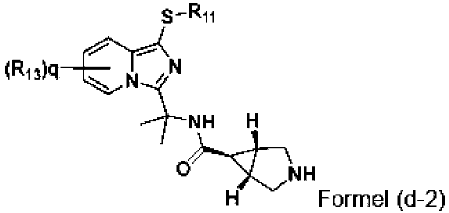 worin:
R₁₁ aus H, einem C₁₋₆-Alkyl, einem halogenierten C₁₋₆-Alkyl, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und SR₁₁ ausgewählt ist;
R' und R" jeweils aus Wasserstoff, einem C₁₋₆-Alkyl und einem halogenierten C₁₋₆-Alkyl ausgewählt sind oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
n = 0, 1, 2 oder 3 ist,
q = 0, 1, 2, 3 oder 4 ist;
wobei gegebenenfalls entweder:
(a) q = 0, 1 oder 2 ist;
R₁₁ aus einem C₁₋₆-Alkyl, einem halogenierten C₁₋₆-Alkyl und einem -(CH₂)ₙ-C_{3 6}-Carbocyclyl ausgewählt ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist;
n = 0 oder 1 ist;
oder
(b) q = 0 oder 1 ist;
R₁₁ aus einem C₁₋₄-Alkyl, einem halogenierten C₁₋₄-Alkyl und einem -(CH₂)ₙ-C₃₋₄-Carbocyclyl ausgewählt ist;
R₁₃ jeweils unabhängig aus H, einem C₁₋₆-Alkyl und einem halogenierten C₁₋₆-Alkyl ausgewählt ist;
n = 0 oder 1 ist;
oder
(c) q = 1 ist;
R₁₁ Methyl ist;
R₁₃ H oder ein C₁₋₄-Alkyl ist.

16. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (d-3) aufweist: 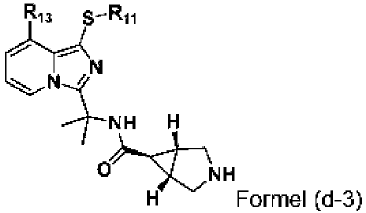 worin:
R₁₁ aus H, einem C₁₋₆-Alkyl, einem halogenierten C₁₋₆-Alkyl, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl, einem -(CH₂)ₙ-5- bis 6-gliedriges Heteroaryl und -SR₁₁ ausgewählt ist;
R' und R" jeweils aus Wasserstoff, einem C₁₋₆-Alkyl und einem halogenierten C₁₋₆-Alkyl ausgewählt sind, oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
n = 0, 1, 2 oder 3 ist,
wobei gegebenenfalls entweder:
(a) R₁₁ aus einem C₁₋₆-Alkyl, einem halogenierten C₁₋₆-Alkyl und einem -(CH₂)ₙ-C₃₋₆-Carbocyclyl ausgewählt ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist;
n = 0 oder 1 ist;
oder
(b) R₁₁ aus einem C₁₋₄-Alkyl, einem halogenierten C₁₋₄-Alkyl und einem -(CH₂)ₙ-C₃₋₄-Carbocyclyl ausgewählt ist;
R₁₃ jeweils unabhängig aus H, einem C₁₋₄-Alkyl und einem halogenierten C₁₋₄-Alkyl ausgewählt ist;
n = 0 oder 1 ist;
oder
(c) R₁₁ Methyl ist;
R₁₃ H oder ein C₁₋₄-Alkyl ist.

17. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (e) aufweist: 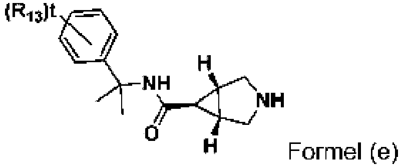 worin:
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₃₋₁₀-Cycloaryl und -SR₁₁ ausgewählt ist und zumindest ein R₁₃ -SR₁₁ ist;
R₁₁ aus H, einem C₁₋₆-Alkyl, einem halogenierten C₁₋₆-Alkyl, -(CH₂)ₙ-OR, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R' und R" jeweils aus Wasserstoff, einem C₁₋₆-Alkyl und einem halogenierten C₁₋₆-Alkyl ausgewählt sind, oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
n = 0, 1, 2 oder 3 ist,
t = 1, 2, 3, 4 oder 5 ist;
wobei gegebenenfalls entweder:
(a) t = 1, 2 oder 3 ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist und zumindest ein R₁₃ -SR₁₁ ist;
R₁₁ aus einem C₁₋₆-Alkyl, einem halogenierten C₁₋₆-Alkyl und einem -(CH₂)ₙ-C₃₋₆-Carbocyclyl ausgewählt ist;
n = 0 oder 1 ist;
oder
(b) t = 1 oder 2 ist;
R₁₃ jeweils unabhängig aus H, einem C₁₋₆-Alkyl, einem halogenierten C₁₋₆-Alkyl und -SR₁₁ ausgewählt ist und zumindest ein R₁₃ -SR₁₁ ist;
R₁₁ aus einem C₁₋₄-Alkyl und einem -(CH₂)ₙ-C₃₋₄-Carbocyclyl ausgewählt ist; n = 0 oder 1 ist;
oder
(c) t = 1 ist;
R₁₁ ein C₁₋₄-Alkyl ist.

18. Verbindung oder Stereoisomer, N-Oxid, Hydrat, Solvat, pharmazeutisch annehmbares Salz oder Polymorph davon nach Anspruch 1, wobei die Verbindung Strukturmerkmale der Formel (e-1) aufweist: 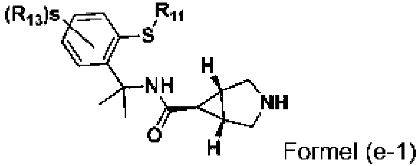 worin:
R₁₃ jeweils unabhängig aus H, einem Halogen, Cyano, -(CH₂)ₙ-NR'R", einem C₁₋₆-Alkyl, einem halogenierten C₁₋₆-Alkyl, -(CH₂)ₙ-OR, einem -(CH₂)ₙ-C₃₋₁₀-Cycloaryl und -SR₁₁ ausgewählt ist;
R₁₁ aus H, einem C₁₋₆-Alkyl, einem halogenierten C₁₋₆-Alkyl, -(CH₂)ₙ-OR, -(CH₂)ₙ-C₂₋₆-Alkenyl, einem -(CH₂)ₙ-C₂₋₆-Alkinyl, einem -(CH₂)ₙ-C₃₋₁₀-Carbocyclyl, einem -(CH₂)ₙ-(3- bis 10-gliedriges Heterocyclyl), einem -(CH₂)ₙ-C₆₋₁₀-Aryl und einem -(CH₂)ₙ-5- bis 6-gliedriges Heteroaryl ausgewählt ist;
R' und R" jeweils aus Wasserstoff, einem C₁₋₆-Alkyl und einem halogenierten C₁₋₆-Alkyl ausgewählt sind, oder R' und R" zusammen mit einem Stickstoffatom, an das sie gebunden sind, ein 3- bis 10-gliedriges Heterocyclyl bilden;
R aus Wasserstoff, einem C₁₋₆-Alkyl und einem halogenierten C₁₋₆-Alkyl ausgewählt ist;
n = 0, 1, 2 oder 3 ist,
s = 0, 1, 2, 3 oder 4 ist;
wobei gegebenenfalls entweder:
(a) s = 0, 1 oder 2 ist;
R₁₃ jeweils unabhängig aus H, einem Halogen, einem C₁₋₆-Alkyl, einem halogenierten C₁₋₆-Alkyl, einem -(CH₂)ₙ-C₃₋₁₀-Carbocyclyl und -SR₁₁ ausgewählt ist;
R₁₁ aus einem C₁₋₆-Alkyl, einem halogenierten C₁₋₆-Alkyl und einem -(CH₂)ₙ-C_{3 6}-Carbocyclyl ausgewählt ist;
n = 0 oder 1 ist;
oder
(b) s = 0 oder 1 ist;
R₁₃ jeweils unabhängig aus H, einem C₁₋₆-Alkyl, einem halogenierten C₁₋₆-Alkyl, einem -(CH₂)ₙ-C₃₋₁₀-Carbocyclyl und -SR₁₁ ausgewählt ist;
R₁₁ aus einem C₁₋₄-Alkyl und einem -(CH₂)ₙ-C₃₋₄-Carbocyclyl ausgewählt ist;
n = 0 oder 1 ist;
oder
(c) s = 0 ist;
R₁₁ ein C₁₋₄-Alkyl ist.

19. Verbindung, Stereoisomer, N-Oxid, Hydrat, Solvat, pharmazeutisch annehmbares Salz oder Polymorph davon nach Anspruch 1, das aus den folgenden Strukturen ausgewählt ist:

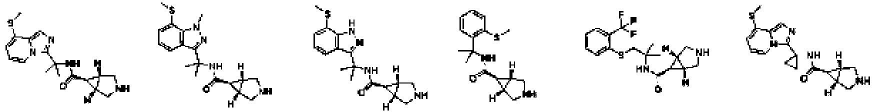

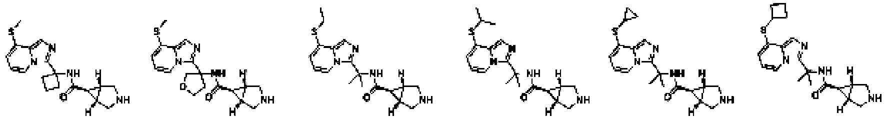

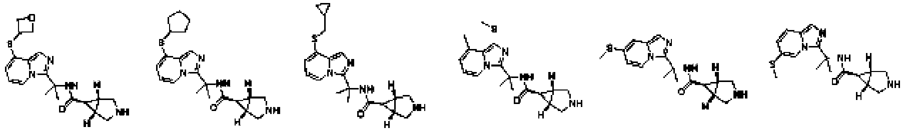

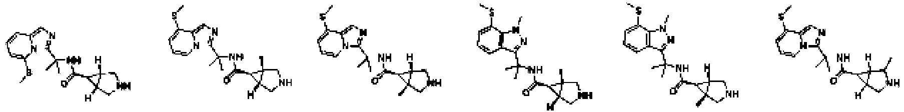

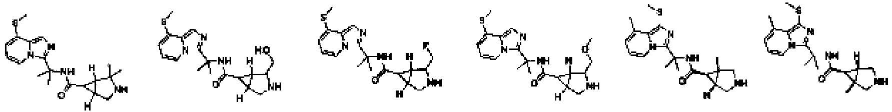

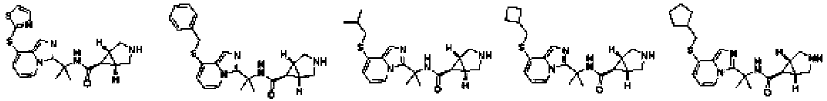

20. Verbindung, Stereoisomer, N-Oxid, Hydrat, Solvat, pharmazeutisch annehmbares Salz oder Polymorph davon nach einem der Ansprüche 1 bis 19, wobei das pharmazeutisch annehmbare Salz aus einem Hydrochlorid, einem Hydrobromid, einem Sulfat, einem Nitrat, einem Phosphat, einem Acetat, einem Maleat, einem Succinat, einem Mandelat, einem Fumarat, einem Malonat, einem Malat, einem 2-Hydroxypropionat, einem Oxalat, einem Glykolat, einem Salicylat, einem Glucuronat, einem Galacturonat, einem Citrat, einem Tartrat, einem Aspartat, einem Glutamat, einem Benzoat, einem Cinnamat, einem p-Toluolsulfonat, einem Benzolsulfonat, einem Mesylat, einem Ethansulfonat und einem Trifluormethansulfonat oder Kombinationen davon ausgewählt ist.

21. Pharmazeutische Zusammensetzung, die durch das Enthalten einer therapeutisch wirksamen Dosis einer Verbindung oder eines Stereoisomers, eines N-Oxids, eines Hydrats, eines Solvats, eines pharmazeutisch annehmbaren Salzes oder eines Polymorphs davon nach einem der Ansprüche 1 bis 20 und eines pharmazeutisch annehmbaren Trägers oder Hilfsstoffs gekennzeichnet ist.

22. Verbindung, Stereoisomer, N-Oxid, Hydrat, Solvat, pharmazeutisch annehmbares Salz oder Polymorph davon nach einem der Ansprüche 1 bis 20 oder pharmazeutische Zusammensetzung nach Anspruch 21 zur Verwendung in einer Therapie.

23. Verbindung, Stereoisomer, N-Oxid, Hydrat, Solvat, pharmazeutisch annehmbares Salz oder Polymorph davon nach einem der Ansprüche 1 bis 20 oder pharmazeutische Zusammensetzung nach Anspruch 21 zur Verwendung bei der Behandlung und/oder Prävention von Schmerzen; wobei gegebenenfalls:
(a) die Schmerzen Neuralgie sind; oder
(b) die Schmerzen Rückenschmerzen, chronische Rückenschmerzen, Trigeminus-Neuralgie, komplexes regionales Schmerzsyndrom Typ I, komplexes regionales Schmerzsyndrom Typ II, Reizdarm, diabetische Neuropathie, durch Osteoarthritis verursachte Schmerzen, Tumorschmerzen oder Muskelfaserschmerzen sind.

## Revendications

1. Composé de formule (I), ou un stéréoisomère, un N-oxyde, un hydrate, un solvate, un sel pharmaceutiquement acceptable ou un polymorphe de celui-ci : 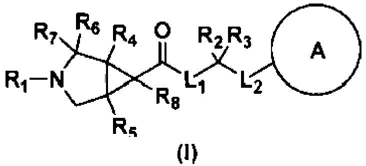
dans laquelle R₁ est choisi parmi -H et un alkyle en C₁₋₆ ;
L₁ est choisi parmi -NH- et -O- ;
R₂ et R₃ sont chacun indépendamment choisis parmi -H, un alkyle en C₁₋₆ et un cycloalkyle en C₃₋₆, lesquels R₂ et R₃ ne sont pas tous deux -H ; ou lesquels R₂ et R₃ forment ensemble un groupe cyclique saturé à 3 à 6 chaînons comprenant de 0 à 1 groupe choisi parmi -O-, -NR₉-, -SO- et -SO₂- ;
R₄, R₅, R₆, R₇ et R₈ sont chacun indépendamment choisis parmi -H, un alkyle en C₁₋₆, un alcoxy en C₁₋₆, un alcoxy en C₁₋₆-alkyle en C₁, un -(CH₂)ₘ-carbocyclyle en C₃₋₁₀, un -(CH₂)ₘ-(hétérocyclyle à 3 à 10 chaînons), un -(CH₂)ₘ-O-carbocyclyle en C₃₋₁₀, un -(CH₂)ₘ-O-(hétérocyclyle à 3 à 10 chaînons), phényle et un hétéroaryle à 5 à 6 chaînons, l'hétérocyclyle ou l'hétéroaryle contenant de 1 à 4 hétéroatomes choisis parmi N, O et S, et l'alkyle, l'alcoxy, le carbocyclyle, le phényle, l'hétéroaryle ou l'hétérocyclyle dans R₄, R₅, R₆, R₇ et R₈ étant chacun indépendamment éventuellement substitués davantage par 0 à 4 substituants choisis parmi -H, -F, -Cl, -Br, -I, hydroxy, sulfhydryle, cyano, amino, un alkyle en C₁₋₄, et un alcoxy en C₁₋₄ ;
R₉ est choisi parmi -H, un alkyle en C₁₋₆, un alcoxy en C₁₋₄-alkyle en C₁₋₆, un halogène, hydroxy, cyano et un cycloalkyle en C₃₋₆ ;
A est choisi parmi un aryle en C₆₋₁₄, un hétéroaryle à 5 à 14 chaînons, un hétérocyclyle à 5 à 14 chaînons et un cycloalkyle à 5 à 14 chaînons, et l'aryle, l'hétéroaryle, l'hétérocyclyle et le cycloalkyle dans A étant éventuellement substitués davantage par 1 à 4 R₁₀, l'hétéroaryle ou l'hétérocyclyle contenant de 1 à 4 hétéroatomes choisis parmi N, O et S ;
lorsque L₂ est choisi parmi une liaison simple, - (CRₐR_{b})ₙ₋, et -(CRₐR_{b})ₘO-, alors R₁₀ est indépendamment choisi parmi -H, -F, -Cl, -Br, -I, hydroxy, cyano, amino, un alkyle en C₁₋₆, un alcoxy en C₁₋₄-alkyle en C₁₋₆, un cycloalkyle en C₃₋₆, et -SR₁₁, et alors au moins un R₁₀ est -SR₁₁, dans lequel R₁₁ est indépendamment choisi parmi -H, un alkyle en C₁₋₆, un alcoxy en C₁₋₄-alkyle en C₁₋₆, un -(CH₂)ₙ-alcényle en C₂₋₆, un - (CH₂)ₙ-alcynyle en C₂₋₆, un -(CH₂)ₙ-carbocyclyle en C₃₋₁₀, un - (CH₂)ₙ-(hétérocyclyle à 3 à 10 chaînons), un aryle en C₆₋₁₀ et un hétéroaryle à 5 à 6 chaînons, l'hétérocyclyle et l'hétéroaryle contenant de 1 à 4 hétéroatomes choisis parmi N, O et S, et l'alkyle, l'alcoxy, l'aryle, l'hétéroaryle, le carbocyclyle ou l'hétérocyclyle étant chacun indépendamment éventuellement substitué davantage par 0 à 4 substituants choisis parmi -H, un halogène, hydroxy, cyano, un alkyle en C₁₋₄ et un alcoxy en C₁₋₄ ;
lorsque L₂ est choisi parmi -(CRₐR_{b})ₙS-, alors R₁₀ est indépendamment choisi parmi -H, -F, -Cl, -Br, -I, hydroxy, cyano, amino, un alkyle en C₁₋₄, un alcoxy en C₁₋₄, un alcoxy en C₁₋₄-alkyle en C₁₋₆, un -(CH₂)ₙ-alcényle en C₂₋₆, un (CH₂)ₙ-alcynyle en C₂₋₆, un (CH₂)ₙ-carbocyclyle en C₃₋₁₀, un (CH₂)ₙ-(hétérocyclyle à 3 à 10 chaînons), un -O-(CH₂)ₙ-carbocyclyle en C₃₋₁₀ ou -O-(CH₂)ₙ-(hétérocyclyle à 3 à 10 chaînons), et - SR₁₁, dans lequel R₁₁ est indépendamment choisi parmi -H, un alkyle en C₁₋₆, un alcoxy en C₁₋₄-alkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un aryle en C₆₋₁₀ et un hétéroaryle à 5 à 6 chaînons, l'hétérocyclyle ou l'hétéroaryle contenant de 1 à 4 hétéroatomes choisis parmi N, O et S, et l'alkyle, l'alcoxy, l'aryle, l'hétéroaryle, le carbocyclyle ou l'hétérocyclyle étant chacun indépendamment éventuellement substitué davantage par 0 à 4 substituants choisis parmi H, un halogène, hydroxy, cyano, un alkyle en C₁₋₄ et un alcoxy en C1-4 ;
Rₐ et R_{b} sont chacun indépendamment choisis parmi -H et un alkyle en C₁₋₆ ;
m est indépendamment choisi parmi 0, 1, 2 et 3 à chaque occurrence ;
n est indépendamment choisi parmi 0, 1, 2 et 3 à chaque occurrence.

2. Composé, ou le stéréoisomère, le *N*-oxyde, l'hydrate, le solvate, le sel pharmaceutiquement acceptable ou le polymorphe de celui-ci selon la revendication 1, dans lequel
L₁ est -NH- ;
R₁ est -H.

3. Composé, ou le stéréoisomère, le *N*-oxyde, l'hydrate, le solvate, le sel pharmaceutiquement acceptable ou le polymorphe de celui-ci selon la revendication 1, dans lequel le composé présente les caractéristiques structurelles de la formule (VI) : 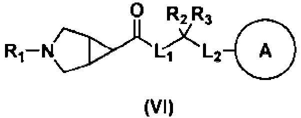
L₁ est choisi parmi -NH- et -O- ;
L₂ est choisi parmi une liaison simple et -(CRₐR_{b})ₙ- ou -(CRₐR_{b})ₙO-, dans lequel Rₐ et R_{b} sont indépendamment choisis parmi -H et un alkyle en C₁₋₆ ;
n est indépendamment choisi parmi 0, 1, 2 et 3 à chaque occurrence.

4. Composé, ou le stéréoisomère, le *N*-oxyde, l'hydrate, le solvate, le sel pharmaceutiquement acceptable ou le polymorphe de celui-ci selon la revendication 1, dans lequel le composé présente les caractéristiques structurelles de la formule (II) : 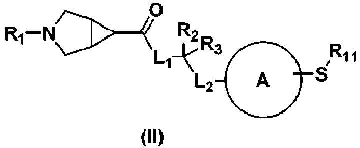
L₁ est choisi parmi -NH- et -O- ;
L₂ est choisi parmi une liaison simple, -(CRₐR_{b})ₙ-, et -(CRₐR_{b})ₙO-, dans lequel Rₐ et R_{b} sont chacun indépendamment choisis parmi -H et un alkyle en C₁₋₆ ;
n est indépendamment choisi parmi 0, 1, 2 et 3 à chaque occurrence ; éventuellement dans lequel soit :
(a) R₁ est -H ; L₁ est -NH- ; L₂ est choisi parmi une liaison simple, -CH₂-, -CH₂CH₂-, -CH₂CH₂CH₂-, -CH₂O-, et -CH₂CH₂O- ; ou
(b) R₂ et R₃ sont chacun indépendamment choisis parmi un alkyle en C₁₋₆ et un cycloalkyle en C₃₋₆ ; ou R₂ et R₃ forment ensemble un groupe cyclique saturé à 3 à 6 chaînons comprenant de 0 à 1 groupe choisi parmi -O-, -SO- et -SO₂- ; ou
(c) R₁₁ est chacun indépendamment choisi parmi -H, un alkyle en C₁₋₆, un alcoxy en C₁₋₄-alkyle en C₁₋₆, un -(CH₂)ₙ-alcényle, un -(CH₂)ₙ-alcynyle, un -(CH₂)ₙ-carbocyclyle en C₃₋₁₀, un -(CH₂)ₙ-(hétérocyclyle à 3 à 10 chaînons), un aryle en C₆₋₁₀ et un hétéroaryle à 5 à 6 chaînons, l'hétérocyclyle ou l'hétéroaryle contenant de 1 à 4 hétéroatomes choisis parmi N, O et S, et l'alkyle, l'alcoxy, l'aryle, l'hétéroaryle, le carbocyclyle ou l'hétérocyclyle étant chacun indépendamment éventuellement substitué davantage par 0 à 4 substituants choisis parmi -H, un halogène, hydroxy, cyano, un alkyle en C₁₋₄ et un alcoxy en C₁₋₄ ; éventuellement dans lequel R₁₁ est chacun indépendamment choisi parmi méthyle, éthyle, isopropyle, trifluorométhyle, cyclopropyle, cyclobutyle, cyclopentyle, cyclohexyle, phényle, imidazolyle, pyrrolyle, furyle, thiényle et pyridyle.

5. Composé, ou le stéréoisomère, le *N*-oxyde, l'hydrate, le solvate, le sel pharmaceutiquement acceptable ou le polymorphe de celui-ci selon la revendication 1, dans lequel le composé présente les caractéristiques structurelles de la formule (III) : 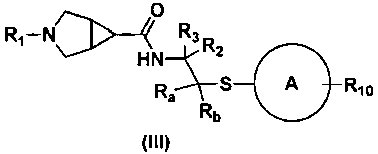
R₁₀ est chacun indépendamment choisi parmi -H, -F, -Cl, -Br, -I, hydroxy, cyano, amino, un alkyle en C₁₋₄, un alcoxy en C₁₋₄, un alcoxy en C₁₋₄-alkyle en C₁₋₆, un -(CH₂)ₙ-alcényle, un
-(CH₂)ₙ-alcynyle, un -O-(CH₂)ₙ-carbocyclyle en C₃₋₁₀, un -O-(CH₂)ₙ-(hétérocyclyle à 3 à 10 chaînons), un -O-(CH₂)ₙ-carbocyclyle en C₃₋₁₀, un -O-(CH₂)ₙ-(hétérocyclyle à 3 à 10 chaînons) et -SR₁₁, dans lequel R₁₁ est chacun indépendamment choisi parmi -H, un alkyle en C₁₋₆, un alcoxy en C₁₋₄-alkyle en C₁₋₆, un -(CH₂)ₙ-alcényle, un -(CH₂)ₙ-alcynyle, un -(CH₂)ₙ-carbocyclyle en C₃₋₁₀, un -(CH₂)ₙ-(hétérocyclyle à 3 à 10 chaînons), un aryle en C₆₋₁₀ et un hétéroaryle à 5 à 6 chaînons, l'hétérocyclyle ou l'hétéroaryle contenant de 1 à 4 hétéroatomes choisis parmi N, O et S, et l'alkyle, l'alcoxy, l'aryle, l'hétéroaryle, le carbocyclyle ou l'hétérocyclyle étant chacun indépendamment éventuellement substitué davantage par 0 à 4 substituants choisis parmi -H, un halogène, hydroxy, cyano, un alkyle en C₁₋₄ et un alcoxy en C1-4 ;
Rₐ et R_{b} sont chacun indépendamment choisis parmi -H et un alkyle en C₁₋₆ ;
éventuellement dans lequel R₁₀ est chacun indépendamment choisi parmi -H, -F, -Cl, -Br, -I, hydroxy, cyano, amino, méthyle, éthyle, isopropyle, trifluorométhyle, cyclopropyle, cyclobutyle, cyclopentyle, cyclobutoxy, tétrahydrofuranyle et -SR₁₁, dans lequel R₁₁ est chacun indépendamment choisi parmi méthyle, éthyle, isopropyle, trifluorométhyle, cyclopropyle, cyclobutyle, cyclopentyle, cyclohexyle, phényle, imidazolyle, pyrrolyle, furyle, thiényle et pyridyle ; Rₐ et R_{b} sont chacun indépendamment choisis parmi -H, méthyle, éthyle et isopropyle.

6. Composé, ou le stéréoisomère, le N-oxyde, l'hydrate, le solvate, le sel pharmaceutiquement acceptable ou le polymorphe de celui-ci selon la revendication 1, dans lequel le composé présente les caractéristiques structurelles de la formule (IV) : 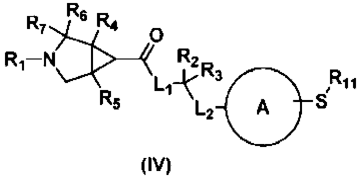 R₄ est choisi parmi un alkyle en C₁₋₆, un alcoxy en C₁₋₆, un alcoxy en C₁₋₆-alkyle en C₁₋₆, un -(CH₂)ₘ-carbocyclyle en C₃₋₁₀, un -(CH₂)ₘ-(hétérocyclyle à 3 à 10 chaînons), un -(CH₂)ₘ-O-carbocyclyle en C₃₋₁₀, un -(CH₂)ₘ-O-(hétérocyclyle à 3 à 10 chaînons), un phényle et un hétéroaryle à 5 à 6 chaînons, l'hétérocyclyle ou l'hétéroaryle contenant de 1 à 4 hétéroatomes choisis parmi N, O et S, et l'alkyle, l'alcoxy, le carbocyclyle, le phényle, l'hétéroaryle ou l'hétérocyclyle dans R₄ étant chacun indépendamment éventuellement substitués davantage par 0 à 4 substituants choisis parmi -H, -F, -Cl, -Br, -I, hydroxy, sulfhydryle, cyano, amino, un alkyle en C₁₋₄ et un alcoxy en C1-4.

7. Composé, ou le stéréoisomère, le N-oxyde, l'hydrate, le solvate, le sel pharmaceutiquement acceptable ou le polymorphe de celui-ci selon la revendication 1, dans lequel le composé présente les caractéristiques structurelles de la formule (V) : 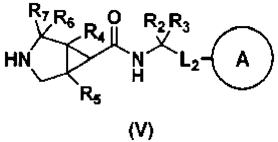
R₂ et R₃ sont chacun indépendamment choisis parmi un alkyle en C₁₋₆ et un cycloalkyle en C₃₋₆, à condition que R₂ et R₃ ne soient pas tous deux -H ; ou R₂ et R₃ forment ensemble un cycloalkyle saturé à 3 à 6 chaînons ;
R₄, R₅, R₆ et R₇ sont chacun indépendamment choisis parmi -H, un alkyle en C₁₋₆, un alcoxy en C₁₋₆ et un alcoxy en C₁₋₆-alkyle en C₁₋₆, et l'alkyle et l'alcoxy dans R₄, R₅, R₆ et R₇ étant chacun indépendamment éventuellement substitués davantage par 0 à 4 substituants choisis parmi -H, -F, -Cl, -Br, -I, hydroxy, sulfhydryle, cyano, amino, un alkyle en C₁₋₄ et un alcoxy en C₁₋₄ ;
A est choisi parmi un aryle en C₆₋₁₄, un hétéroaryle à 5 à 14 chaînons, un hétérocyclyle à 5 à 14 chaînons et un cycloalkyle à 5 à 14 chaînons, et l'aryle, l'hétéroaryle, l'hétérocyclyle et le cycloalkyle dans A sont éventuellement substitués davantage par 1 à 4 R₁₀, l'hétéroaryle ou l'hétérocyclyle contenant de 1 à 4 atomes N ;
lorsque L₂ est choisi parmi une liaison simple, R₁₀ est indépendamment choisi parmi -H, -F, -Cl, -Br, -I, hydroxy, cyano, amino, un alkyle en C₁₋₆, un alcoxy en C₁₋₄-alkyle en C₁₋₆, un cycloalkyle en C₃₋₆ et -SR₁₁, et au moins un R₁₀ est - SR₁₁, dans lequel R₁₁ est chacun indépendamment choisi parmi -H, un alkyle en C₁₋₆, un alcoxy en C₁₋₄-alkyle en C₁₋₆, un - (CH₂)ₙ-carbocyclyle en C₃₋₁₀, un -(CH₂)ₙ-(hétérocyclyle à 3 à 10 chaînons) et un hétéroaryle à 5 à 6 chaînons, l'hétérocyclyle ou l'hétéroaryle contenant de 1 à 4 hétéroatomes choisis parmi O, S et N, et l'alkyle, l'alcoxy, le carbocyclyle ou l'hétérocyclyle étant chacun indépendamment éventuellement substitué davantage par 0 à 4 substituants choisis parmi -H, un halogène, hydroxy, cyano, un alkyle en C₁₋₄ et un alcoxy en C₁₋₄ ;
lorsque L₂ est choisi parmi -(CRₐR_{b})ₙS-, R₁₀ est chacun indépendamment choisi parmi -H, -F, -Cl, -Br, -I, hydroxy, cyano, amino, un alkyle en C₁₋₄, un alcoxy en C₁₋₄ et un alcoxy en C₁₋₄-alkyle en C₁₋₆, l'alkyle et l'alcoxy étant chacun indépendamment éventuellement substitués davantage par 0 à 4 substituants choisis parmi H, un halogène, hydroxy, cyano, un alkyle en C₁₋₄ et un alcoxy en C₁₋₄ ;
Rₐ et R_{b} sont chacun indépendamment choisis parmi -H et un alkyle en C₁₋₆ ;
n est indépendamment choisi parmi 0, 1, 2 et 3 à chaque occurrence.

8. Composé, ou le stéréoisomère, le *N*-oxyde, l'hydrate, le solvate, le sel pharmaceutiquement acceptable ou le polymorphe de celui-ci selon la revendication 1, dans lequel A est choisi parmi les structures suivantes : 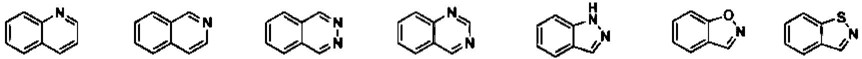 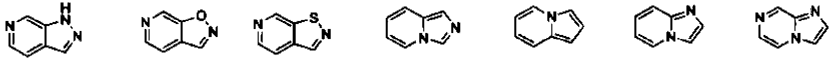 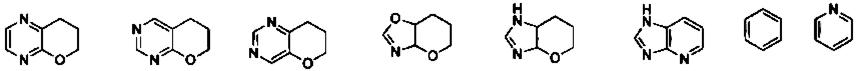 ; éventuellement dans lequel A est choisi parmi l'une des structures suivantes : 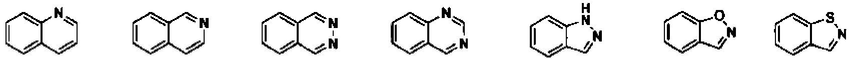 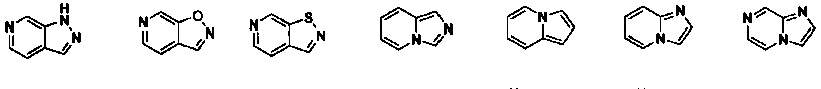 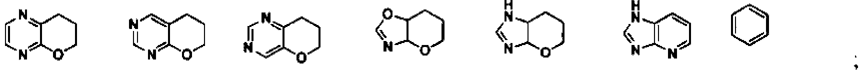 ; éventuellement dans lequel A est choisi parmi l'une des structures suivantes :

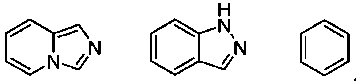

9. Composé, ou le stéréoisomère, le *N*-oxyde, l'hydrate, le solvate, le sel pharmaceutiquement acceptable, ou le polymorphe de celui-ci selon la revendication 1, dans lequel le composé présente les caractéristiques structurelles de la Formule (a) : 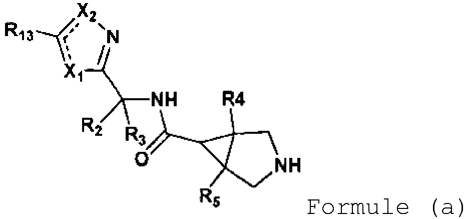 dans laquelle
-̅ -̅ -̅ représente une liaison simple ou double, à condition qu'une et une seule des deux --- représente une liaison double ;
X₁ est CR₁₃ ou NR₁₂ ;
X₂ est CR₁₃ ou NR₁₂ ;
en variante, X₁ et un substituant de celui-ci, ensemble avec un atome de carbone adjacent et un substituant R₁₃ de celui-ci, forment un cycle benzène ou un cycle hétéroaromatique à 5 ou 6 chaînons, et le cycle benzène ou le cycle hétéroaromatique à 5 ou 6 chaînons est éventuellement substitué par 1 à 4 R₁₃ ;
R₁₃ est, indépendamment pour chacun, choisi parmi -H, un halogène, cyano, -(CH₂)ₙ-NR'R", un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, un -(CH₂)ₙ-hétéroaryle à 5 ou 6 chaînons, et - SR₁₁ ;
R₁₂ est choisi parmi H, un alkyle en C₁₋₆, et un halogénoalkyle en C₁₋₆ ;
R₁₁ est choisi parmi -H, un alkyle en C₁₋₆, un halogénoalkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 ou 6 chaînons ;
R' et R" sont chacun choisis parmi l'hydrogène, un alkyle en C₁₋₆, et un halogénoalkyle en C₁₋₆, ou R' et R" ensemble avec un atome d'azote auquel ils sont attachés forment un hétérocyclyle à 3 à 10 chaînons ;
R est choisi parmi l'hydrogène, un alkyle en C₁₋₆, et un halogénoalkyle en C₁₋₆ ;
R₂ et R₃ sont, indépendamment pour chacun, choisis parmi H, un alkyle en C₁₋₆, un halogénoalkyle en C₁₋₆, et -(CH₂)ₙ-OR ;
R₄ et R₅ sont, indépendamment pour chacun, choisis parmi H, un alkyle en C₁₋₆, un halogénoalkyle en C₁₋₆, et -(CH₂)ₙ-OR ;
n est 0, 1, 2 ou 3,
à condition qu'au moins un R₁₃ soit un substituant - SR₁₁ ;
éventuellement dans lequel soit :
(a)
X₂ est CR₁₃ ;
X₁ et le substituant de celui-ci, ensemble avec l'atome de carbone adjacent et le substituant R₁₃ de celui-ci, forment un cycle benzène ou un cycle hétéroaromatique à 5 ou 6 chaînons, et le cycle benzène ou le cycle hétéroaromatique à 5 ou 6 chaînons est substitué par 1 à 4 R₁₃ ;
R₁₃ est, indépendamment pour chacun, choisi parmi -H, un halogène, cyano, -(CH₂)ₙ-NR'R", un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, un -(CH₂)ₙ-hétéroaryle à 5 ou 6 chaînons, et - SR₁₁ ;
R₁₁ est choisi parmi -H, un alkyle en C₁₋₆, un halogénoalkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 ou 6 chaînons ;
R' et R" sont chacun choisis parmi l'hydrogène, un alkyle en C₁₋₆, et un halogénoalkyle en C₁₋₆, ou R' et R" ensemble avec un atome d'azote auquel ils sont attachés forment un hétérocyclyle à 3 à 10 chaînons ;
R est choisi parmi l'hydrogène, un alkyle en C₁₋₆, et un halogénoalkyle en C₁₋₆ ;
R₂ et R₃ sont, indépendamment pour chacun, choisis parmi H, un alkyle en C₁₋₆, un halogénoalkyle en C₁₋₆, et -(CH₂)ₙ-OR ;
R₄ et R₅ sont, indépendamment pour chacun, choisis parmi H, un alkyle en C₁₋₆, un halogénoalkyle en C₁₋₆, et -(CH₂)ₙ-OR ;
n est 0, 1, 2 ou 3,
à condition qu'au moins un R₁₃ soit un substituant - SR₁₁ ;
soit
(b)
X₂ est NR₁₂ ;
X₁ et le substituant de celui-ci, ensemble avec l'atome de carbone adjacent et le substituant R₁₃ de celui-ci, forment un cycle benzène ou un cycle hétéroaromatique à 5 ou 6 chaînons, et le cycle benzène ou le cycle hétéroaromatique à 5 ou 6 chaînons est substitué par 1 à 4 R₁₃ ;
R₁₃ est, indépendamment pour chacun, choisi parmi -H, un halogène, cyano, -(CH₂)ₙ-NR'R", un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, un -(CH₂)ₙ-hétéroaryle à 5 ou 6 chaînons, et - SR₁₁ ;
R₁₁ est choisi parmi -H, un alkyle en C₁₋₆, un halogénoalkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 ou 6 chaînons ;
R' et R" sont chacun choisis parmi l'hydrogène, un alkyle en C₁₋₆, et un halogénoalkyle en C₁₋₆, ou R' et R" ensemble avec un atome d'azote auquel ils sont attachés forment un hétérocycle à 3 à 10 chaînons ;
R est choisi parmi l'hydrogène, un alkyle en C₁₋₆, et un halogénoalkyle en C₁₋₆ ;
R₂ et R₃ sont, indépendamment pour chacun, choisis parmi H, un alkyle en C₁₋₆, un halogénoalkyle en C₁₋₆, et -(CH₂)ₙ-OR ;
R₄ et R₅ sont, indépendamment pour chacun, choisis parmi H, un alkyle en C₁₋₆, un halogénoalkyle en C₁₋₆, et - (CH₂)ₙ-OR ;
n est 0, 1, 2 ou 3,
à condition qu'au moins un R₁₃ soit -SR₁₁.

10. Composé, ou le stéréoisomère, le N-oxyde, l'hydrate, le solvate, le sel pharmaceutiquement acceptable, ou le polymorphe de celui-ci selon la revendication 1, dans lequel le composé présente les caractéristiques structurelles de la Formule (b) : 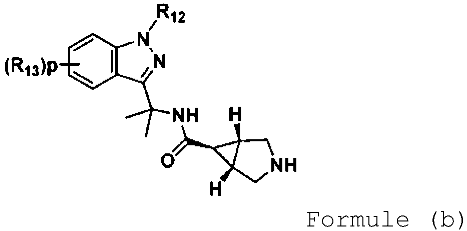
dans laquelle R₁₃ est, indépendamment pour chacun, choisi parmi H, un halogène, cyano, - (CH₂)ₙ-NR'R", un alkyle en C₁₋₆, un halogénoalkyle en C₁₋₆, - (CH₂)ₙ-OR, un - (CH₂)ₙ-alcényle en C₂₋₆, un - (CH₂)ₙ-alcynyle en C₂₋₆, un - (CH₂)ₙ-carbocyclyle en C₃₋₁₀, un - (CH₂)ₙ-(hétérocyclyle à 3 à 10 chaînons), un - (CH₂)ₙ-aryle en C₆₋₁₀, un - (CH₂)ₙ-hétéroaryle à 5 à 6 chaînons, et - SR₁₁, et au moins un R₁₃ est -SR₁₁ ;
R₁₂ est choisi parmi H, un alkyle en C₁₋₆, et un halogénoalkyle en C₁₋₆ ;
R₁₁ est choisi parmi H, un alkyle en C₁₋₆, un halogénoalkyle en C₁₋₆, un -(CH₂)ₙ-alcényle en C₂₋₆, un -(CH₂)ₙ-alcynyle en C₂₋₆, un - (CH₂)ₙ-carbocyclyle en C₃₋₁₀, un - (CH₂)ₙ-(hétérocyclyle à 3 à 10 chaînons), un -(CH₂)ₙ-aryle en C₆₋₁₀, et un - (CH₂)ₙ-hétéroaryle à 5 à 6 chaînons ;
R est choisi parmi l'hydrogène, un alkyle en C₁₋₆, et un halogénoalkyle en C₁₋₆ ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocycle à 3 à 10 chaînons ;
n est 0, 1, 2 ou 3 ;
p est 1, 2, 3 ou 4 ;
optionnellement, dans lequel soit :
(a)
p est 1, 2 ou 3 ;
R₁₃ est, indépendamment pour chacun, choisi parmi H, un halogène, un alkyle en C₁₋₆, un halogénoalkyle en C₁₋₆, un -(CH₂)ₙ-carbocyclyle en C₃₋₁₀, et -SR₁₁, et au moins un R₁₃ est -SR₁₁ ;
R₁₁ est choisi parmi un alkyle en C₁₋₆, un halogénoalkyle en C₁₋₆, et un -(CH₂)ₙ-carbocyclyle en C₃₋₆ ;
R₁₂ est choisi parmi H, un alkyle en C₁₋₆, et un halogénoalkyle en C₁₋₆ ;
n est 0 ou 1 ;
soit
(b)
p est 1 ou 2 ;
R₁₃ est, indépendamment pour chacun, choisi parmi H, un alkyle en C₁₋₆, un halogénoalkyle en C₁₋₆, et -SR₁₁, et au moins un R₁₃ est -SR₁₁ ;
R₁₁ est choisi parmi un alkyle en C₁₋₄ et un -(CH₂)ₙ-carbocyclyle en C₃₋₄ ;
R₁₂ est choisi parmi H, un alkyle en C₁₋₄, et un halogénoalkyle en C₁₋₄ ;
n est 0 ou 1 ;
soit
(c)
p est 1 ;
R₁₃ est -SMe ;
R₁₂ est H ou un alkyle en C₁₋₄.

11. Composé, ou le stéréoisomère, le *N*-oxyde, l'hydrate, le solvate, le sel pharmaceutiquement acceptable, ou le polymorphe de celui-ci selon la revendication 1, dans lequel le composé présente les caractéristiques structurelles de la Formule (b-1) : 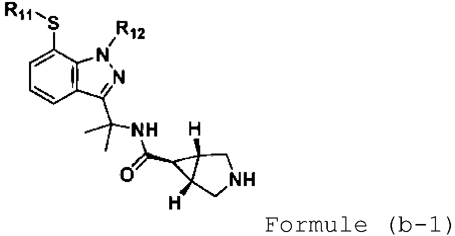 dans laquelle
R₁₁ est choisi parmi H, un alkyle en C₁₋₆, un halogénoalkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 à 6 chaînons ;
R₁₂ est choisi parmi H, un alkyle en C₁₋₆, et un halogénoalkyle en C₁₋₆ ; et
n est 0, 1, 2 ou 3 ;
optionnellement, dans lequel soit :
(a)
R₁₁ est choisi parmi un alkyle en C₁₋₆, un halogénoalkyle en C₁₋₆, et un -(CH₂)ₙ-carbocyclyle en C₃₋₆ ;
R₁₂ est choisi parmi H, un alkyle en C₁₋₆, et un halogénoalkyle en C₁₋₆ ; et
n est 0 ou 1 ;
soit
(b)
R₁₁ est choisi parmi un alkyle en C₁₋₄ et un -(CH₂)ₙ-carbocyclyle en C₃₋₄ ;
R₁₂ est choisi parmi H, un alkyle en C₁₋₄, et un halogénoalkyle en C₁₋₄ ;
n est 0 ou 1 ;
soit
(c)
R₁₁ est méthyle ;
R₁₂ est H ou un alkyle en C₁₋₄.

12. Composé, ou le stéréoisomère, le N-oxyde, l'hydrate, le solvate, le sel pharmaceutiquement acceptable, ou le polymorphe de celui-ci selon la revendication 1, dans lequel le composé présente les caractéristiques structurelles de la Formule (c) ou de la Formule (c-1) ou de la Formule (c-2) : 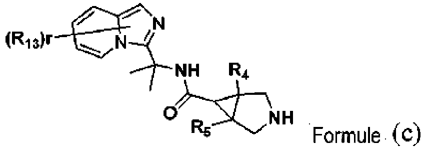 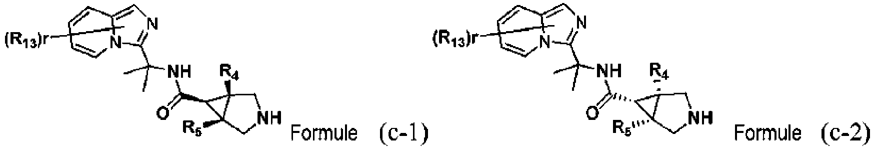 dans lesquelles
R₁₃ est, indépendamment pour chacun, choisi parmi H, un halogène, cyano, -(CH₂)ₙ-NR'R", un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, un -(CH₂)ₙ-hétéroaryle à 5 à 6 chaînons, et - SR₁₁, et au moins un R₁₃ est -SR₁₁ ;
R₁₁ est choisi parmi H, un alkyle en C₁₋₆, un halogénoalkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 à 6 chaînons ;
R₄ et R₅ sont, indépendamment pour chacun, choisis parmi H, un alkyle en C₁₋₆, un halogénoalkyle en C₁₋₆, et -(CH₂)ₙ-OR ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocyclyle à 3 à 10 chaînons ;
R est choisi parmi l'hydrogène, un alkyle en C₁₋₆, et un halogénoalkyle en C₁₋₆ ;
n est 0, 1, 2 ou 3 ;
r est 1, 2, 3, 4 ou 5 ;
optionnellement, dans lequel soit :
(a)
r est 1, 2 ou 3 ;
R₁₃ est, indépendamment pour chacun, choisi parmi H, un halogène, un alkyle en C₁₋₆, un halogénoalkyle en C₁₋₆, un -(CH₂)ₙ-carbocyclyle en C₃₋₁₀, et -SR₁₁, et au moins un R₁₃ est -SR₁₁ ;
R₁₁ est choisi parmi un alkyle en C₁₋₆, un halogénoalkyle en C₁₋₆, et un -(CH₂)ₙ-carbocyclyle en C₃₋₆ ;
R₄ et R₅ sont, indépendamment pour chacun, choisis parmi H et un alkyle en C₁₋₆ ;
n est 0 ou 1 ; soit
(b)
r est 1 ou 2 ;
R₁₃ est, indépendamment pour chacun, choisi parmi H, un alkyle en C₁₋₆, un halogénoalkyle en C₁₋₆, et -SR₁₁, et au moins un R₁₃ est -SR₁₁ ;
R₁₁ est choisi parmi un alkyle en C₁₋₄ et un -(CH₂)ₙ-carbocyclyle en C₃₋₄ ;
R₄ et R₅ sont, indépendamment pour chacun, choisis parmi H et un alkyle en C₁₋₆ ; n est 0 ou 1 ; soit
(c)
r est 2 ;
l'un des R₁₃ est -SMe, et un autre est choisi parmi H et un alkyle en C₁₋₄ ;
R₄ et R₅ sont, indépendamment pour chacun, choisis parmi H et un alkyle en C₁₋₄ ; soit
(d)
r est 1 ;
R₁₃ est -SMe ;
R₄ et R₅ sont, indépendamment pour chacun, choisis parmi H et un alkyle en C₁₋₄.

13. Composé, ou le stéréoisomère, le *N*-oxyde, l'hydrate, le solvate, le sel pharmaceutiquement acceptable, ou le polymorphe de celui-ci selon la revendication 1, dans lequel le composé présente des caractéristiques structurelles de la formule (d) : 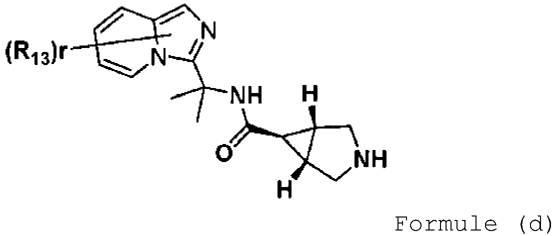 dans laquelle
R₁₃ est, indépendamment pour chacun, choisi parmi H, un halogène, cyano, -(CH₂)ₙ-NR'R", un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, un -(CH₂)ₙ-hétéroaryle à 5 à 6 chaînons, et - SR₁₁, et au moins un R₁₃ est -SR₁₁ ;
R₁₁ est choisi parmi H, un alkyle en C₁₋₆, un halogénoalkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 à 6 chaînons ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocyclyle à 3 à 10 chaînons ;
R est choisi parmi l'hydrogène, un alkyle en C₁₋₆, et un halogénoalkyle en C₁₋₆ ;
n est 0, 1, 2 ou 3 ;
r est 1, 2, 3, 4 ou 5 ;
optionnellement, dans lequel soit :
(a)
r est 1, 2 ou 3 ;
R₁₃ est, indépendamment pour chacun, choisi parmi H, un halogène, un alkyle en C₁₋₆, un halogénoalkyle en C₁₋₆, un -(CH₂)ₙ-carbocyclyle en C₃₋₁₀, et -SR₁₁, et au moins un R₁₃ est -SR₁₁ ;
R₁₁ est choisi parmi un alkyle en C₁₋₆, un halogénoalkyle en C₁₋₆, et un -(CH₂)ₙ-carbocyclyle en C₃₋₆ ;
n est 0 ou 1 ;
soit
(b)
r est 1 ou 2 ;
R₁₃ est, indépendamment pour chacun, choisi parmi H, un alkyle en C₁₋₆, un halogénoalkyle en C₁₋₆, et -SR₁₁, et au moins un R₁₃ est -SR₁₁ ;
R₁₁ est choisi parmi un alkyle en C₁₋₄ et un -(CH₂)ₙ-carbocyclyle en C₃₋₄ ;
n est 0 ou 1 ;
soit
(c)
r est 2 ;
l'un des R₁₃ est -SMe et un autre est choisi parmi H et un alkyle en C₁₋₄ ;
soit
(d) r est 1 ;
R₁₃ est -SMe.

14. Composé, ou le stéréoisomère, le *N*-oxyde, l'hydrate, le solvate, le sel pharmaceutiquement acceptable, ou le polymorphe de celui-ci selon la revendication 1, dans lequel le composé présente des caractéristiques structurelles de la formule (d-1) : 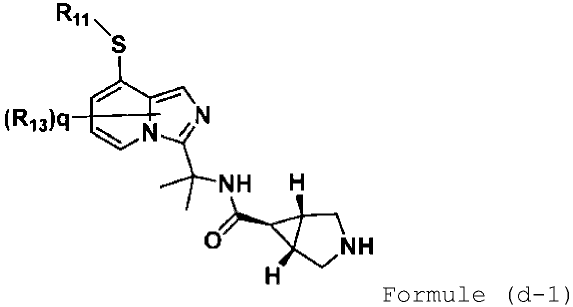 dans laquelle
R₁₁ est choisi parmi H, un alkyle en C₁₋₆, un halogénoalkyle en C₁₋₆, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 à 6 chaînons ;
R₁₃ est, indépendamment pour chacun, choisi parmi H, un halogène, cyano, -(CH₂)ₙ-NR'R", un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, un -(CH₂)ₙ-hétéroaryle à 5 à 6 chaînons, et - SR₁₁ ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocycle à 3 à 10 chaînons ;
R est choisi parmi l'hydrogène, un alkyle en C₁₋₆, et un halogénoalkyle en C₁₋₆ ;
n est 0, 1, 2 ou 3 ;
q est 0, 1, 2, 3 ou 4 ;
optionnellement, dans lequel soit :
(a)
q est 0, 1 ou 2 ;
R₁₃ est, indépendamment pour chacun, choisi parmi H, un halogène, un alkyle en C₁₋₆, un halogénoalkyle en C₁₋₆, un -(CH₂)ₙ-carbocyclyle en C₃₋₁₀, et -SR₁₁ ;
R₁₁ est choisi parmi un alkyle en C₁₋₆, un halogénoalkyle en C₁₋₆, et un -(CH₂)ₙ-carbocyclyle en C₃₋₆ ;
n est 0 ou 1 ;
soit
(b)
q est 0 ou 1 ;
R₁₁ est choisi parmi un alkyle en C₁₋₄ et un -(CH₂)ₙ-carbocyclyle en C₃₋₄ ;
R₁₃ est choisi parmi H, un alkyle en C₁₋₄, et un halogénoalkyle en C₁₋₄ ;
n est 0 ou 1 ;
soit
(c)
q est 0 ;
R₁₁ est un alkyle en C₁₋₄.

15. Composé, ou le stéréoisomère, le *N*-oxyde, l'hydrate, le solvate, le sel pharmaceutiquement acceptable, ou le polymorphe de celui-ci selon la revendication 1, dans lequel le composé présente des caractéristiques structurelles de la formule (d-2) : 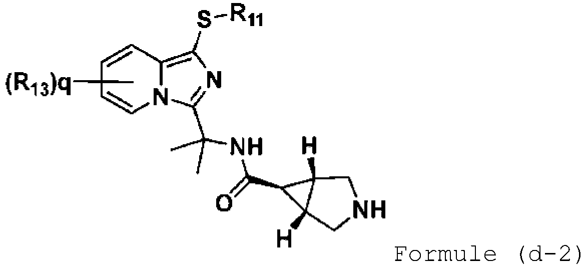 dans laquelle
R₁₁ est choisi parmi H, un alkyle en C₁₋₆, un halogénoalkyle en C₁₋₆, un - (CH₂)ₙ-alcényle en C₂₋₆, un - (CH₂)ₙ-alcynyle en C₂₋₆, un - (CH₂)ₙ-carbocyclyle en C₃₋₁₀, un - (CH₂)ₙ-(hétérocyclyle à 3 à 10 chaînons), un -(CH₂)ₙ-aryle en C₆₋₁₀, et un - (CH₂)ₙ-hétéroaryle à 5 à 6 chaînons ;
R₁₃ est, indépendamment pour chacun, choisi parmi H, un halogène, cyano, - (CH₂)ₙ-NR'R", un alkyle en C₁₋₆, un halogénoalkyle en C₁₋₆, - (CH₂)ₙ-OR, un - (CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, un -(CH₂)ₙ-hétéroaryle à 5 à 6 chaînons, et - SR₁₁ ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocycle à 3 à 10 chaînons ;
R est choisi parmi l'hydrogène, un alkyle en C₁₋₆, et un halogénoalkyle en C₁₋₆ ;
n est 0, 1, 2 ou 3 ;
q est 0, 1, 2, 3 ou 4 ;
optionnellement, dans lequel soit :
(a)
q est 0, 1 ou 2 ;
R₁₁ est choisi parmi un alkyle en C₁₋₆, un halogénoalkyle en C₁₋₆, et un -(CH₂)ₙ-carbocyclyle en C₃₋₆ ;
R₁₃ est, indépendamment pour chacun, choisi parmi H, un halogène, un alkyle en C₁₋₆, un halogénoalkyle en C₁₋₆, un -(CH₂)ₙ-carbocyclyle en C₃₋₁₀, et -SR₁₁ ;
n est 0 ou 1 ;
soit
(b)
q est 0 ou 1 ;
R₁₁ est choisi parmi un alkyle en C₁₋₄, un halogénoalkyle en C₁₋₄, et un -(CH₂)ₙ-carbocyclyle en C₃₋₄ ;
R₁₃ est, indépendamment pour chacun, choisi parmi H, un alkyle en C₁₋₆, et un halogénoalkyle en C₁₋₆ ;
n est 0 ou 1 ;
soit
(c)
q est 1 ;
R₁₁ est méthyle ;
R₁₃ est H ou un alkyle en C₁₋₄.

16. Composé, ou le stéréoisomère, le *N*-oxyde, l'hydrate, le solvate, le sel pharmaceutiquement acceptable, ou le polymorphe de celui-ci selon la revendication 1, dans lequel le composé présente les caractéristiques structurelles de la Formule (d-3) : 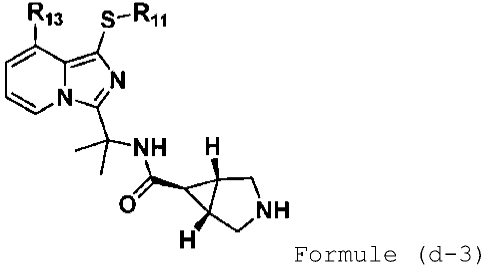 dans laquelle
R₁₁ est choisi parmi H, un alkyle en C₁₋₆, un halogénoalkyle en C₁₋₆, un - (CH₂)ₙ-alcényle en C₂₋₆, un - (CH₂)ₙ-alcynyle en C₂₋₆, un - (CH₂)ₙ-carbocyclyle en C₃₋₁₀, un - (CH₂)ₙ-(hétérocyclyle à 3 à 10 chaînons), un -(CH₂)ₙ-aryle en C₆₋₁₀, et un -(CH₂)ₙ-hétéroaryle à 5 ou 6 chaînons ;
R₁₃ est, indépendamment pour chacun, choisi parmi H, un halogène, cyano, - (CH₂)ₙ-NR'R", un alkyle en C₁₋₆, un halogénoalkyle en C₁₋₆, - (CH₂)ₙ-OR, un - (CH₂)ₙ-alcényle en C₂₋₆, un - (CH₂)ₙ-alcynyle en C₂₋₆, un - (CH₂)ₙ-carbocyclyle en C₃₋₁₀, un - (CH₂)ₙ-(hétérocyclyle à 3 à 10 chaînons), un - (CH₂)ₙ-aryle en C₆₋₁₀, un - (CH₂)ₙ-hétéroaryle à 5 ou 6 chaînons, et - SR₁₁ ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocyclyle à 3 à 10 chaînons ;
R est choisi parmi l'hydrogène, un alkyle en C₁₋₆, et un halogénoalkyle en C₁₋₆ ;
n est 0, 1, 2 ou 3 ;
optionnellement, dans lequel soit
(a)
R₁₁ est choisi parmi un alkyle en C₁₋₆, un halogénoalkyle en C₁₋₆, et un -(CH₂)ₙ-carbocyclyle en C₃₋₆ ;
R₁₃ est, indépendamment pour chacun, choisi parmi H, un halogène, un alkyle en C₁₋₆, un halogénoalkyle en C₁₋₆, un -(CH₂)ₙ-carbocyclyle en C₃₋₁₀, et -SR₁₁ ;
n est 0 ou 1 ;
soit
(b)
R₁₁ est choisi parmi un alkyle en C₁₋₄, un halogénoalkyle en C₁₋₄, et un -(CH₂)ₙ-carbocyclyle en C₃₋₄ ;
R₁₃ est, indépendamment pour chacun, choisi parmi H, un alkyle en C₁₋₄, et un halogénoalkyle en C₁₋₄ ;
n est 0 ou 1 ;
soit
(c)
R₁₁ est méthyle ;
R₁₃ est H ou un alkyle en C₁₋₄.

17. Composé, ou le stéréoisomère, le *N*-oxyde, l'hydrate, le solvate, le sel pharmaceutiquement acceptable, ou le polymorphe de celui-ci selon la revendication 1, dans lequel le composé présente les caractéristiques structurelles de la Formule (e) : 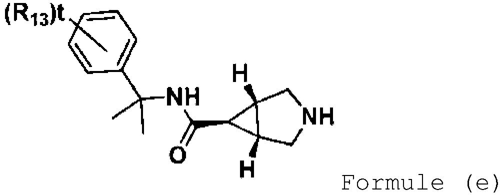 dans laquelle
R₁₃ est, indépendamment pour chacun, choisi parmi H, un halogène, cyano, -(CH₂)ₙ-NR'R", un alkyle en C₁₋₆, un halogénoalkyle en C₁₋₆, - (CH₂)ₙ-OR, un - (CH₂)ₙ-cycloaryle en C₃₋₁₀, et -SR₁₁, et au moins un R₁₃ est -SR₁₁ ;
R₁₁ est choisi parmi H, un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un - (CH₂)ₙ-(hétérocyclyle à 3 à 10 chaînons), un - (CH₂)ₙ-aryle en C₆₋₁₀, et un -(CH₂)ₙ-hétéroaryle à 5 ou 6 chaînons ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocyclyle à 3 à 10 chaînons ;
R est choisi parmi l'hydrogène, un alkyle en C₁₋₆, et un halogénoalkyle en C₁₋₆ ;
n est 0, 1, 2 ou 3 ;
t est 1, 2, 3, 4 ou 5 ;
optionnellement, dans lequel soit
(a)
t est 1, 2 ou 3 ;
R₁₃ est, indépendamment pour chacun, choisi parmi H, un halogène, un alkyle en C₁₋₆, un halogénoalkyle en C₁₋₆, un - (CH₂)ₙ-carbocyclyle en C₃₋₁₀, et -SR₁₁, et au moins un R₁₃ est -SR₁₁ ;
R₁₁ est choisi parmi un alkyle en C₁₋₆, un halogénoalkyle en C₁₋₆, et un -(CH₂)ₙ-carbocyclyle en C₃₋₆ ;
n est 0 ou 1 ;
soit
(b)
t est 1 ou 2 ;
R₁₃ est, indépendamment pour chacun, choisi parmi H, un alkyle en C₁₋₆, un halogénoalkyle en C₁₋₆, et -SR₁₁, et au moins un R₁₃ est -SR₁₁ ;
R₁₁ est choisi parmi un alkyle en C₁₋₄ et un -(CH₂)ₙ-carbocyclyle en C₃₋₄ ;
n est 0 ou 1 ;
soit
(c)
t est 1 ;
R₁₁ est un alkyle en C₁₋₄.

18. Composé, ou le stéréoisomère, le *N*-oxyde, l'hydrate, le solvate, le sel pharmaceutiquement acceptable, ou le polymorphe de celui-ci selon la revendication 1, dans lequel le composé présente les caractéristiques structurelles de la Formule (e-1) : 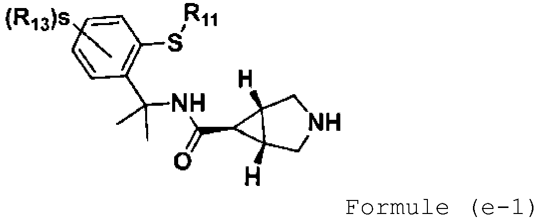 dans laquelle
R₁₃ est, indépendamment pour chacun, choisi parmi H, un halogène, cyano, -(CH₂)ₙ-NR'R", un alkyle en C₁₋₆, un halogénoalkyle en C₁₋₆, -(CH₂)ₙ-OR, un -(CH₂)ₙ-cycloaryle en C₃₋₁₀, et -SR₁₁ ;
R₁₁ est choisi parmi H, un alkyle en C₁₋₆, un halogénoalkyle en C₁₋₆, -(CH₂)ₙ-OR, un -(CH₂)ₙ-alcényle en C₂₋₆, un -(CH₂)ₙ-alcynyle en C₂₋₆, un -(CH₂)ₙ-carbocyclyle en C₃₋₁₀, un -(CH₂)ₙ-(hétérocyclyle à 3 à 10 chaînons), un -(CH₂)ₙ-aryle en C₆₋₁₀, et un -(CH₂)ₙ-hétéroaryle à 5 ou 6 chaînons ;
R' et R" sont chacun choisis parmi l'hydrogène, un alkyle en C₁₋₆, et un halogénoalkyle en C₁₋₆, ou R' et R" forment ensemble, avec un atome d'azote auquel ils sont attachés, un hétérocyclyle à 3 à 10 chaînons ;
R est choisi parmi l'hydrogène, un alkyle en C₁₋₆, et un halogénoalkyle en C₁₋₆ ;
n est 0, 1, 2 ou 3 ;
s est 0, 1, 2, 3 ou 4 ;
optionnellement, dans lequel soit
(a)
s est 0, 1 ou 2 ;
R₁₃ est, indépendamment pour chacun, choisi parmi H, un halogène, un alkyle en C₁₋₆, un halogénoalkyle en C₁₋₆, un -(CH₂)ₙ-carbocyclyle en C₃₋₁₀, et -SR₁₁ ;
R₁₁ est choisi parmi un alkyle en C₁₋₆, un halogénoalkyle en C₁₋₆, et un -(CH₂)ₙ-carbocyclyle en C₃₋₆ ;
n est 0 ou 1 ;
soit
(b)
s est 0 ou 1 ;
R₁₃ est, indépendamment pour chacun, choisi parmi H, un alkyle en C₁₋₆, un halogénoalkyle en C₁₋₆, un -(CH₂)ₙ-carbocyclyle en C₃₋₁₀, et -SR₁₁ ;
R₁₁ est choisi parmi un alkyle en C₁₋₄ et un -(CH₂)ₙ-carbocyclyle en C₃₋₄ ;
n est 0 ou 1 ;
soit
(c)
s est 0 ;
R₁₁ est un alkyle en C₁-₄.

19. Composé, ou le stéréoisomère, le *N*-oxyde, l'hydrate, le solvate, le sel pharmaceutiquement acceptable, ou le polymorphe de celui-ci selon la revendication 1, choisi parmi les structures suivantes :

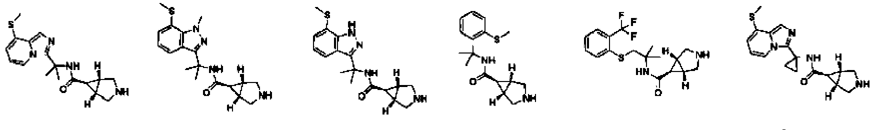

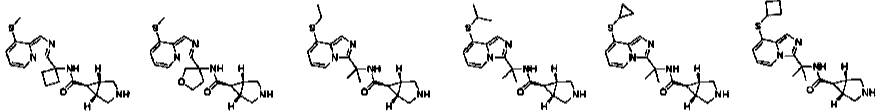

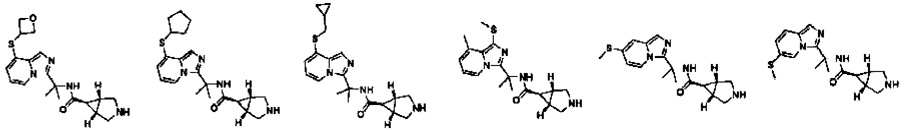

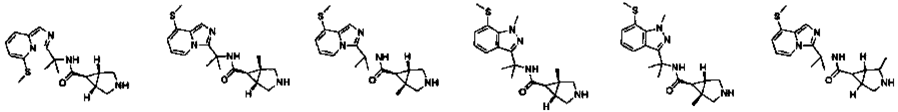

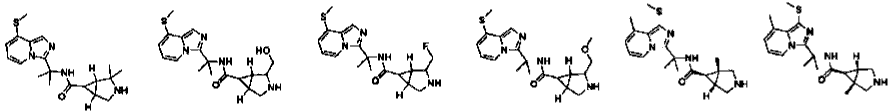

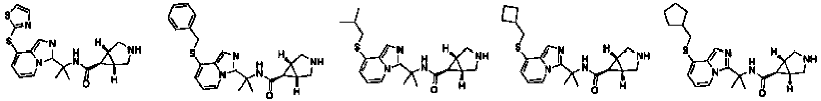

20. Composé, ou le stéréoisomère, le *N*-oxyde, l'hydrate, le solvate, le sel pharmaceutiquement acceptable, ou le polymorphe de celui-ci selon l'une quelconque des revendications 1 à 19, dans lequel le sel pharmaceutiquement acceptable est choisi parmi un chlorhydrate, un bromhydrate, un sulfate, un nitrate, un phosphate, un acétate, un maléate, un succinate, un mandélate, un fumarate, un malonate, un malate, un 2-hydroxypropionate, un oxalate, un glycolate, un salicylate, un glucuronate, un galacturonate, un citrate, un tartrate, un aspartate, un glutamate, un benzoate, un cinnamate, un *p*-toluènesulfonate, un benzènesulfonate, un mésylate, un éthanesulfonate et un trifluorométhanesulfonate, ou des combinaisons de ceux-ci.

21. Composition pharmaceutique, **caractérisée en ce qu'**elle contient une dose thérapeutiquement efficace du composé ou du stéréoisomère, du *N*-oxyde, de l'hydrate, du solvate, du sel pharmaceutiquement acceptable, ou du polymorphe de celui-ci selon l'une quelconque des revendications 1 à 20, et un excipient ou véhicule pharmaceutiquement acceptable.

22. Composé, ou le stéréoisomère, le *N*-oxyde, l'hydrate, le solvate, le sel pharmaceutiquement acceptable, ou le polymorphe de celui-ci selon l'une quelconque des revendications 1 à 20, ou composition pharmaceutique selon la revendication 21, pour une utilisation en thérapie.

23. Composé ou le stéréoisomère, le *N*-oxyde, l'hydrate, le solvate, le sel pharmaceutiquement acceptable, ou le polymorphe de celui-ci selon l'une quelconque des revendications 1 à 20, ou composition pharmaceutique selon la revendication 21, utilisé pour traiter et/ou prévenir la douleur ; éventuellement dans lequel :
(a) la douleur est une névralgie ; ou
(b) la douleur est une douleur dorsale, une douleur dorsale chronique, une névralgie du trijumeau, un syndrome douloureux régional complexe de type I, un syndrome douloureux régional complexe de type II, un syndrome du côlon irritable, une neuropathie diabétique, une douleur causée par l'arthrose, une douleur tumorale, ou une douleur des fibres musculaires.
